# FASCICULE DE BREVET EUROPEEN

(11) **EP 1 584 615 B1**
(45) Date de publication et mention de la délivrance du brevet: **28.05.2008**
(21) Numéro de dépôt: 05290715.1
(22) Date de dépôt: 01.04.2005
(51) Int. Cl.: C07C 323/41, C07C 323/60, A61K 8/44, A61K 8/46, A61Q 5/04

(54) **Utilisation de dimercaptoamides pour la déformation permanente des fibres kératiniques**
Anwendung von Dimercaptoamiden zur dauerhaften Verformung von Keratinfasern
Use of dimercaptoamides for permanently deforming of keratinic fibres

(30) Priorité: 06.04.2004 FR 0403598
(43) Date de publication de la demande: 12.10.2005
(73) Titulaire: L'ORÉAL, 75008 Paris (FR)
(72) Inventeur: Philippe, Michel, 91320 Wissous (FR); Malle, Gérard, 77580 Villiers S/Morin (FR); Blaise, Christian, 94160 Saint Mande (FR)
(74) Mandataire: Dossmann, Gérard

(56) Documents cités:
- EP-A- 0 368 763
- EP-A- 0 432 000
- EP-A- 0 514 282
- WO-A-01/74318
- DE-B- 1 095 805
- US-A- 4 137 420
- DATABASE CAPLUS CHEMICAL ABSTRACTS SERVICE, COLUMBUS, OHIO, US; XP002307151 extrait de STN Database accession no. 1996:546085 & JP 08 157448 A (KAO CORP) 18 juin 1996 (1996-06-18)
- DATABASE CAPLUS CHEMICAL ABSTRACTS SERVICE, COLUMBUS, OHIO, US; XP002307149 extrait de STN Database accession no. 1991:679408 & JP 03 176466 A (SANTEN PHARMACEUTICAL CO LTD) 31 juillet 1991 (1991-07-31)
- J. HUANG ET AL: INORGANIC CHEMISTRY, vol. 34, no. 5, 1995, pages 1090-1093, XP002307033

## Description

La présente invention est relative à l'utilisation de dimercaptoamides pour la déformation permanente des fibres kératiniques, et en particulier des fibres kératiniques humaines telles que les cheveux. Elle vise également un procédé de déformation permanente des fibres kératiniques mettant en oeuvre lesdits dimercaptoamides, ainsi que de nouveaux dimercaptoamides, leur procédé de préparation et les compositions les comprenant.

La technique la plus usuelle pour obtenir une déformation permanente des cheveux consiste, dans un premier temps à réaliser l'ouverture des liaisons disulfures de la kératine (ou cystine) à l'aide d'une composition contenant un agent réducteur, puis après avoir de préférence rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur de façon à donner à la chevelure la forme recherchée. Cette technique permet indifféremment de réaliser soit l'ondulation des cheveux, soit leur défrisage ou leur décrêpage ou leur lissage.

Les compositions réductrices généralement utilisées pour la première étape d'une opération de permanente contiennent à titre d'agent réducteur des sulfites, des bisulfites, ou de préférence des thiols. Parmi ces derniers, ceux couramment utilisés sont l'acide thioglycolique, la cystéamine, l'acide thiolactique, la cystéine et le monothioglycolate de glycérol. L'acide thioglycolique est particulièrement efficace pour réduire les liaisons disulfures de la kératine à pH alcalin, notamment sous forme de thioglycolate d'ammonium, et constitue le produit le plus utilisé en permanente. On a toutefois constaté que l'acide thioglycolique devait être utilisé en milieu suffisamment basique (en pratique à pH ≥ 8,5) si on voulait obtenir une frisure satisfaisante en intensité. Outre l'inconvénient de dégager une odeur désagréable nécessitant l'usage de parfums plus ou moins efficaces pour masquer les odeurs, la combinaison acide thioglycolique et pH alcalin conduit à des dégradations de la fibre capillaire.

Les sulfites ou bisulfites ont été utilisés antérieurement aux thiols en général et à l'acide thioglycolique en particulier. Contrairement aux thiols, ils sont utilisés à un pH acide généralement compris entre pH 4 et pH 6. Cependant, le degré de frisure obtenu est très inférieur et loin d'être satisfaisant.

La cystéine produit une odeur beaucoup plus faible que celle de l'acide thioglycolique mais le degré de frisure obtenu est également très inférieur et loin d'être satisfaisant. De plus, la cystéine nécessite l'utilisation d'un pH très alcalin.

Le monothioglycolate de glycérol est également très malodorant. Il est, par contre, utilisé à un pH proche de la neutralité, mais ses performances sont notablement inférieures à celles de l'acide thioglycolique.

La cystéamine peut être utilisée sur une plus large gamme de pH. Son efficacité est voisine de celle de l'acide thioglycolique mais elle conduit également à des dégradations importantes de la fibre capillaire.

Diverses études ont été conduites en vue de remédier aux inconvénients de ces agents réducteurs, et à cet effet, il a été proposé l'emploi de nouveaux composés ou systèmes réducteurs. Cependant, très peu de dithiols ont été proposés. Dans le passé, deux dithiols avaient notamment été largement étudiés : le dithiothréitol DTT et l'acide 2,5-dimercaptoadipique mais ils n'ont jamais été développés pour la déformation permanente des cheveux, en particulier à cause d'une odeur très désagréable pour le DTT et d'une activité insuffisante pour l'acide dimercaptoadipique. Plus récemment, dans la demande de brevet EP-A-0721772, il a déjà été proposé d'utiliser l'acide 2,3-dimercaptosuccinique, qui s'est avéré moins efficace que l'acide thioglycolique. Il a également été proposé dans le brevet US 5350572 d'utiliser des polyoxyéthylèneglycols dimercaptoalkyesters. Si ces composés possèdent une certaine efficacité, leur conservation dans le temps n'est pas satisfaisante.

Le brevet WO 01/74318 divulgue la N-2-hydroxypropyl, 2-mercaptopropylamide et le N-2-méthoxyéthyl, 2-mercaptopropylamide et leur utilisation comme agent réducteur dans des compositions pour permanente.

Après diverses études, la demanderesse a maintenant découvert de façon tout à fait surprenante et inattendue que l'utilisation de certains dimercaptoamides comme agents réducteurs conduisait à une frisure satisfaisante en intensité et en tenue dans le temps ainsi qu'à une dégradation moins importante de la fibre par rapport aux agents réducteurs précédemment cités.

La présente invention a donc pour objet l'utilisation, en tant qu'agent réducteur pour la déformation permanente des fibres kératiniques, et en particulier des fibres kératiniques humaines telles que les cheveux, d'un dimercaptoamide de formule générale (I) suivante :

HS-A-CO-NH-B-SH (I)

dans laquelle :
A représente un radical (CH₂)ₙ, n étant un nombre entier compris entre 1 et 5, éventuellement substitué par :
   (i) un radical alkyle en C1-C5, linéaire ou ramifié ; phényle ; benzyle ; amino ; acétylamino ; NH-CO-CH2-NH2 ; NH-CO-CH2-CH2-CH(NH2)COOH ; NH-CO-CH2-CH2-CH(COOH)COOH ; CH2-COOH; CH2-COOCH3 ; ou CH2-COOCH2-CH3, ou
   (ii) deux radicaux méthyle ou deux radicaux éthyle
et B représente un radical (CH₂)ₚ, p étant un nombre entier compris entre 1 et 5, éventuellement substitué par :
   (i) un radical alkyle en C1-C5, linéaire ou ramifié ; carboxyle ; COOCH3 ; COOEt ; CONH2 ; CONH-CH3 ; CON(CH3)2 ; CONH-CH2-CH3 ; CON(CH2-CH3)2 ; CONH-CH2-CHOH-CH3 ; ; CO-NH-CH(COOH)-(CH2)4-NH2 ; CO-NH-CH(COOH)-(CH2)4-N(CH3)2 ; CO-NH-CH2-CH2-COOEt ; CO-NH-CH(COOH)-iPr ; CO-NH-CH2-R avec R = CO-NH2 ou (CH2)3-NH2 ou (CH2)4-NH2 ou (CH2)5-NH2 ou (CH2)4-OH, ou
   (ii) 2 radicaux méthyle,
la somme n+p étant comprise entre 2 au minimum et 6 au maximum, et leurs sels organiques et minéraux.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

La neutralisation des groupes anioniques peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la dimethylamino2-propanol. On peut encore citer la lysine ou la 3-(diméthylamino)propylamine.

L'invention vise également un procédé de déformation permanente des fibres kératiniques, et notamment des fibres kératiniques humaines telles que les cheveux, mettant en oeuvre une composition réductrice comprenant au moins un composé de formule (I).

Elle a également pour objet une composition aqueuse réductrice, pour le premier temps d'une opération de déformation permanente des cheveux, contenant, en tant qu'agent réducteur, un dipercaptoamide de formule (I).

Elle a enfin pour objet des dipercaptoamides nouveaux.

D'autres caractéristiques, aspects, objets et avantages de l'invention apparaîtront encore plus clairement à la lecture de la description et des exemples qui suivent.

On entend par « déformation permanente des fibres kératiniques», le frisage permanent (encore appelé permanente), le défrisage ou le décrêpage des fibres kératiniques.

Les composés de formule (I) sont généralement préparés suivant les modes opératoires décrits dans les références suivantes :
J.Chem.Soc.PERKIN Trans.1,(7), 1994, 797-806
Chem.Pharm.Bull., 26, 1978, 1333-1335
Chem.Pharm.Bull., 41(6), 1993, 1066-1073
Chem.Pharm.Bull., 29(4), 1981, 940-947
Santen DE 2709820
J.Org.Chem., 44(25), 1979, 4699-4701
J.Org.Chem., 60(25), 1995, 8120-8121
Isr.J.Chem., 11, 1973, 573-586
J.Biol.Chem., 118, 1937, 327
J.Biol.Chem., 121, 1937, 12
J.Chem.Soc.PERKIN Trans.2, 1980, 1297-1300
J.Chem.Soc., 1956, 3148-3151
US 2003158184
Bioconjugate Chemistry 2002, 13(2), 285-294
WO 9819672 A1
WO 9730027 A1
Inorganic Chemistry 1995, 34(5), 1090-3
International Journal of Pharmaceutics 1994, 105(1), 11-18
WO 9319024 A3
WO 9108199A1
EP 326326 B1
EP 254032 A3
Spectroscopy Letters 1983, 16(6), 463-70
Chemical & Pharmaceutical Bulletin 1981, 29(4), 940-7
Chemica Scripta 1980, 16(3), 97-101
US 4305958

Parmi les composés de formule générale (I), on peut notamment citer les composés préférés ci-après :
- le 2-mercapto-N-(mercaptométhyl)-acétamide
- le 2-mercapto-N-(2-mercaptoéthyl)-acétamide
- le 2-mercapto-N-(3-mercaptopropyl)-acétamide
- le 2-mercapto-N-(4-mercaptobutyl)-acétamide
- le 2-mercapto-N-(5-mercaptopentyl)-acétamide
- le 3-mercapto-2-(2-mercapto-acétylamino)-propionamide
- le 3-mercapto-2-(2-mercapto-acetylamino)-N,N-diméthylpropionamide
- le 2-mercapto-N-(2-mercapto-1,1-diméthyl-éthyl)-acétamide
- le 2-mercapto-N-(1-mercaptométhyl-propyl)-acétamide
- le mercapto-[(mercaptoacétyl)amino]-acétate d'éthyle
- la N-(mercaptoacétyl)-L-cystéine
- la N-(mercaptoacétyl)-L-Homocystéine
- la N-(mercaptoacétyl)-L-Homocystéine, sel de sodium
- le 2-Mercapto-N-mercaptométhyl-propionamide
- le 2-Mercapto-N-(2-mercapto-éthyl)-propionamide
- le 2-Mercapto-N-(3-mercapto-propyl)-propionamide
- le 2-Mercapto-N-(4-mercapto-butyl)-propionamide
- le 2-Mercapto-N-(5-mercapto-pentyl)-propionamide
- l'acide mercapto[(2-mercapto-1-oxopropyl)amino]-acétique
- la N-(2-mercapto-1-oxopropyl)-DL-cystéine
- la (R)-N-(2-mercapto-1-oxopropyl)-L-cystéine
- la (S)-N-(2-mercapto-1-oxopropyl)-L-cystéine
- la 2-mercaptopropionyl-L-cystéine
- le N-(1-Carbamoyl-2-mercapto-éthyl)-2-mercapto-propionamide
- le N-(1-Diméthylcarbamoyl-2-mercapto-éthyl)-2-mercapto-propionamide
- le 4-Mercapto-2-(2-mercapto-propionylamino)-butyric acid
- le 2-Mercapto-N-(2-mercapto-1,1-diméthyl-éthyl)-propionamide
- le 2-Mercapto-butanoic acid mercaptométhyl-amide
- le 2-Mercapto-butanoic acid (2-mercapto-éthyl)-amide
- le 2-Mercapto-butanoic acid (3-mercapto-propyl)-amide
- le 2-Mercapto-butanoic acid (4-mercapto-butyl)-amide
- le 2-Mercapto-butanoic acid (5-mercapto-pentyl)-amide
- le 2-Mercapto-butanoic acid (1-carbamoyl-2-mercapto-éthyl)-amide
- le N-(1-Diméthylcarbamoyl-2-mercapto-éthyl)-2-mercapto-butyramide
- le 2-Mercapto-butanoic acid (1-carbamoyl-3-mercapto-propyl)-amide
- le 2-Mercapto-butanoic acid (2-mercapto-1,1-diméthyl-éthyl)-amide
- le 2-Mercapto-pentanoic acid mercaptométhyl-amide
- le 2-Mercapto-pentanoic acid (2-mercapto-éthyl)-amide
- le 2-Mercapto-pentanoic acid (3-mercapto-propyl)-amide
- le 2-Mercapto-pentanoic acid (4-mercapto-butyl)-amide
- le 2-Mercapto-pentanoic acid (5-mercapto-pentyl)-amide
- le 3-Mercapto-2-(2-mercapto-pentanoylamino)-propionic acid
- le 2-Mercapto-pentanoic acid (1-carbamoyl-2-mercapto-éthyl)-amide
- le 2-Mercapto-pentanoic acid (1-diméthylcarbamoyl-2-mercapto-éthyl)-amide
- le 2-Mercapto-pentanoic acid (1-carbamoyl-3-mercapto-propyl)-amide
- le 2-Mercapto-pentanoic acid (2-mercapto-1,1-diméthyl-éthyl)-amide
- le mercapto[(mercaptophénylacétyl)amino]-acétate d'éthyle
- la a (R)-N-[2-(mercaptométhyl)-1-oxo-3-phénylpropyl]-L-Cystéine
- la (R)-N-[2-(mercaptométhyl)-1-oxo-3-phénylpropyl]-L-Homocystéine
- la (S)-N-[2-(mercaptométhyl)-1-oxo-3-phénylpropyl]-L-Homocystéine
- le 3-mercapto-N-(2-mercaptoéthyl)-propanamide
- le 3-Mercapto-N-mercaptométhyl-propionamide
- le 3-Mercapto-N-(3-mercapto-propyl)-propionamide
- le 3-Mercapto-N-(4-mercapto-butyl)-propionamide
- la N-(3-mercapto-1-oxopropyl)-L-Cystéine
- le 3-Mercapto-2-(3-mercapto-propionylamino)-propionamide
- le N-(1-Diméthylcarbamoyl-2-mercapto-éthyl)-3-mercapto-propionamide
- le 4-Mercapto-2-(3-mercapto-propionylamino)-butyric acid
- le 3-Mercapto-N-(2-mercapto-1,1-diméthyl-éthyl)-propionamide
- le 3-Mercapto-N-mercaptométhyl-2-méthyl-propionamide
- le 3-Mercapto-N-(2-mercaptoéthyl)-2-méthyl-propionamide
- le 3-Mercapto-N-(3-mercapto-propyl)-2-méthyl-propionamide
- le 3-Mercapto-N-(4-mercapto-butyl)- 2-méthyl-propionamide
- le 3-Mercapto-2-(3-mercapto-2-méthyl-propionylamino)-propionic acid
- le N-(1-Carbamoyl-2-mercapto-éthyl)-3-mercapto-2-méthyl-propionamide
- le N-(1-Diméthylcarbamoyl-2-mercapto-éthyl)-3-mercapto-2-méthyl-propionamide
- le 3-Mercapto-N-(2-mercapto-1,1-diméthyl-éthyl)- 2-méthyl-propionamide
- la N-(3-mercapto-2-méthyl-1-oxopropyl)-L-Homocystéine
- la N-[2-(mercaptométhyl)-1-oxo-3-phénylpropyl]-Cystéine
- le 4-[(1-carboxy-2-mercaptoéthyl)amino]-3-(mercaptométhyl)-4-oxo-Butanoate de méthyle
- le N-mercaptométhyl-3-mercaptométhyl-succinamic acid
- le N-(2-Mercapto-éthyl)-3-mercaptométhyl-succinamic acid
- le N-(3-Mercapto-propyl)-3-mercaptométhyl-succinamic acid
- le N-(4-Mercapto-butyl)-3-mercaptométhyl-succinamic acid
- le N-(1-Carboxy-2-mercapto-éthyl)-3-mercaptométhyl-succinamic acid
- le N-(1-Carboxy-3-mercapto-propyl)-3-mercaptométhyl-succinamic acid
- le N-(2-Mercapto-1,1-diméthyl-éthyl)-3-mercaptométhyl-succinamic acid
- le N-(2-Mercapto-1-éthyl)-3-mercaptométhyl-succinamic acid
- le 2-Mercapto-N-mercaptométhyl-2-méthyl-propionamide
- le 2-mercapto-N-(2-mercaptoéthyl)-2-méthyl-propanamide
- le 2-Mercapto-N-(3-mercapto-propyl)-2-méthyl-propionamide
- le 2-Mercapto-N-(4-mercapto-butyl)-2-méthyl-propionamide
- le 2-Mercapto-N-(5-mercapto-pentyl)-2-méthyl-propionamide
- le 2-Mercapto-N-(2-mercapto-1,1-diméthyl-éthyl)-2-méthyl-propionamide
- le 2-Mercapto-N-(2-mercapto-1-éthyl)-2-méthyl-propionamide
- la N-(2-mercapto-2-méthyl-1-oxopropyl)-D-Cystéine
- la N-(2-mercapto-2-méthyl-1-oxopropyl)-Cystéine
- la N-(2-mercapto-2-méthyl-1-oxopropyl)-L-Cystéine, monosel de sodium
- la N-(2-mercapto-2-méthyl-1-oxopropyl)-L-Cystéine
- le (R)-N-[2-amino-1-(mercaptométhyl)-2-oxoéthyl]-2-mercapto-2-méthyl-Propanamide
- le N-(2-mercapto-2-méthyl-1-oxopropyl)-L-Cystéinate de méthyle
- le (2R)-3-mercapto-2-[(2-mercapto-2-méthyl-1-oxopropyl)amino]-N,N-diméthyl-Propanamide
- la N2-[N-(2-mercapto-2-méthyl-1-oxopropyl)-L-cystéinyl]-L-Lysine
- la N-(2-mercapto-2-méthyl-1-oxopropyl)-Homocystéine
- la N-(2-éthyl-2-mercapto-1-oxobutyl)-L-Cystéine
- le 3-Mercapto-N-mercaptométhyl-3-méthyl-butyramide
- le 3-mercapto-N-(2-mercaptoéthyl)-3-méthyl-Butanamide
- le 3-Mercapto-N-(3-mercapto-propyl)-3-méthyl-butyramide
- le 3-Mercapto-N-(4-mercapto-butyl)-3-méthyl-butyramide
- la N-(3-mercapto-3-méthyl-1-oxobutyl)-L-Cystéine
- le N-(1-Carbamoyl-2-mercapto-éthyl)-3-mercapto-3-méthyl-butyramide
- le N-(1-Diméthylcarbamoyl-2-mercapto-éthyl)-3-mercapto-3-méthyl-butyramide
- le 4-Mercapto-2-(3-mercapto-3-méthyl-butyrylamino)-butyric acid
- le 3-Mercapto-N-(2-mercapto-1,1-diméthyl-éthyl)-3-méthyl-butyramide
- le 3-Mercapto-N-(2-mercapto-1-éthyl)-3-méthyl-butyramide
- le 3-Mercapto-N-mercaptométhyl-2,2-diméthyl-propionamide
- le 3-Mercapto-N-(2-mercaptoéthyl)-2,2-diméthyl-propionamide
- le 3-Mercapto-N-(3-mercapto-propyl)-2,2-diméthyl-propionamide
- le 3-Mercapto-N-(4-mercapto-butyl)- 2,2-diméthyl-propionamide
- le 3-Mercapto-N-(2-mercapto-1,1-diméthyl-éthyl)- 2,2-diméthyl-propionamide
- la N-(3-mercapto-2,2-diméthyl-1-oxopropyl)-D-Cystéine
- la N-(3-mercapto-2,2-diméthyl-1-oxopropyl)-L-Cystéine
- le N-(1-Carbamoyl-2-mercapto-éthyl)-3-mercapto-2,2-diméthyl-propionamide
- le N-(1-Diméthylcarbamoyl-2-mercapto-éthyl)-3-mercapto-2,2-diméthyl-propionamide
- le N-(3-mercapto-2,2-diméthyl-1-oxopropyl)-D-cystéinyl-Glycinamide
- la N6-[(1,1-diméthyléthoxy)carbonyl]-N2-[N-(3-mercapto-2,2-diméthyl-1-oxopropyl)-L-cystéinyl]-L-Lysine
- le N-[2-[(4-aminobutyl)amino]-1-(mercaptométhyl)-2-oxoéthyl]-3-mercapto-2,2-diméthyl-Propanamide, monochlorhydrate
- le N-[2-[(5-aminopentyl)amino]-1-(mercaptométhyl)-2-oxoéthyl]-3-mercapto-2,2-diméthyl-Propanamide, monochlorhydrate
- le N-[2-[(6-aminohexyl)amino]-1-(mercaptométhyl)-2-oxoéthyl]-3-mercapto-2,2-diméthyl-Propanamide, monochlorhydrate
- le (R)-N-[2-[(5-hydroxypentyl)amino]-1-(mercaptométhyl)-2-oxoéthyl]-3-mercapto-2,2-diméthyl-Propanamide
- la N2-[N-(3-mercapto-2,2-diméthyl-1-oxopropyl)-L-cystéinyl]-L-Lysine
- la N2-[N-(3-mercapto-2,2-diméthyl-1-oxopropyl)-L-cystéinyl]-N6,N6-diméthyl-L-Lysine
- la 3-mercapto-N-(3-mercapto-2,2-diméthyl-1-oxopropyl)-D-Valine
- la N-(3-mercapto-2,2-diméthyl-1-oxopropyl)-Homocystéine
- la N-[2-éthyl-2-(mercaptométhyl)-1-oxobutyl]-L-cystéine
- le 2-Amino-3-mercapto-N-mercaptométhyl-propionamide
- le (2R)- 2-amino-3-mercapto-N-(2-mercaptoéthyl)-Propanamide
- le (2S)-2-amino-3-mercapto-N-(2-mercaptoéthyl)-Propanamide
- le 2-Amino-3-mercapto-N-(3-mercapto-propyl)-propionamide
- le 2-Amino-3-mercapto-N-(4-mercapto-butyl)-propionamide
- la L-cystéinyl-L-Cystéine
- le Mercapto[(mercaptoacétyl)amino]-acétate de méthyle
- le L-cystéinyl-L-Cystéinate d'éthyle
- le 2-Amino-N-(1-carbamoyl-2-mercapto-éthyl)-3-mercapto-propionamide
- le 2-Amino-N-(1-carbamoyl-2-mercapto-éthyl)-3-mercapto-N,N-diméthyl-propionamide
- le 2-(2-Amino-3-mercapto-propionylamino)-4-mercapto-butyric acid
- le 2-Amino-3-mercapto-N-(2-mercapto-1,1-diméthyl-éthyl)-propionamide
- le 2-Amino-3-mercapto-N-(2-mercapto-1-éthyl)-propionamide
- le 2-Acétylamino-3-mercapto-N-mercaptométhyl-propionamide
- le (2R)-2-(acétylamino)-3-mercapto-N-(2-mercaptoéthyl)-Propanamide
- le 2-Acétylamino-3-mercapto-N-(3-mercapto-propyl)-propionamide
- le 2-Acétylamino-3-mercapto-N-(4-mercapto-butyl)-propionamide
- le 2-(2-Acétylamino-3-mercapto-propionylamino)-3-mercapto-3-méthyl-butyric acid
- le 2-Acétylamino-3-mercapto-N-(1-mercaptométhyl-propyl)-propionamide
- le 2-Acétylamino-N-(1-diméthylcarbamoyl-2-mercapto-éthyl)-3-mercapto-propionamide
- la N-acétyl-L-cystéinyl-L-Cystéine
- la N-(N-acétylcystéinyl)-L-Cystéine
- la N-(N-acétylcystéinyl)-DL-Cystéine
- le N-acétyl-L-cystéinyl-D-Cystéinate d'éthyle
- le N-acétyl-L-cystéinyl-L-Cystéinate d'éthyle
- le 2-acétamido-N-(1-carbamoyl-2-mercaptoéthyl)-3-mercapto-Propionamide
- le N-acétyl-L-cystéinyl-L-cystéinyl-Glycinate d'éthyle
- le 2-(2-Acétylamino-3-mercaptô-propionylamino)-4-mercapto-butyric acid
- le 2-(carboxyméthylamino)-3-mercapto-N-mercaptométhyl-propionamide
- le 2-(carboxyméthylamino)-3-mercapto-N-(2-mercapto-éthyl)-propionamide
- le 2-(carboxyméthylamino)-3-mercapto-N-(3-mercapto-propyl)-propionamide
- le 2-(carboxyméthylamino)-3-mercapto-N-(4-mercapto-butyl)-propionamide
- le 2-(carboxyméthylamino)-N-(1-carbamoyl-2-mercapto-éthyl)-3-mercapto- propionamide
- le 2-[2-(Carboxyméthyl-amino)-3-mercapto-propionylamino]-3-mercapto-propionic acid
- le [1-(1-Carbamoyl-2-mercapto-éthylcarbamoyl)-2-mercapto-éthylamino]-acetic acid
- le 2-(carboxyméthylamino)-3-mercapto-N-(2-mercapto-1,1-diméthyl-éthyl)-propionamide
- la N-[N-[N-(carboxyméthyl)-L-cystéinyl]-L-cystéinyl]-D-Valine
- le 2-Amino-4-[2-mercapto-1-(mercaptométhyl-carbamoyl)-éthylcarbamoyl]-butyric acid
- le 2-Amino-4-[2-mercapto-1-(2-mercapto-éthylcarbamoyl)-éthylcarbamoyl]-butyric acid
- le 2-Amino-4-[2-mercapto-1-(3-mercapto-propylcarbamoyl)-éthylcarbamoyl]-butyric acid
- le 2-Amino-4-[2-mercapto-1-(4-mercapto-butylcarbamoyl)-éthylcarbamoyl]-butyric acid
- la L-.gamma.-glutamyl-L-cystéinyl-L-Cystéine
- le 2-[2-(4-Amino-4-carboxy-butyrylamino)-3-mercapto-propionylamino]-4-mercapto-butyric acid
- le 2-Amino-4-[1-(1-carbamoyl-2-mercapto-éthylcarbamoyl)-2-mercapto-éthylcarbamoyl]-butyric acid
- le 2-Amino-4-[1-(1-diméthylcarbamoyl-2-mercapto-éthylcarbamoyl)-2-mercapto-éthylcarbamoyl]-butyric acid
- le 2-Amino-4-[2-mercapto-1-(2-mercapto-1,1-diméthyl-éthylcarbamoyl)-éthylcarbamoyl]-butyric acid
- le 2-Amino-4-[2-mercapto-1-(2-mercapto-1-éthyl-éthylcarbamoyl)-éthylcarbamoyl]-butyric acid
- le 2-(2-Amino-acétylamino)-3-mercapto-N-mercaptométhyl-propionamide
- le 2-(2-Amino-acétylamino)-3-mercapto-N-(2-mercapto-éthyl)-propionamide
- le 2-(2-Amino-acétylamino)-3-mercapto-N-(3-mercapto-propyl)-propionamide
- le 2-(2-Amino-acétylamino)-3-mercapto-N-(4-mercapto-butyl)-propionamide
- le 2-(2-Amino-acétylamino)-3-mercapto-N-(5-mercapto-pentyl)- propionamide
- la glycyl-L-cystéinyl-L-Cystéine
- le 2-[2-(2-Amino-acétylamino)-3-mercapto-propionylamino]-4-mercapto-butyric acid
- le 2-(2-Amino-acétylamino)-3-mercapto-N-(2-mercapto-1,1-diméthyl-éthyl)-propionamide
- le 2-(2-Amino-acétylamino)-3-mercapto-N-(1-mercaptométhyl-propyl)-propionamide
- la 4-carboxy-L-.alpha.-glutamyl-L-cystéinyl-L-Cystéine
- la (S)-N-[N-(5-amino-5-carboxy-1-oxopentyl)-L-homocystéinyl]-L-Cystéine
- le 4-Mercapto-N-mercaptométhyl-butyramide
- le 4-Mercapto-N-(2-mercapto-éthyl)-butyramide
- le 4-Mercapto-N-(3-mercapto-propyl)-butyramide
- le N-(4-mercapto-1-oxobutyl)-L-Cystéine
- le 4-Mercapto-2-(3-mercapto-propionylamino)-butyramide
- le N-(1-Diméthylcarbamoyl-2-mercapto-éthyl)-4-mercapto-butyramide
- le N-(4-mercapto-1-oxobutyl)-L-homocystéine
- le 4-Mercapto-N-(2-mercapto-1,1-diméthyl-éthyl)-butyramide
- le 4-Mercapto-N-(1-mercaptométhyl-propyl)-butyramide
- le 5-Mercapto-pentanoic acid mercaptométhyl-amide
- le 5-Mercapto-pentanoic acid (2-mercapto-éthyl)-amide
- le N-(5-mercapto-1-oxopentyl)-L-Cystéine
- le 5-Mercapto-2-(3-mercapto-propionylamino)-pentanamide
- le N-(1-Diméthylcarbamoyl-2-mercapto-éthyl)-5-mercapto-pentanamide
- le 5-Mercapto-N-(2-mercapto-1,1-diméthyl-éthyl)-pentanamide
- le 5-Mercapto-N-(1-mercaptométhyl-propyl)-pentanamide
- le 6-Mercapto-hexanoic acid mercaptométhyl-amide

On préfère tout particulièrement les composés suivants :
- le 2-mercapto-N-(mercaptométhyl)-acétamide
- le 2-mercapto-N-(2-mercaptoéthyl)-acétamide
- le 2-mercapto-N-(3-mercaptopropyl)-acétamide
- le 2-mercapto-N-(4-mercaptobutyl)-acétamide
- le 2-mercapto-N-(5-mercaptopentyl)-acétamide
- le 3-mercapto-2-(2-mercapto-acétylamino)-propionamide
- le 3-mercapto-2-(2-mercapto-acetylamino)-N,N-diméthyl-propionamide
- le 2-mercapto-N-(2-mercapto-1,1-diméthyl-éthyl)-acétamide
- le 2-mercapto-N-(1-mercaptométhyl-propyl)-acétamide
- la N-(mercaptoacétyl)-L-cystéine
- la N-(mercaptoacétyl)-L-Homocystéine
- le 2-Mercapto-N-mercaptométhyl-propionamide
- le 2-Mercapto-N-(2-mercapto-éthyl)-propionamide
- le 2-Mercapto-N-(3-mercapto-propyl)-propionamide
- le 2-Mercapto-N-(4-mercapto-butyl)-propionamide
- le 2-Mercapto-N-(5-mercapto-pentyl)-propionamide
- l'acide mercapto[(2-mercapto-1-oxopropyl)amino]-acétique
- la 2-mercaptopropionyl-L-cystéine
- le N-(1-Carbamoyl-2-mercapto-éthyl)-2-mercapto-propionamide
- le N-(1-Diméthylcarbamoyl-2-mercapto-éthyl)-2-mercapto-propionamide
- le 4-Mercapto-2-(2-mercapto-propionylamino)-butyric acid
- le 2-Mercapto-N-(2-mercapto-1,1-diméthyl-éthyl)-propionamide
- le 2-Mercapto-butanoic acid mercaptométhyl-amide
- le 2-Mercapto-butanoic acid (2-mercapto-éthyl)-amide
- le 2-Mercapto-butanoic acid (3-mercapto-propyl)-amide
- le 2-Mercapto-butanoic acid (4-mercapto-butyl)-amide
- le 2-Mercapto-pentanoic acid mercaptométhyl-amide
- le 2-Mercapto-pentanoic acid (2-mercapto-éthyl)-amide
- le 2-Mercapto-pentanoic acid (3-mercapto-propyl)-amide
- le 3-mercapto-N-(2-mercaptoéthyl)-propanamide
- le 3-Mercapto-N-mercaptométhyl-propionamide
- le 3-Mercapto-N-(3-mercapto-propyl)-propionamide
- le 3-Mercapto-N-(4-mercapto-butyl)-propionamide
- la N-(3-mercapto-1-oxopropyl)-L-Cystéine
- le 3-Mercapto-2-(3-mercapto-propionylamino)-propionamide
- le N-(1-Diméthylcarbamoyl-2-mercapto-éthyl)-3-mercapto-propionamide
- le 4-Mercapto-2-(3-mercapto-propionylamino)-butyric acid
- le 3-Mercapto-N-(2-mercapto-1,1-diméthyl-éthyl)-propionamide
- le 3-Mercapto-N-mercaptométhyl-2-méthyl-propionamide
- le 3-Mercapto-N-(2-mercaptoéthyl)-2-méthyl-propionamide
- le 3-Mercapto-N-(3-mercapto-propyl)-2-méthyl-propionamide
- le 3-Mercapto-N-(4-mercapto-butyl)- 2-méthyl-propionamide
- le 3-Mercapto-2-(3-mercapto-2-méthyl-propionylamino)-propionic acid
- le N-(1-Carbamoyl-2-mercapto-éthyl)-3-mercapto-2-méthyl-propionamide
- le N-(1-Diméthylcarbamoyl-2-mercapto-éthyl)-3-mercapto-2-méthyl-propionamide
- le 3-Mercapto-N-(2-mercapto-1,1-diméthyl-éthyl)- 2-méthyl-propionamide
- la N-(3-mercapto-2-méthyl-1-oxopropyl)-L-Homocystéine
- le N-mercaptométhyl-3-mercaptométhyl-succinamic acid
- le N-(2-Mercapto-éthyl)-3-mercaptométhyl-succinamic acid
- le N-(3-Mercapto-propyl)-3-mercaptométhyl-succinamic acid
- le N-(4-Mercapto-butyl)-3-mercaptométhyl-succinamic acid
- le N-(1-Carboxy-2-mercapto-éthyl)-3-mercaptométhyl-succinamic acid
- le 2-Mercapto-N-mercaptométhyl-2-méthyl-propionamide
- le 2-mercapto-N-(2-mercaptoéthyl)-2-méthyl-propanamide
- le 2-Mercapto-N-(3-mercapto-propyl)-2-méthyl-propionamide
- le 2-Mercapto-N-(4-mercapto-butyl)-2-méthyl-propionamide
- le 2-Mercapto-N-(2-mercapto-1,1-diméthyl-éthyl)-2-méthyl-propionamide
- le 2-Mercapto-N-(2-mercapto-1-éthyl)-2-méthyl-propionamide
- la N-(2-mercapto-2-méthyl-1-oxopropyl)-L-Cystéine
- la N-(2-éthyl-2-mercapto-1-oxobutyl)-L-Cystéine
- le 3-Mercapto-N-mercaptométhyl-3-méthyl-butyramide
- le 3-mercapto-N-(2-mercaptoéthyl)-3-méthyl-Butanamide
- le 3-Mercapto-N-(3-mercapto-propyl)-3-méthyl-bùtyramide
- la N-(3-mercapto-3-méthyl-1-oxobutyl)-L-Cystéine
- le N-(1-Carbamoyl-2-mercapto-éthyl)-3-mercapto-3-méthyl-butyramide
- le N-(1-Diméthylcarbamoyl-2-mercapto-éthyl)-3-mercapto-3-méthyl-butyramide
- le 4-Mercapto-2-(3-mercapto-3-méthyl-butyrylamino)-butyric acid
- le 3-Mercapto-N-mercaptométhyl-2,2-diméthyl-propionamide
- le 3-Mercapto-N-(2-mercaptoéthyl)-2,2-diméthyl-propionamide
- le 3-Mercapto-N-(3-mercapto-propyl)-2,2-diméthyl-propionamide
- le 3-Mercapto-N-(4-mercapto-butyl)- 2,2-diméthyl-propionamide
- la N-(3-mercapto-2,2-diméthyl-1-oxopropyl)-L-Cystéine
- le N-(1-Carbamoyl-2-mercapto-éthyl)-3-mercapto-2,2-diméthyl-propionamide
- le N-(1-Diméthylcarbamoyl-2-mercapto-éthyl)-3-mercapto-2,2-diméthyl-propionamide
- la N-[2-éthyl-2-(mercaptométhyl)-1-oxobutyl]-L-cystéine
- le 2-Amino-3-mercapto-N-mercaptométhyl-propionamide
- le (2R)- 2-amino-3-mercapto-N-(2-mercaptoéthyl)-Propanamide
- le (2S)-2-amino-3-mercapto-N-(2-mercaptoéthyl)-Propanamide
- le 2-Amino-3-mercapto-N-(3-mercapto-propyl)-propionamide
- le 2-Amino-3-mercapto-N-(4-mercapto-butyl)-propionamide
- la L-cystéinyl-L-Cystéine
- le 2-Amino-N-(1-carbamoyl-2-mercapto-éthyl)-3-mercapto-propionamide
- le 2-Amino-N-(1-carbamoyl-2-mercapto-éthyl)-3-mercapto-N,N-diméthyl-propionamide
- le 2-(2-Amino-3-mercapto-propionylamino)-4-mercapto-butyric acid
- le 2-Amino-3-mercapto-N-(2-mercapto-1,1-diméthyl-éthyl)-propionamide
- le 2-Amino-3-mercapto-N-(2-mercapto-1-éthyl)-propionamide
- le 2-Acétylamino-3-mercapto-N-mercaptométhyl-propionamide
- la N-acétyl-L-cystéinyl-L-Cystéine
- le 2-(carboxyméthylamino)-3-mercapto-N-mercaptométhyl-propionamide
- le 2-(carboxyméthylamino)-3-mercapto-N-(2-mercapto-éthyl)-propionamide
- le 2-(carboxyméthylamino)-3-mercapto-N-(3-mercapto-propyl)-propionamide
- la L-.gamma.-glutamyl-L-cystéinyl-L-Cystéine
- le 2-(2-Amino-acétylamino)-3-mercapto-N-mercaptométhyl-propionamide
- le 2-(2-Amino-acétylamino)-3-mercapto-N-(2-mercapto-éthyl)-propionamide
- la glycyl-L-cystéinyl-L-Cystéine
- la 4-carboxy-L-.alpha.-glutamyl-L-cystéinyl-L-Cystéine
- le 4-Mercapto-N-mercaptométhyl-butyramide
- le 4-Mercapto-N-(2-mercapto-éthyl)-butyramide
- le 4-Mercapto-N-(3-mercapto-propyl)-butyramide
- le N-(4-mercapto-1-oxobutyl)-L-Cystéine
- le 4-Mercapto-2-(3-mercapto-propionylamino)-butyramide
- le N-(1-Diméthylcarbamoyl-2-mercapto-éthyl)-4-mercapto-butyramide
- le 4-Mercapto-N-(2-mercapto-1,1-diméthyl-éthyl)-butyramide
- le 4-Mercapto-N-(1-mercaptométhyl-propyl)-butyramide
- le 5-Mercapto-pentanoic acid mercaptométhyl-amide
- le 5-Mercapto-pentanoic acid (2-mercapto-éthyl)-amide
- le N-(5-mercapto-1-oxopentyl)-L-Cystéine
- le 5-Mercapto-2-(3-mercapto-propionylamino)-pentanamide
- le N-(1-Diméthylcarbamoyl-2-mercapto-éthyl)-5-mercapto-pentanamide
- le 5-Mercapto-N-(2-mercapto-1,1-diméthyl-éthyl)-pentanamide
- le 5-Mercapto-N-(1-mercaptométhyl-propyl)-pentanamide
- le 6-Mercapto-hexanoic acid mercaptométhyl-amide.

Le procédé conforme à l'invention de déformation permanente des fibres kératiniques comprend l'application sur les fibres kératiniques d'une composition réductrice comprenant, en tant qu'agent réducteur, au moins un composé de formule (I), puis d'une composition oxydante (de fixation), avec ou sans étape intermédiaire
ou subséquente de rinçage ou d'application de composition intermédiaire. Les cheveux sont mis en forme en utilisant des moyens mécaniques bien connus de l'homme de l'art, comme des bigoudis, la composition réductrice étant appliquée avant ou après les moyens de mise en forme des cheveux.

Selon la présente invention, le procédé de déformation permanente des fibres kératiniques consiste, de préférence, dans une première étape, à réduire les liaisons disulfures de la kératine par application, pendant environ 5 à 60 min, d'une composition réductrice telle que définie ci-dessus puis, dans une seconde étape, à reformer lesdites liaisons par application d'une composition oxydante ou, éventuellement, en laissant agir l'oxygène de l'air.

De préférence, on applique une composition réductrice telle que définie ci-dessus sur des cheveux mouillés préalablement enroulés sur des rouleaux ayant de 2 à 30 mm de diamètre, la composition pouvant éventuellement être appliquée au fur et à mesure de l'enroulage des cheveux; on laisse ensuite agir la composition réductrice pendant un temps de 5 à 60 minutes, de préférence de 10 à 40 minutes, puis on rince abondamment ; après quoi on applique, sur les cheveux enroulés, une composition oxydante permettant de reformer les liaisons disulfures de la kératine pendant un temps de pose de 2 à 10 minutes. Après avoir enlevé les rouleaux, on rince abondamment la chevelure.

On peut également, après application de la composition réductrice, soumettre la chevelure à un traitement thermique par chauffage à une température comprise entre 30 et 60°C pendant tout ou partie du temps de pose. Dans la pratique, cette opération peut être effectuée à l'aide d'un casque de coiffure, d'un sèche cheveux, d'un dispensateur de rayons infrarouges et d'autres appareils chauffants classiques.

La composition d'oxydation ou oxydante est du type couramment utilisé et contient, par exemple, comme agent oxydant de l'eau oxygénée, un bromate alcalin, un persel, un polythionate ou un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 10. Cette oxydation peut être effectuée immédiatement ou être différée.

La déformation des fibres kératiniques selon l'invention peut également consister en un procédé de défrisage ou de décrêpage des fibres kératiniques, dans lequel on applique sur les fibres kératiniques une composition réductrice selon l'invention, puis l'on soumet les fibres kératiniques à une déformation mécanique permettant de les fixer dans leur nouvelle forme, par une opération de lissage des fibres kératiniques avec un peigne à larges dents, avec le dos d'un peigne ou à la main. Après un temps de pose de 5 à 60 minutes, en particulier, de 15 à 45 minutes, on procède alors à un nouveau lissage, puis on rince soigneusement et on applique une composition oxydante ou fixatrice telle que définie ci-dessus, qu'on laisse agir pendant environ 2 à 10 minutes, puis on rince abondamment les fibres kératiniques.

Dans chacun des procédés décrits ci-dessus, on peut intercaler une étape avec utilisation d'un fer chauffant entre 60 et 220°C, et de préférence entre 120 et 200°C.

L'invention a également pour objet une composition aqueuse réductrice, pour le premier temps d'une opération de déformation permanente des cheveux, contenant, en tant qu'agent réducteur un dimercaptoamide de formule (I) tel que décrit ci-dessus.

Dans les compositions de permanente selon l'invention, l'agent réducteur de formule générale (I) est généralement présent à une concentration comprise entre 0,05 et 35 % et, de préférence, entre 1 et 20 % en poids par rapport au poids total de la composition réductrice.

Le pH de la composition est de préférence compris entre 4 et 11, et plus particulièrement entre 6 et 10 et peut être obtenu à l'aide d'un agent alcalin tel que, par exemple, l'ammoniaque, la monoéthanolamine, la diéthanolamine, la triéthanolamine, l'isopropylamine, la propanediamine-1,3, un carbonate ou bicarbonate alcalin ou d'ammonium, un carbonate organique tel que le carbonate de guanidine, un hydroxyde alcalin ou à l'aide d'un agent acidifiant tel que par exemple l'acide chlorhydrique, l'acide acétique, l'acide lactique, l'acide oxalique ou l'acide borique ou bien encore à l'aide de tampons usuels, tels que les tampons borate, phosphate, TRIS ou autres.

La composition réductrice peut également contenir d'autres agents réducteurs connus, tels que par exemple l'acide thioglycolique ou l'acide thiolactique et leurs dérivés esters et amides, notamment le monothioglycolate de glycérol, la cystéamine et ses dérivés acylés en C1-C4 tels que la N-acétyl-cystéamine ou la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides décrits dans la demande de brevet EP-A-354 835, les N-mono ou N,N-dialkylmercapto-4-butyramides décrits dans le demande de brevet EP-A-368 763, les aminomercaptoalkylamides, décrits dans le demande de brevet EP-A-432 000, les dérivés des acides N-(mercaptoalkyl)succinamiques et des N-(mercaptoalkyl)succinimides décrits dans le demande de brevet EP-A-465-342, les alkylamino mercaptoalkylamides décrits dans la demande de brevet EP-A-514 282, le mélange azéotrope de thioglycolate de 2-hydroxypropyle et de thioglycolate de (2-hydroxy-1-méthyl)éthyle décrits dans la demande de brevet FR-A-2 679 448, les mercaptoalkylaminoamides décrits dans la demande de brevet FR-A-2 692 481, les N-mercaptoalkylalcanediamides décrits dans le demande de brevet EP-A-653 202, ainsi que les dérivés d'acide formamidine sulfinique décrits dans la demande PCT/US01/43124, déposée par la Demanderesse.

Selon un mode de réalisation préféré, la composition réductrice contient en outre au moins un agent tensioactif de type non-ionique, anionique, cationique ou amphotère, ou un mélange, et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, et de préférence, comprise entre 0,5 et 10 % en poids par rapport au poids total de la composition réductrice.

Dans le but d'améliorer les propriétés cosmétiques des cheveux ou encore d'en atténuer ou d'éviter leur dégradation, la composition réductrice peut également contenir au moins un agent traitant de nature cationique, anionique, non-ionique ou amphotère, ou un mélange.

Parmi les agents traitants particulièrement préférés, on peut notamment citer ceux décrits dans les brevets français n° 2.598.613 et n° 2.470.596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2.535.730, les polyorganosiloxanes à groupements aminoalkyles modifiés par des groupements alcoxycarbonyalkyles tels que ceux décrits dans le brevet US n° 4.749.732, des polyorganosiloxanes tels que le copolymère polydiméthylsiloxane- polyoxyalkyle du type Diméthicone Copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

La composition réductrice peut également contenir d'autres ingrédients traitants tels que des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n°83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO2/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C3-C6 tels que par exemple le propanediol-1,2, le propanediol-1,3 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs, des épaississants, des gélifiants.

La composition réductrice selon l'invention se présente essentiellement sous forme aqueuse, notamment sous la forme d'une lotion épaissie ou non, d'une crème ou d'un gel.

La composition réductrice selon l'invention peut être du type exothermique, c'est-à-dire provoquant un certain échauffement lors de l'application sur les cheveux, ce qui apporte un agrément à la personne qui subit le premier temps de la permanente ou du défrisage.

La composition réductrice selon l'invention peut également comprendre un solvant tel qu'un monoalcool en C1-C6, notamment l'éthanol, le propanol, l'isopropanol ou le butanol, et/ou un polyol tel que le glycérol, qui peut être présent à raison de 0,01 à 20% en poids, notamment 0,1 à 15% en poids, par rapport au poids total de la composition.

Le véhicule des compositions selon l'invention est de préférence l'eau ou une solution hydroalcoolique comprenant de l'eau et un solvant tel que défini ci-dessus.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables ou d'alcools gras.

On peut également utiliser des liquides ou des gels contenant des agents épaississants tels que des polymères ou des copolymères carboxyvinyliques qui "collent" les cheveux et les maintiennent dans la position lisse pendant le temps de pose.

L'invention concerne également un kit, notamment, pour la déformation permanente des cheveux comprenant, dans un premier compartiment, en tant que composition réductrice, une composition conforme à l'invention comprenant un composé de formule (I), et dans un second compartiment, une composition oxydante.

La présente invention a également pour objet, à titre de composés nouveaux, les dimercaptoamides répondant à la formule générale (II) suivante :

HS-A-CO-NH-B-SH (II)

dans laquelle :
(i) A représente un radical CH2 et B représente un radical (CH2)x dans lequel x = 2, 3, 4 ou 5 ; un radical C(CH3)2-CH2 ; un radical CH(Et)-CH2 ; un radical CH(CO-NRIR2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(ii) A représente un radical CH(CH3) et B représente un radical (CH2)x dans lequel x = 1, 2, 3, 4 ou 5 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(iii) A représente un radical CH-R3 dans lequel R3 désigne un radical alkyle, linéaire ou ramifié, en C2-C5 et B représente un radical (CH2)x dans lequel x = 1, 2, 3, 4 ou 5 ; un radical C(CH3)2-CH2 ; un radical CH(Et)-CH2 ; un radical CH(COOH)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(iv) A représente un radical CH2-CH2 ou C(CH3)2-CH2 et B représente un radical (CH2)x dans lequel x = 1, 3 ou 4; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(v) A représente un radical CH2-CH(CH3) et B représente un radical (CH2)x dans lequel x = 1, 2, 3 ou 4 ; un radical C(CH3)2-CH2 ; un radical CH(Et)-CH2 ; un radical CH(COOH)-CH2 ; un radical CH(CO-NRIR2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(vi) A représente un radical CH2-CH-R4 dans lequel R4 désigne un radical phényle ou COO-CH3 ou COO-CH2-CH3 et B représente un radical (CH2)x dans lequel x = 1, 2, 3 ou 4 ; un radical C(CH3)2-CH2 ; un radical CH(Et)-CH2 ; un radical CH(COOH)-CH2-CH2 ; un radical CH(CO-NRIR2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(vii) A représente un radical CH2-CH(CH2-COOH) ou CH2-CH(NH-CH2-COOH) et B représente un radical (CH2)x dans lequel x = 1, 2, 3 ou 4 ; un radical C(CH3)2-CH2 ; un radical CH(Et)-CH2 ; un radical CH(COOH)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(viii) A représente un radical C(CH3)2 et B représente un radical (CH2)x dans lequel x = 1, 3, 4 ou 5 ; un radical C(CH3)2-CH2 ; un radical CH(Et)-CH2 ; un radical CH(CO-NHCH3)-CH2 ; un radical CH(CO-NHEt)-CH2 ; un radical CH(CO-NH-CH2-CHOH-CH3)-CH2 ; ou CH(CO-NR1R2)-CH2-CH2 dans lequel R1 et R2 représentent, indépendamment l'un de l'autre, un atome d'hydrogène ou un radical méthyle, éthyle ou 2-hydroxypropyle
(ix) A représente un radical C(Et)2 et B représente un radical (CH2)x dans lequel x = 1, 2, 3, 4 ou 5 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(x) A représente un radical CH2-C(CH3)2 ou CH(CH2-Ph) et B représente un radical (CH2)x dans lequel x = 1, 2, 3 ou 4 ; un radical C(CH3)2-CH2 ; un radical CH(Et)-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xi) A représente un radical CH2-C(Et)2 et B représente un radical (CH2)x dans lequel x = 1, 2, 3 ou 4 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xii) A représente un radical CH2-CH(NH2) et B représente un radical (CH2)x dans lequel x = 1, 3 ou 4 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xiii) A représente un radical CH2-CH(NHAc) et B représente un radical (CH2)x dans lequel x = 1, 3 ou 4 ; un radical C(CH3)2-CH2 ; un radical CH(Et)-CH2 ; un radical CH(COOH)-CH2-CH2 ; un radical CH(CO-NH-CH3)-CH2 ; un radical CH(CO-NH-Et)-CH2 ; un radical CH(CO-N(CH3)2)-CH2 ; un radical CH(CO-NR1R2)-CH2-CH2 dans lequel R1 et R2 représentent, indépendamment l'un de l'autre, un atome d'hydrogène ou un radical méthyle, éthyle ou 2-hydroxypropyle
(xiv) A représente un radical CH2-CH(NH-CO-CH2-CH2-CH(NH2)COOH) ou CH2-CH(NH-CO-CH2-NH2) ou CH2-CH(NH-CO-CH(NH2)-CH2-CH(COOH)2 ou CH2-CH(NH-CO-CH2-CH2-CH2-CH(NH2)COOH) et B représente un radical (CH2)x dans lequel x = 1, 2, 3 ou 4 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xv) A représente un radical (CH2)3 et B représente un radical (CH2)x dans lequel x = 1, 2 ou 3; un radical C(CH3)2-CH2 ; un radical CH(Et)-CH2 ; un radical CH(COOH)-CH2 ; un radical CH(COOH)-CH2-CH2 ; un radical CH(CO-NRIR2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xvi) A représente un radical (CH2)4 et B représente un radical (CH2)x dans lequel x = 1 ou 2; un radical C(CH3)2-CH2 ; un radical CH(Et)-CH2 ; un radical CH(COOH)-CH2 ; un radical CH(COOH)-CH2-CH2 ; un radical CH(CO-NRIR2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xvii) A représente un radical (CH2)5 et B représente un radical CH2
(xviii) A représente un radical CH-Ph et B représente un radical (CH2)x dans lequel x = 1, 2, 3,4 ou 5; un radical C(CH3)2-CH2 ; un radical CH(Et)-CH2 ; un radical CH(COOH)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
et les sels organiques et minéraux desdits composés de formule (II).

Les composés de formule générale (II) peuvent être utilisés de la même façon que les composés de formule générale (I) mentionnés ci-dessus.

Parmi les composés de formule générale (II), on peut notamment citer les composés préférés ci-après :
- le 2-Mercapto-N-(2-mercapto-éthyl)-acétamide
- le 2-Mercapto-N-(3-mercapto-propyl)-acétamide
- le 2-Mercapto-N-(4-mercapto-butyl)-acétamide
- le 2-Mercapto-N-(5-mercapto-pentyl)-acétamide
- le 3-Mercapto-2-(2-mercapto-acétylamino)-propionamide
- le 3-Mercapto-2-(2-mercapto-acétylamino)-N,N-diméthyl-propionamide
- le 2-Mercapto-N-(2-mercapto-1,1-diméthyl-éthyl)-acétamide
- le 2-Mercapto-N-(1-mercaptométhyl-propyl)-acétamide
- le 2-Mercapto-N-mercaptométhyl-propionamide
- le 2-Mercapto-N-(2-mercapto-éthyl)-propionamide
- le 2-Mercapto-N-(3-mercapto-propyl)-propionamide
- le 2-Mercapto-N-(4-mercapto-butyl)-propionamide
- le 2-Mercapto-N-(5-mercapto-pentyl)-propionamide
- le N-(1-Carbamoyl-2-mercapto-éthyl)-2-mercapto-propionamide
- le N-(1-Diméthylcarbamoyl-2-mercapto-éthyl)-2-mercapto-propionamide
- le 4-Mercapto-2-(2-mercapto-propionylamino)-butyric acid
- le 2-Mercapto-N-(2-mercapto-1,1-diméthyl-éthyl)-propionamide
- le 2-Mercapto-butanoic acid mercaptométhyl-amide
- le 2-Mercapto-butanoic acid (2-mercapto-éthyl)-amide
- le 2-Mercapto-butanoic acid (3-mercapto-propyl)-amide
- le 2-Mercapto-butanoic acid (4-mercapto-butyl)-amide
- le 2-Mercapto-butanoic acid (5-mercapto-pentyl)-amide
- le 2-Mercapto-butanoic acid (1-carbamoyl-2-mercapto-éthyl)-amide
- le N-(1-Diméthylcarbamoyl-2-mercapto-éthyl)-2-mercapto-butyramide
- le 2-Mercapto-butanoic acid (1-carbamoyl-3-mercapto-propyl)-amide
- le 2-Mercapto-butanoic acid (2-mercapto-1,1-diméthyl-éthyl)-amide
- le 2-Mercapto-pentanoic acid mercaptométhyl-amide
- le 2-Mercapto-pentanoic acid (2-mercapto-éthyl)-amide
- le 2-Mercapto-pentanoic acid (3-mercapto-propyl)-amide
- le 2-Mercapto-pentanoic acid (4-mercapto-butyl)-amide
- le 2-Mercapto-pentanoic acid (5-mercapto-pentyl)-amide
- le 3-Mercapto-2-(2-mercapto-pentanoylamino)-propionic acid
- le 2-Mercapto-pentanoic acid (1-carbamoyl-2-mercapto-éthyl)-amide
- le 2-Mercapto-pentanoic acid (1-diméthylcarbamoyl-2-mercapto-éthyl)-amide
- le 2-Mercapto-pentanoic acid (1-carbamoyl-3-mercapto-propyl)-amide
- le 2-Mercapto-pentanoic acid (2-mercapto-1,1-diméthyl-éthyl)-amide
- le 3-Mercapto-N-mercaptométhyl-propionamide
- le 3-Mercapto-N-(3-mercapto-propyl)-propionamide
- le 3-Mercapto-N-(4-mercapto-butyl)-propionamide
- le 3-Mercapto-2-(3-mercapto-propionylamino)-propionamide
- le N-(1-Diméthylcarbamoyl-2-mercapto-éthyl)-3-mercapto-propionamide
- le 4-Mercapto-2-(3-mercapto-propionylamino)-butyric acid
- le 3-Mercapto-N-(2-mercapto-1,1-diméthyl-éthyl)-propionamide
- le 3-Mercapto-N-mercaptométhyl-2-méthyl-propionamide
- le 3-Mercapto-N-(2-mercaptoéthyl)-2-méthyl-propionamide
- le 3-Mercapto-N-(3-mercapto-propyl)-2-méthyl-propionamide
- le 3-Mercapto-N-(4-mercapto-butyl)- 2-méthyl-propionamide
- le 3-Mercapto-2-(3-mercapto-2-méthyl-propionylamino)-propionic acid
- le N-(1-Carbamoyl-2-mercapto-éthyl)-3-mercapto-2-méthyl-propionamide
- le N-(1-Diméthylcarbamoyl-2-mercapto-éthyl)-3-mercapto-2-méthyl-propionamide
- le 3-Mercapto-N-(2-mercapto-1,1-diméthyl-éthyl)- 2-méthyl-propionamide
- le N-mercaptométhyl-3-mercaptométhyl-succinamic acid
- le N-(2-Mercapto-éthyl)-3-mercaptométhyl-succinamic acid
- le N-(3-Mercapto-propyl)-3-mercaptométhyl-succinamic acid
- le N-(4-Mercapto-butyl)-3-mercaptométhyl-succinamic acid
- le N-(1-Carboxy-2-mercapto-éthyl)-3-mercaptométhyl-succinamic acid
- le N-(1-Carboxy-3-mercapto-propyl)-3-mercaptométhyl-succinamic acid
- le N-(2-Mercapto-1,1-diméthyl-éthyl)-3-mercaptométhyl-succinamic acid
- le N-(2-Mercapto-1-éthyl)-3-mercaptométhyl-succinamic acid
- le 2-Mercapto-N-mercaptométhyl-2-méthyl-propionamide
- le 2-Mercapto-N-(3-mercapto-propyl)-2-méthyl-propionamide
- le 2-Mercapto-N-(4-mercapto-butyl)-2-méthyl-propionamide
- le 2-Mercapto-N-(5-mercapto-pentyl)-2-méthyl-propionamide
- le 2-Mercapto-N-(2-mercapto-1,1-diméthyl-éthyl)-2-méthyl-propionamide
- le 2-Mercapto-N-(2-mercapto-1-éthyl)-2-méthyl-propionamide
- le 3-Mercapto-N-mercaptométhyl-3-méthyl-butyramide
- le 3-Mercapto-N-(3-mercapto-propyl)-3-méthyl-butyramide
- le 3-Mercapto-N-(4-mercapto-butyl)-3-méthyl-butyramide
- le N-(1-Carbamoyl-2-mercapto-éthyl)-3-mercapto-3-méthyl-butyramide
- le N-(1-Diméthylcarbamoyl-2-mercapto-éthyl)-3-mercapto-3-méthyl-butyramide
- le 4-Mercapto-2-(3-mercapto-3-méthyl-butyrylamino)-butyric acid
- le 3-Mercapto-N-(2-mercapto-1,1-diméthyl-éthyl)-3-méthyl-butyramide
- le 3-Mercapto-N-(2-mercapto-1-éthyl)-3-méthyl-butyramide
- le 3-Mercapto-N-mercaptométhyl-2,2-diméthyl-propionamide
- le 3-Mercapto-N-(2-mercaptoéthyl)-2,2-diméthyl-propionamide
- le 3-Mercapto-N-(3-mercapto-propyl)-2,2-diméthyl-propionamide
- le 3-Mercapto-N-(4-mercapto-butyl)- 2,2-diméthyl-propionamide
- le 3-Mercapto-N-(2-mercapto-1,1-diméthyl-éthyl)- 2,2-diméthyl-propionamide
- le N-(1-Carbamoyl-2-mercapto-éthyl)-3-mercapto-2,2-diméthyl-propionamide
- le N-(1-Diméthylcarbamoyl-2-mercapto-éthyl)-3-mercapto-2,2-diméthyl-propionamide
- le 2-Amino-3-mercapto-N-mercaptométhyl-propionamide
- le 2-Amino-3-mercapto-N-(3-mercapto-propyl)-propionamide
- le 2-Amino-3-mercapto-N-(4-mercapto-butyl)-propionamide
- le 2-Amino-N-(1-carbamoyl-2-mercapto-éthyl)-3-mercapto-propionamide
- le 2-Amino-N-(1-carbamoyl-2-mercapto-éthyl)-3-mercapto-N,N-diméthyl-propionamide
- le 2-(2-Amino-3-mercapto-propionylamino)-4-mercapto-butyric acid
- le 2-Amino-3-mercapto-N-(2-mercapto-1,1-diméthyl-éthyl)-propionamide
- le 2-Amino-3-mercapto-N-(2-mercapto-1-éthyl)-propionamide
- le 2-Acétylamino-3-mercapto-N-mercaptométhyl-propionamide
- le 2-Acétylamino-3-mercapto-N-(3-mercapto-propyl)-propionamide
- le 2-Acétylamino-3-mercapto-N-(4-mercapto-butyl)-propionamide
- le 2-(2-Acétylamino-3-mercapto-propionylamino)-3-mercapto-3-méthyl-butyric acid
- le 2-Acétylamino-3-mercapto-N-(1-mercaptométhyl-propyl)-propionamide
- le 2-Acétylamino-N-(1-diméthylcarbamoyl-2-mercapto-éthyl)-3-mercapto-propionamide
- le 2-(2-Acétylamino-3-mercapto-propionylamino)-4-mercapto-butyric acid
- le 2-(carboxyméthylamino)-3-mercapto-N-mercaptométhyl-propionamide
- le 2-(carboxyméthylamino)-3-mercapto-N-(2-mercapto-éthyl)-propionamide
- le 2-(carboxyméthylamino)-3-mercapto-N-(3-mercapto-propyl)-propionamide
- le 2-(carboxyméthylamino)-3-mercapto-N-(4-mercapto-butyl)-propionamide
- le 2-(carboxyméthylamino)-N-(1-carbamoyl-2-mercapto-éthyl)-3-mercapto- propionamide
- le 2-[2-(Carboxyméthyl-amino)-3-mercapto-propionylamino]-3-mercapto-propionic acid
- le [1-(1-Carbamoyl-2-mercapto-éthylcarbamoyl)-2-mercapto-éthylamino]-acetic acid
- le 2-(carboxyméthylamino)-3-mercapto-N-(2-mercapto-1,1-diméthyl-éthyl)-propionamide
- le 2-Amino-4-[2-mercapto- 1 -(mercaptométhyl-carbamoyl)-éthylcarbamoyly-butyric acid
- le 2-Amino-4-[2-mercapto-1-(2-mercapto-éthylcarbamoyl)-éthylcarbamoyl]-butyric acid
- le 2-Amino-4-[2-mercapto-1-(3-mercapto-propylcarbamoyl)-éthylcarbamoyll-butyric acid
- le 2-Amino-4-[2-mercapto-1-(4-mercapto-butylcarbamoyl)-éthylcarbamoyl]-butyric acid
- le 2-[2-(4-Amino-4-carboxy-butyrylamino)-3-mercapto-propionylamino]-4-mercapto-butyric acid
- le 2-Amino-4-[1-(1-carbamoyl-2-mercapto-éthylcarbamoyl)-2-mercapto-éthylcarbamoyl]-butyric acid
- le 2-Amino-4-[1-(1-diméthylcarbamoyl-2-mercapto-éthylcarbamoyl)-2-mercapto-éthylcarbamoyl]-butyric acid
- le 2-Amino-4-[2-mercapto-1-(2-mercapto-1,1-diméthyl-éthylcarbamoyl)-éthylcarbamoyl]-butyric acid
- le 2-Amino-4-[2-mercapto-1-(2-mercapto-1-éthyl-éthylcarbamoyl)-éthylcarbamoyl]-butyric acid
- le 2-(2-Amino-acétylamino)-3-mercapto-N-mercaptométhyl-propionamide
- le 2-(2-Amino-acétylamino)-3-mercapto-N-(2-mercapto-éthyl)-propionamide
- le 2-(2-Amino-acétylamino)-3-mercapto-N-(3-mercapto-propyl)-propionamide
- le 2-(2-Amino-acétylamino)-3-mercapto-N-(4-mercapto-butyl)-propionamide
- le 2-(2-Amino-acétylamino)-3-mercapto-N-(5-mercapto-pentyl)- propionamide
- le 2-[2-(2-Amino-acétylamino)-3-mercapto-propionylamino]-4-mercapto-butyric acid
- le 2-(2-Amino-acétylamino)-3-mercapto-N-(2-mercapto-1,1-diméthyl-éthyl)-propionamide
- le 2-(2-Amino-acétylamino)-3-mercapto-N-(1-mercaptométhyl-propyl)-propionamide
- le 4-Mercapto-N-mercaptométhyl-butyramide
- le 4-Mercapto-N-(2-mercapto-éthyl)-butyramide
- le 4-Mercapto-N-(3-mercapto-propyl)-butyramide
- le N-(4-mercapto-1-oxobutyl)-L-Cystéine
- le 4-Mercapto-2-(3-mercapto-propionylamino)-butyramide
- le N-(1-Diméthylcarbamoyl-2-mercapto-éthyl)-4-mercapto-butyramide
- le N-(4-mercapto-1-oxobutyl)-L-homocystéine
- le 4-Mercapto-N-(2-mercapto-1,1-diméthyl-éthyl)-butyramide
- le 4-Mercapto-N-(1-mercaptométhyl-propyl)-butyramide
- le 5-Mercapto-pentanoic acid mercaptométhyl-amide
- le 5-Mercapto-pentanoic acid (2-mercapto-éthyl)-amide
- le N-(5-mercapto-1-oxopentyl)-L-Cystéine
- le 5-Mercapto-2-(3-mercapto-propionylamino)-pentanamide
- le N-(1-Diméthylcarbamoyl-2-mercapto-éthyl)-5-mercapto-pentanamide
- le 5-Mercapto-N-(2-mercapto-1,1-diméthyl-éthyl)-pentanamide
- le 5-Mercapto-N-(1-mercaptométhyl-propyl)-pentanamide
- le 6-Mercapto-hexanoic acid mercaptométhyl-amide.

On préfère tout particulièrement les composés suivants :
- le 2-Mercapto-N-(2-mercapto-éthyl)-acétamide
- le 2-Mercapto-N-(3-mercapto-propyl)-acétamide
- le 2-Mercapto-N-(4-mercapto-butyl)-acétamide
- le 2-Mercapto-N-(5-mercapto-pentyl)-acétamide
- le 3-Mercapto-2-(2-mercapto-acétylamino)-propionamide
- le 3-Mercapto-2-(2-mercapto-acétylamino)-N,N-diméthyl-propionamide
- le 2-Mercapto-N-(2-mercapto-1,1-diméthyl-éthyl)-acétamide
- le 2-Mercapto-N-(1-mercaptométhyl-propyl)-acétamide
- le 2-Mercapto-N-mercaptométhyl-propionamide
- le 2-Mercapto-N-(2-mercapto-éthyl)-propionamide
- le 2-Mercapto-N-(3-mercapto-propyl)-propionamide
- le 2-Mercapto-N-(4-mercapto-butyl)-propionamide
- le 2-Mercapto-N-(5-mercapto-pentyl)-propionamide
- le N-(1-Carbamoyl-2-mercapto-éthyl-)-2-mercapto-propionamide
- le N-(1-Diméthylcarbamoyl-2-mercapto-éthyl)-2-mercapto-propionamide
- le 4-Mercapto-2-(2-mercapto-propionylamino)-butyric acid
- le 2-Mercapto-N-(2-mercapto-1,1-diméthyl-éthyl)-propionamide
- le 2-Mercapto-butanoic acid mercaptométhyl-amide
- le 2-Mercapto-butanoic acid (2-mercapto-éthyl)-amide
- le 2-Mercapto-butanoic acid (3-mercapto-propyl)-amide
- le 2-Mercapto-butanoic acid (4-mercapto-butyl)-amide
- le 2-Mercapto-pentanoic acid mercaptométhyl-amide
- le 2-Mercapto-pentanoic acid (2-mercapto-éthyl)-amide
- le 2-Mercapto-pentanoic acid (3-mercapto-propyl)-amide
- le 3-Mercapto-N-mercaptométhyl-propionamide
- le 3-Mercapto-N-(3-mercapto-propyl)-propionamide
- le 3-Mercapto-2-(3-mercapto-propionylamino)-propionamide
- le N-(1-Diméthylcarbamoyl-2-mercapto-éthyl)-3-mercapto-propionamide
- le 4-Mercapto-2-(3-mercapto-propionylamino)-butyric acid
- le 3-Mercapto-N-(2-mercapto-1,1-diméthyl-éthyl)-propionamide
- le 3-Mercapto-N-mercaptométhyl-2-méthyl-propionamide
- le 3-Mercapto-N-(2-mercaptoéthyl)-2-méthyl-propionamide
- le 3-Mercapto-N-(3-mercapto-propyl)-2-méthyl-propionamide
- le 3-Mercapto-N-(4-mercapto-butyl)- 2-méthyl-propionamide
- le 3-Mercapto-2-(3-mercapto-2-méthyl-propionylamino)-propionic acid
- le N-(1-Carbamoyl-2-mercapto-éthyl)-3-mercapto-2-méthyl-propionamide
- le N-(1-Diméthylcarbamoyl-2-mercapto-éthyl)-3-mercapto-2-méthyl-propionamide
- le 3-Mercapto-N-(2-mercapto-1,1-diméthyl-éthyl)- 2-méthyl-propionamide
- le N-mercaptométhyl-3-mercaptométhyl-succinamic acid
- le N-(2-Mercapto-éthyl)-3-mercaptométhyl-succinamic acid
- le N-(3-Mercapto-propyl)-3-mercaptométhyl-succinamic acid
- le N-(4-Mercapto-butyl)-3-mercaptométhyl-succinamic acid
- le N-(1-Carboxy-2-mercapto-éthyl)-3-mercaptométhyl-succinamic acid
- le 2-Mercapto-N-mercaptométhyl-2-méthyl-propionamide
- le 2-Mercapto-N-(3-mercapto-propyl)-2-méthyl-propionamide
- le 2-Mercapto-N-(4-mercapto-butyl)-2-méthyl-propionamide
- le 2-Mercapto-N-(2-mercapto-1,1-diméthyl-éthyl)-2-méthyl-propionamide
- le 2-Mercapto-N-(2-mercapto-1-éthyl)-2-méthyl-propionamide
- le 3-Mercapto-N-mercaptométhyl-3-méthyl-butyramide
- le 3-Mercapto-N-(3-mercapto-propyl)-3-méthyl-butyramide
- le N-(1-Carbamoyl-2-mercapto-éthyl)-3-mercapto-3-méthyl-butyramide
- le N-(1-Diméthylcarbamoyl-2-mercapto-éthyl)-3-mercapto-3-méthyl-butyramide
- le 4-Mercapto-2-(3-mercapto-3-méthyl-butyrylamino)-butyric acid
- le 3-Mercapto-N-mercaptométhyl-2,2-diméthyl-propionamide
- le 3-Mercapto-N-(2-mercaptoéthyl)-2,2-diméthyl-propionamide
- le 3-Mercapto-N-(3-mercapto-propyl)-2,2-diméthyl-propionamide
- le 3-Mercapto-N-(4-mercapto-butyl)- 2,2-diméthyl-propionamide
- le N-(1-Carbamoyl-2-mercapto-éthyl)-3-mercapto-2,2-diméthyl-propionamide
- le N-(1-Diméthylcarbamoyl-2-mercapto-éthyl)-3-mercapto-2,2-diméthyl-propionamide
- le 2-Amino-3-mercapto-N-mercaptométhyl-propionamide
- le 2-Amino-3-mercapto-N-(3-mercapto-propyl)-propionamide
- le 2-Amino-3-mercapto-N-(4-mercapto-butyl)-propionamide
- le 2-Amino-N-(1-carbamoyl-2-mercapto-éthyl)-3-mercapto-propionamide
- le 2-Amino-N-(1-carbamoyl-2-mercapto-éthyl)-3-mercapto-N,N-diméthyl-propionamide
- le 2-(2-Amino-3-mercapto-propionylamino)-4-mercapto-butyric acid
- le 2-Amino-3-mercapto-N-(2-mercapto-1,1-diméthyl-éthyl)-propionamide
- le 2-Amino-3-mercapto-N-(2-mercapto-1-éthyl)-propionamide
- le 2-Acétylamino-3-mercapto-N-mercaptométhyl-propionamide
- le 2-(carboxyméthylamino)-3-mercapto-N-mercaptométhyl-propionamide
- le 2-(carboxyméthylamino)-3-mercapto-N-(2-mercapto-éthyl)-propionamide
- le 2-(carboxyméthylamino)-3-mercapto-N-(3-mercapto-propyl)-propionamide
- le 2-(2-Amino-acétylamino)-3-mercapto-N-mercaptométhyl-propionamide
- le 2-(2-Amino-acétylamino)-3-mercapto-N-(2-mercapto-éthyl)-propionamide
- le 4-Mercapto-N-mercaptométhyl-butyramide
- le 4-Mercapto-N-(2-mercapto-éthyl)-butyramide
- le 4-Mercapto-N-(3-mercapto-propyl)-butyramide
- le N-(4-mercapto-1-oxobutyl)-L-Cystéine
- le 4-Mercapto-2-(3-mercapto-propionylamino)-butyramide
- le N-(1-Diméthylcarbamoyl-2-mercapto-éthyl)-4-mercapto-butyramide
- le 4-Mercapto-N-(2-mercapto-1,1-diméthyl-éthyl)-butyramide
- le 4-Mercapto-N-(1-mercaptométhyl-propyl)-butyramide
- le 5-Mercapto-pentanoic acid mercaptométhyl-amide
- le 5-Mercapto-pentanoic acid (2-mercapto-éthyl)-amide
- le N-(5-mercapto-1-oxopentyl)-L-Cystéine
- le 5-Mercapto-2-(3-mercapto-propionylamino)-pentanamide
- le N-(1-Diméthylcarbamoyl-2-mercapto-éthyl)-5-mercapto-pentanamide
- le 5-Mercapto-N-(2-mercapto-1,1-diméthyl-éthyl)-pentanamide
- le 5-Mercapto-N-(1-mercaptométhyl-propyl)-pentanamide
- le 6-Mercapto-hexanoic acid mercaptométhyl-amide.

L'invention a également pour objet des procédés de préparation des nouveaux dimercaptoamides de formule générale (II).

Les dimercaptoamides de formule générale (II) peuvent être préparés par un procédé comprenant a) la réaction d'un mercapto halogénure d'acide protégé de formule P-S-A-CO-X, avec un aminothiol protégé de formule H₂N-B-S-P, P étant un groupe protecteur de la fonction thiol tel que par exemple un groupe trityle ou acétyle ou benzyle, et X étant le chlore ou le brome, dans un solvant aprotique, en présence d'un agent destiné à capter l'acide chlorhydrique ou bromhydrique libéré, tel que par exemple une amine tertiaire, de façon à former un dipercaptoamide protégé de formule P-S-A-CO-NH-B-S-P, et b) la déprotection du dipercaptoamide protégé, par hydrolyse ou par réduction.

Ils peuvent également être préparés par un procédé comprenant la réaction d'un mercaptoacide de formule HS-A-COOH avec un aminothiol de formule H₂N-B-SH, en présence d'un agent de couplage et/ou de déshydratation, dans un solvant aprotique.

Ils peuvent également être préparés par un procédé comprenant la réaction d'un mercapto-ester de formule HS-A-COOR, R étant un radical méthyle ou éthyle, avec un aminothiol de formule H₂N-B-SH, à une température comprise entre 30°C et 180°C.

Ils peuvent également être préparés par un procédé comprenant
a) la réaction d'un halogénure d'acide halogéné de formule X-A-COCl avec une halogénoamine de formule H₂N-B-X, X représentant un atome de chlore ou de brome, dans un solvant aprotique et en présence d'un agent destiné à capter l'acide chlorhydrique ou bromhydrique libéré, de façon à former un dihalogénoamide de formule X-A-CO-NH-B-X, puis b) la transformation du dihalogénoamide obtenu en dimercaptoamide de formule (II), soit par réaction directe avec de l'hydrogène sulfuré dans un solvant approprié, soit par réaction avec l'acide thioacétique, de préférence sous forme salifiée, suivie d'une hydrolyse acide ou alcaline du di(S-acétyl)mercaptoamide, soit encore par réaction avec la thiourée suivie d'une hydrolyse du di sel d'isothiouronium.

Ils peuvent également être préparés par réaction d'une thiolactone de formule 11 avec un aminothiol de formule 5, dans un solvant aprotique, selon la réaction suivante :

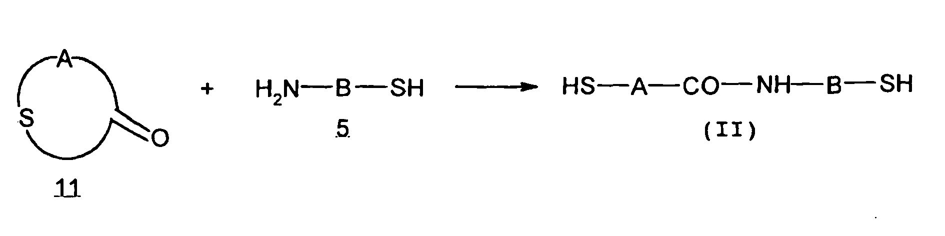

Enfin, ils peuvent être préparés par un procédé comprenant la réaction d'un mercaptoamide de formule HS-A-CO-NH₂ avec un sel d'aminothiol de formule HX, H₂N-B-SH, X représentant un atome de chlore ou brome, à une température comprise entre 60 et 200°C.

Dans les procédés qui viennent d'être décrits, les groupes A et B ont la même signification que les groupes de la formule (II) mentionnée plus haut.

Les nouveaux dimercaptoamides de formule générale (II) peuvent ainsi être préparés en suivant les procédés décrits ci-dessous :

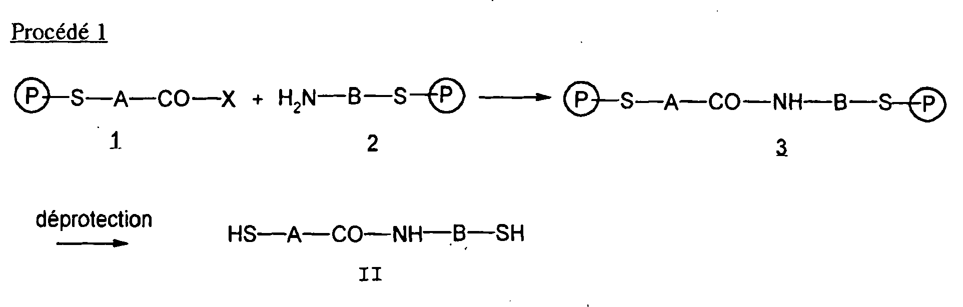

On fait réagir un mercapto halogénure d'acide S protégé 1 avec un aminothiol S protégé 2 (Ⓟ représentant un groupe protecteur de la fonction thiol tel que par exemple un groupe trityle ou acétyle ou benzyle et X un atome de chlore ou brome) dans un solvant aprotique tel que par exemple le dichlorométhane, le dichloro-1,2-éthane, le tétrahydrofurane, le diméthylformamide, ou le dioxane, en présence d'un agent destiné à capter l'acide chlorhydrique ou bromhydrique libéré, tel que par exemple une amine tertiaire, à une température comprise entre 0°C et la température d'ébullition du solvant.

Selon une variante, le mercapto halogénure d'acide S protégé 1 peut être remplacé par un mercapto anhydride d'acide S protégé ou un mercapto ester S protégé. Lorsqu'on met en oeuvre un mercapto ester S protégé, on peut également effectuer la réaction dans un solvant protique et en particulier un alcool aliphatique tel que défini dans le procédé 3 ci-dessous.

Le dimercaptoamide S protégé 3 est ensuite déprotégé en dimercaptoamide (II), soit par hydrolyse, soit par réduction selon la nature du groupe protecteur Ⓟ.

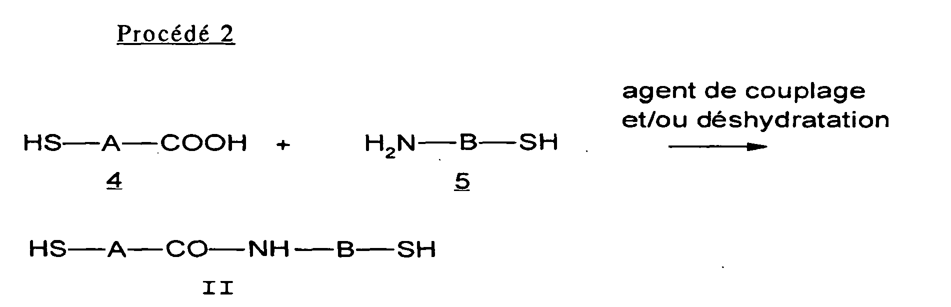

On fait réagir directement un mercaptoacide 4 avec un aminothiol 5 en présence d'un agent de couplage et/ou de déshydratation tels que ceux utilisés pour la synthèse peptidique, par exemple le dicyclohexyl carbodiimide ou le carbonyldiimidazole, dans un solvant aprotique tel que le dichlorométhane, le dichloro-1-2-éthane, le tétrahydrofurane, le diméthylformamide, le dioxane à une température généralement comprise entre -5°C et 40°C.

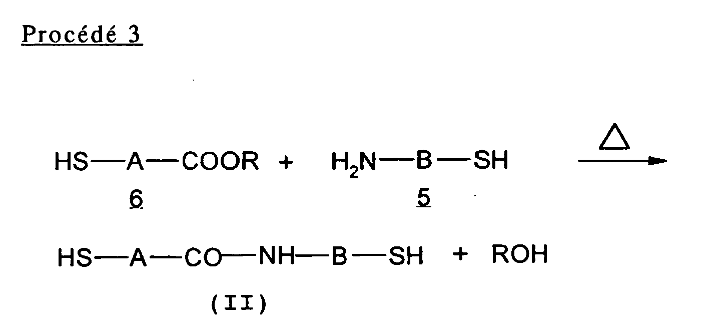

On fait réagir un mercapto-ester 6, R étant un radical méthyle ou éthyle, avec un aminothiol 5 en présence éventuellement d'un solvant aprotique ou protique, tel que par exemple un alcool aliphatique en C₁-C₅ linéaire ou ramifié, à une température comprise entre 30°C et 180°C. De façon avantageuse, on élimine par distillation l'alcool ROH formé au cours de la réaction. De façon également avantageuse, le solvant de la réaction est un alcool aliphatique tel que défini ci-dessus et de préférence le méthanol.

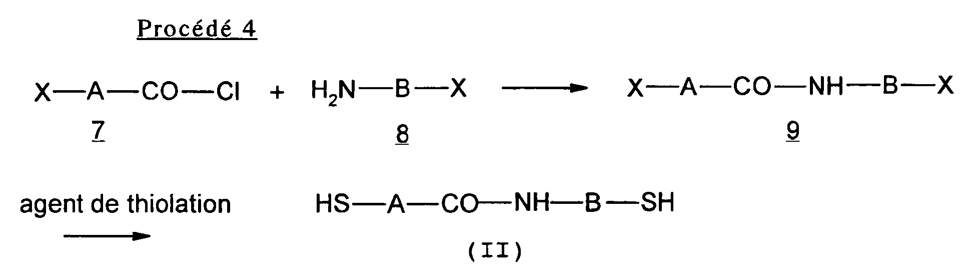

On fait réagir un halogénure d'acide halogéné 7 avec une halogéno amine 8, X représentant un atome de chlore ou brome, dans un solvant aprotique et à une température et en présence d'un agent capteur d'acide chlorhydrique tels que définis dans le procédé 1.

De la même façon que dans le procédé 1, on peut remplacer l'halogénure d'acide halogéné 7 par un anhydride d'acide halogéné ou un halogéno-ester

X-A-COOR 10

Lorsqu'on met en oeuvre un halogéno-ester 10, on peut également effectuer la réaction dans un solvant protique et en particulier un alcool aliphatique tel que défini dans le procédé 3.

Le dihalogéno-amide 9 obtenu est transformé en dimercaptoamide (II), soit par réaction directe avec de l'hydrogène sulfuré dans un solvant approprié, soit par réaction avec l'acide thioacétique, de préférence sous forme salifiée, suivie d'une hydrolyse acide ou alcaline du di(S-acétyl)mercaptoamide, soit encore par réaction avec la thiourée suivie d'une hydrolyse du di sel d'isothiouronium.

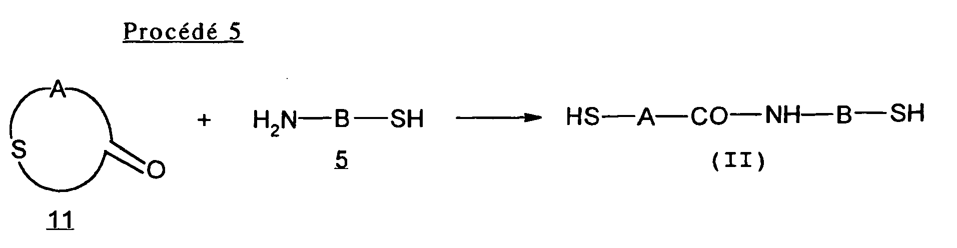

On fait réagir une thiolactone 11 avec un aminothiol 5 dans un solvant aprotique tel que le dichlorométhane, le dichloro-1-2-éthane, le tétrahydrofurane, le diméthylformamide, le dioxane à une température généralement comprise entre 0°C et la température d'ébullition du solvant.

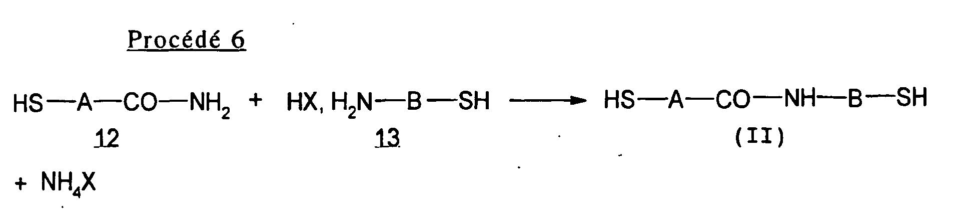

On fait réagir un mercaptoamide 12 avec un sel d'aminothiol 13, X représentant un atome de chlore ou brome, en présence éventuellement d'un solvant inerte, à une température comprise entre 60 et 200°C, de façon à obtenir directement le dimercaptoamide (II).

En fonction de la nature des substituants A et B et de la disponibilité des matières premières, on peut également combiner ces différents procédés en faisant par exemple réagir un mercaptoester 6 HS-A-COOR avec une halogénoamine 8 pour obtenir le mercapto-halogénoamide 14 HS-A-CO-NH-B-X qui est ensuite transformé en dimercaptoamide (II) par utilisation d'un agent de thiolation tel que défini dans le procédé 4.

On peut également, de façon inverse, faire réagir un halogénoester 10 X-A-COOR avec un aminothiol 5 H₂N-B-SH pour obtenir le mercapto-halogénoamide 15 X-A-CO-NH-B-SH qui est ensuite transformé en dimercaptoamide (II) par utilisation d'un agent de thiolation tel que défini ci-dessus.

Dans les procédés mettant en oeuvre un aminothiol S-protégé 2 ou un aminothiol 5 ou une halogénoamine 8, ces composés aminés sont mis en oeuvre, soit directement sous forme base, soit sous forme salifiée en présence d'un équivalent d'agent basique approprié.

L'invention pourra être mieux comprise à l'aide des exemples qui suivent.

### EXEMPLES DE PREPARATION

### Exemple 1 : Préparation du 2-mercapto-N-(2-mercaptoéthyl)-acétamide

### Exemple 1a : préparation selon le procédé 2

A une solution de 23.8 g d'acide mercaptoacétique(0.2584 mole) dans 250ml de dichlorométhane, refroidie à 0°C par un bain de glace, on a ajouté, en goutte à goutte, une solution de 53.5 g de dicyclohexylcarbodiimide (0.2593 mole) dans 175 ml de dichlorométhane.

L'addition a été effectuée de manière à maintenir la température inférieure à 5°C.

Un précipité blanc s'est formé lors de l'addition. L'addition terminée, le milieu réactionnel a été maintenu à 0°C pendant 2 heures sous atmosphère inerte (argon).

Une suspension de 20 g de cystéamine (0.2592 mole) dans 75 ml de dichlorométhane a ensuite été ajoutée en une seule fois puis le mélange a été maintenu sous agitation pendant 12 heures supplémentaires à température ambiante sous atmosphère inerte d'argon.

Après filtration sur verre fritté (élimination de la dicyclohexylurée), le filtrat a été évaporé à sec sous pression réduite, puis repris avec 100ml d'acétone et à nouveau filtré puis évaporé sous pression réduite.

L'huile obtenue a été purifiée sur un filtre de silice dans le dichlorométhane.

Après évaporation et séchage, on a obtenu 30 g de 2-mercapto-N-(2-mercaptoéthyl)-acétamide sous la forme d'une huile incolore.

Le dosage de SH par iodométrie a indiqué un titre de 98%.

Les Spectres RMN 1H 400Mhz (CDCl3), RMN 13C 100Mhz (CDCl3) ainsi que le spectre de masse ont été conformes à la structure attendue.

### Exemple 1b : préparation selon le procédé 3

A une solution de 7,8 g (0,1 mole) de cystéamine dans 100 cm³ de méthanol, maintenue sous argon, on a ajouté 10,6 g (0,1 mole) de thioglycolate de méthyle. On a ensuite chauffé au reflux sous agitation pendant 2H00 puis distillé le méthanol sous pression normale en environ 30'.

On a alors chauffé à 85°C puis distillé les dernières traces de méthanol sous pression réduite. On a ainsi obtenu 13,2 g d'huile translucide qui a été purifiée par chromatographie sur colonne de silice dans le dichlorométhane.

Après évaporation à sec et séchage prolongé, on a obtenu 12 g de 2-mercapto-N-(2-mercaptoéthyl)-acétamide sous la forme d'une huile incolore.

Le dosage de SH par iodométrie indique un titre de 98,2 %.

Les spectres RMN ¹H 400MHz (CDCl₃) et ¹³C 100MHz (CDCl₃) sont conformes à la structure attendue.

### Exemple 2 : Préparation du 3-mercapto-N-(2-mercaptoéthyl)-propionamide.

A une solution de 9,92 g (0,093 mole) d'acide 3-mercaptopropionique dans 100 cm³ de dichlorométhane, maintenue sous argon et refroidie à 0-5°C par un bain de glace, on a ajouté en goutte à goutte une solution de 19,36 g (0,0938 mole) de N,N'-dicyclohexylcarbodiimide dans 60 cm³ de dichlorométhane.

Après 1H00 d'agitation, on a ensuite ajouté en une seule fois une suspension de 7,3 g (0,094 mole) de cystéamine dans 50 cm³ de dichlorométhane.

L'agitation a été maintenue une nuit sous atmosphère d'argon en laissant revenir à température ambiante. Le précipité de dicyclohexylurée a été séparé par filtration sur verre fritté. Le filtrat a été évaporé à sec sous pression réduite. Le solide obtenu a été purifiée par chromatographie sur colonne de silice dans le dichlorométhane en augmentant progressivement la polarité avec du méthanol.

Après évaporation à sec sous pression réduite et séchage, on a obtenu 12 g de 3-mercapto-N-(2-mercaptoéthyl)-propionamide sous la forme d'un solide blanc de point de fusion de 40°C.

Le dosage de SH par iodométrie indique un titre de 98,6 %.

Les spectres RMN ¹H 400MHz (CDCl₃) et ¹³C 100MHz (CDCl₃) ainsi que le spectre de masse sont conformes à la structure attendue.

### Exemple 3 : Préparation du 2-mercapto-N-(2-mercaptoéthyl)-propionamide.

A une solution de 2,3 g (0,0216 mole) d'acide thiolactique dans 50 cm³ de dichlorométhane, maintenue sous argon et refroidie entre 0 et +5°C avec un bain de glace, on a ajouté en goutte à goutte une solution de 4,5 g (0,0218 mole) de N,N'-dicyclohexylcarbodiimide dans 30 cm³ de dichlorométhane. L'agitation a été maintenue 1H00 à température inférieure à 10°C.

On a ensuite ajouté une suspension de 1,67 g (0,0216 mole) de cystéamine dans 20 cm³ de dichlorométhane. L'agitation a été maintenue 4H00 en laissant revenir à température ambiante. Le précipité de dicyclohexylurée a été séparé par filtration sur verre fritté et le filtrat a été évaporé à sec sous pression réduite.

Le solide obtenu a été purifiée par chromatographie sur colonne de silice dans le dichlorométhane en augmentant progressivement la polarité avec du méthanol.

Après évaporation à sec sous pression réduite et séchage, on a obtenu 2,8 g de 2-mercapto-N-(2-mercaptoéthyl)-propionamide sous la forme d'un solide blanc de point de fusion de 53°C.

Le dosage de SH par iodométrie indique un titre de 98,8 %.

Les spectres RMN ¹H 400MHz (CDCl₃) et ¹³C 100MHz (CDCl₃) ainsi que le spectre de masse sont conformes à la structure attendue.

### Exemple 4 : Préparation de la N-(mercaptoacétyl)-L-cystéine.

### a) Acide S-tritylthioglycolique

A une solution de 5 g (0,054 mole) d'acide thioglycolique dans 100 cm³ d'acide acétique, agitée sous argon, on a ajouté 15,5 g (0,059 mole) de triphénylméthanol puis en goutte à goutte 6 cm³ d'éthérate de trifluorure de bore. L'agitation a été maintenue 48H00 à température ambiante sous argon.

Le milieu réactionnel a été versé dans 1 1 d'eau. Le précipité obtenu a été essoré sur verre fritté, lavé avec une solution aqueuse de bicarbonate de sodium jusqu'à pH neutre des eaux de lavage puis une fois à l'eau et séché sous vide à 50°C.

On a ainsi obtenu 17 g d'acide S-tritylthioglycolique sous la forme d'un solide blanc de point de fusion de 163,4°C.

Les spectres RMN ¹H 400MHz (DMSO) et ¹³C 100MHz (DMSO) sont conformes à la structure attendue.

### b) Acide 3-mercaptotrityl-2(2-mercaptotritylacétylamino)-propionique

A une solution de 5 g (0,015 mole) d'acide S-tritylthioglycolique obtenu précédemment dans 80 cm³ de N,N-diméthylformamide, refroidie entre 0 et +5°C avec un bain de glace, on a ajouté 2,5 g de N,N'-dicyclohexyl-carbodiimide.

L'agitation a été maintenue 1H00 puis on a ajouté une suspension de 5,2 g (0,016 mole) de S-tritylcystéamine dans 80 cm³ de N,N-diméthylformamide. On a maintenu l'agitation 16H00 en laissant revenir à température ambiante.

Le mélange réactionnel a été versé dans 1 litre d'eau acidifiée par HCI. Le précipité formé a été essoré sur verre fritté et lavé abondamment à l'eau jusqu'à pH neutre.

Après séchage sous vide à 45°C, on a obtenu 9,7 g d'acide 3-mercaptotrityl-2(2-mercaptotritylacétylamino)-propionique sous la forme d'un solide blanc de point de fusion de 208°C.

Les spectres RMN ¹H 400MHz (CD₃CN) et ¹³C 100MHz (CD₃CN) sont conformes à la structure attendue.

### c) N-mercaptoacétyl-L-cystéine

A une solution de 9,7 g (0,029 mole) d'acide 3-mercaptotrityl-2(2-mercaptotritylacétylamino)-propionique obtenu précédemment dans 250 cm3 de dichlorométhane, maintenue sous argon et refroidie à 0°C, on a ajouté en goutte à goutte 125 cm³ d'acide trifluoroacétique puis 7,5 g (0,065 mole) de triéthylsilane en maintenant la température inférieure à 10°C.

Après 1H00 d'agitation, le milieu réactionnel a été évaporé à sec sous pression réduite. Le solide brut obtenu a été repris par 100 cm³ d'éthanol. La suspension a été filtrée sur verre fritté et le filtrat évaporé à sec sous pression réduite puis à nouveau repris par 30 cm³ d'acétate d'éthyle. L'insoluble a été séparé par filtration sur verre fritté et le filtrat évaporé à sec sous pression réduite.

Après séchage, on a obtenu 1,3 g de N-mercaptoacétyl-L-cystéine sous la forme d'un solide blanc de point de fusion de 121°C.

Les spectres RMN ¹H 400MHz (DMSO) et ¹³C 100MHz (DMSO) ainsi que le spectre de masse sont conformes à la structure attendue.

### EXEMPLES DE COMPOSITIONS

### Exemple 5

On a préparé une composition réductrice de déformation permanente des cheveux en procédant au mélange des ingrédients suivants :
- 2-mercapto-N-(2-mercaptoéthyl)-acétamide 12,lu
- ammoniaque à 20% qsp pH 8,5
- eau déminéralisée qsp 100 g

Cette composition a été appliquée sur des cheveux mouillés préalablement enroulés sur des rouleaux de mise en plis. On a laissé agir la composition pendant environ 15 minutes, puis on a séché l'ensemble au sèche-cheveux pendant 5 minutes puis on a rincé abondamment à l'eau. On a appliqué ensuite la composition oxydante suivante :
- eau oxygénée à 200 volumes 4,8 g
- acide citrique qsp pH 3
- eau déminéralisée qsp 100 g

On a laissé agir la composition oxydante pendant 5 minutes puis on a rincé abondamment à l'eau, on a enlevé les bigoudis et on a séché sous casque. On a obtenu une belle frisure soutenue et nerveuse.

### Exemple 6

On a préparé une composition réductrice de déformation permanente des cheveux en procédant au mélange des ingrédients suivants :
- 2-mercapto-N-(2-mercaptoéthyl)-acétamide 12,1 g
- ammoniaque à 20% qsp pH 7,0
- eau déminéralisée qsp 100 g

Cette composition a été appliquée sur des cheveux mouillés préalablement enroulés sur des rouleaux de mise en plis. On a laissé agir la composition pendant environ 15 minutes, puis on a séché l'ensemble au sèche-cheveux pendant 5 minutes et on a rincé abondamment à l'eau. On a appliqué ensuite la composition oxydante suivante :
- eau oxygénée à 200 volumes 4,8 g
- acide citrique qsp pH 3
- eau déminéralisée qsp 100 g

On a laissé agir la composition oxydante pendant 5 minutes puis on a rincé abondamment à l'eau, on a enlevé les bigoudis et on a séché sous casque. On a obtenu une belle frisure soutenue et nerveuse.

### Exemple 7

On a préparé selon l'invention une composition réductrice de déformation permanente des cheveux en procédant au mélange des ingrédients suivants :
- 3-mercapto-N-(2-mercaptoéthyl)-propionamide 8,3g
- ammoniaque à 20% qsp pH 7,1
- eau déminéralisée qsp 100 g

Cette composition a été appliquée sur des cheveux mouillés préalablement enroulés sur des rouleaux de mise en plis. Après avoir laissé agir la composition pendant environ 20 minutes, on a séché l'ensemble au sèche-cheveux pendant 5 minutes puis on a rincé abondamment à l'eau. On a ensuite appliqué la composition oxydante suivante :
- eau oxygénée à 200 volumes 4,8 g
- acide citrique qsp pH 3
- eau déminéralisée qsp 100 g

On a laissé agir la composition oxydante pendant 5 minutes puis rincé abondamment à l'eau, enlevé les bigoudis et séché sous casque. On a obtenu une belle frisure soutenue et nerveuse.

### Exemple 8

On a préparé selon l'invention une composition réductrice de déformation permanente des cheveux en procédant au mélange des ingrédients suivants:
- 2-mercapto-N-(2-mercaptoéthyl)-propionamide 8,3g
- ammoniaque à 20% qsp pH 7,2
- eau déminéralisée qsp 100 g

Cette composition a été appliquée sur des cheveux mouillés préalablement enroulés sur des rouleaux de mise en plis. Après avoir laissé agir la composition pendant environ 25 minutes, on a séché l'ensemble au sèche-cheveux pendant 5 minutes puis on a rincé abondamment à l'eau. On a ensuite appliqué la composition oxydante suivante :
- eau oxygénée à 200 volumes 4,8 g
- acide citrique qsp pH 3
- eau déminéralisée qsp 100 g

On a laissé agir la composition oxydante pendant 5 minutes puis rincé abondamment à l'eau, enlevé les bigoudis et séché sous casque. On a obtenu une belle frisure soutenue et nerveuse.

### Exemple 9

On a préparé selon l'invention une composition réductrice de déformation permanente des cheveux en procédant au mélange des ingrédients suivants :
- N-mercaptoacétyl-L-cystéine 18,83g
- ammoniaque à 20% qsp pH 7,0
- eau déminéralisée qsp 100 g

Cette composition a été appliquée sur des cheveux mouillés préalablement enroulés sur des rouleaux de mise en plis. Après avoir laissé agir la composition pendant environ 25 minutes, on a séché l'ensemble au sèche-cheveux pendant 5 minutes puis on a rincé abondamment à l'eau. On a ensuite appliqué la composition oxydante suivante :
- eau oxygénée à 200 volumes 4,8 g
- acide citrique qsp pH 3
- eau déminéralisée qsp 100 g

On a laissé agir la composition oxydante pendant 5 minutes puis rincé abondamment à l'eau, enlevé les bigoudis et séché sous casque. On a obtenu une belle frisure soutenue et nerveuse.

## Revendications

1. Utilisation, en tant qu'agent réducteur pour la déformation permanente des fibres kératiniques, et notamment des fibres kératiniques humains telles que les cheveux, d'un dimercaptoamide de formule générale (I) suivante :
HS-A-CO-NH-B-SH (I)
dans laquelle :
A représente un radical (CH2)n, n étant un nombre entier compris entre 1 et 5, éventuellement substitué par :
(i) un radical alkyle en C1-C5, linéaire ou ramifié ; phényle ; benzyle ; amino ; acétylamino ; NH-CO-CH2-NH2 ; NH-CO-CH2-CH2-CH(NH2)COOH ; NH-CO-CH2-CH2-CH(COOH)COOH ; CH2-COOH; CH2-COOCH3 ; ou CH2-COOCH2-CH3, ou
(ii) deux radicaux méthyle ou deux radicaux éthyle
et B représente un radical (CH₂)ₚ, p étant un nombre entier compris entre 1 et 5, éventuellement substitué par :
(i) un radical alkyle en C1-C5, linéaire ou ramifié ; carboxyle ; COOCH3 ; COOEt ; CONH2 ; CONH-CH3 ; CON(CH3)2 ; CONH-CH2-CH3 ; CON(CH2-CH3)2 ; CONH-CH2-CHOH-CH3 ; ; CO-NH-CH(COOH)-(CH2)4-NH2 ; CO-NH-CH(COOH)-(CH2)4-N(CH3)2 ; CO-NH-CH2-CH2-COOEt ; CO-NH-CH(COOH)-iPr ; CO-NH-CH2-R avec R = CO-NH2 ou (CH2)3-NH2 ou (CH2)4-NH2 ou (CH2)5-NH2 ou (CH2)4-OH, ou
(ii) 2 radicaux méthyle,
la somme n+p étant comprise entre 2 et 6,
et leurs sels organiques et minéraux

2. Utilisation selon la revendication 1, **caractérisée en ce que** le dimercaptoamide de formule (I) est choisi parmi les composés suivants :
- le 2-mercapto-N-(mercaptométhyl)-acétamide
- le 2-mercapto-N-(2-mercaptoéthyl)-acétamide
- le 2-mercapto-N-(3-mercaptopropyl)-acétamide
- le 2-mercapto-N-(4-mercaptobutyl)-acétamide
- le 2-mercapto-N-(5-mercaptopentyl)-acétamide
- le 3-mercapto-2-(2-mercapto-acétylamino)-propionamide
- le 3-mercapto-2-(2-mercapto-acetylamino)-N,N-diméthyl-propionamide
- le 2-mercapto-N-(2-mercapto-1,1-diméthyl-éthyl)-acétamide
- le 2-mercapto-N-(1-mercaptométhyl-propyl)-acétamide
- le mercapto-[(mercaptoacétyl)amino]-acétate d'éthyle
- la N-(mercaptoacétyl)-L-cystéine
- la N-(mercaptoacétyl)-L-Homocystéine
- la N-(mercaptoacétyl)-L-Homocystéine, sel de sodium
- le 2-Mercapto-N-mercaptométhyl-propionamide
- le 2-Mercapto-N-(2-mercapto-éthyl)-propionamide
- le 2-Mercapto-N-(3-mercapto-propyl)-propionamide
- le 2-Mercapto-N-(4-mercapto-butyl)-propionamide
- le 2-Mercapto-N-(5-mercapto-pentyl)-propionamide
- l'acide mercapto[(2-mercapto-1-oxopropyl)amino]-acétique
- la N-(2-mercapto-1-oxopropyl)-DL-cystéine
- la (R)-N-(2-mercapto-1-oxopropyl)-L-cystéine
- la (S)-N-(2-mercapto-1-oxopropyl)-L-cystéine
- la 2-mercaptopropionyl-L-cystéine
- le N-(1-Carbamoyl-2-mercapto-éthyl)-2-mercapto-propionamide
- le N-(1-Diméthylcarbamoyl-2-mercapto-éthyl)-2-mercapto-propionamide
- le 4-Mercapto-2-(2-mercapto-propionylamino)-butyric acid
- le 2-Mercapto-N-(2-mercapto-1,1-diméthyl-éthyl)-propionamide
- le 2-Mercapto-butanoic acid mercaptométhyl-amide
- le 2-Mercapto-butanoic acid (2-mercapto-éthyl)-amide
- le 2-Mercapto-butanoic acid (3-mercapto-propyl)-amide
- le 2-Mercapto-butanoic acid (4-mercapto-butyl)-amide
- le 2-Mercapto-butanoic acid (5-mercapto-pentyl)-amide
- le 2-Mercapto-butanoic acid (1-carbamoyl-2-mercapto-éthyl)-amide
- le N-(1-Diméthylcarbamoyl-2-mercapto-éthyl)-2-mercapto-butyramide
- le 2-Mercapto-butanoic acid (1-carbamoyl-3-mercapto-propyl)-amide
- le 2-Mercapto-butanoic acid (2-mercapto-1,1-diméthyl-éthyl)-amide
- le 2-Mercapto-pentanoic acid mercaptométhyl-amide
- le 2-Mercapto-pentanoic acid (2-mercapto-éthyl)-amide
- le 2-Mercapto-pentanoic acid (3-mercapto-propyl)-amide
- le 2-Mercapto-pentanoic acid (4-mercapto-butyl)-amide
- le 2-Mercapto-pentanoic acid (5-mercapto-pentyl)-amide
- le 3-Mercapto-2-(2-mercapto-pentanoylamino)-propionic acid
- le 2-Mercapto-pentanoic acid (1-carbamoyl-2-mercapto-éthyl)-amide
- le 2-Mercapto-pentanoic acid (1-diméthylcarbamoyl-2-mercapto-éthyl)-amide
- le 2-Mercapto-pentanoic acid (1-carbamoyl-3-mercapto-propyl)-amide
- le 2-Mercapto-pentanoic acid (2-mercapto-1,1-diméthyl-éthyl)-amide
- le mercapto[(mercaptophénylacétyl)amino]-acétate d'éthyle
- la (R)-N-[2-(mercaptométhyl)-1-oxo-3-phénylpropyl]-L-Cystéine
- la (R)-N-[2-(mercaptométhyl)-1-oxo-3-phénylpropyl]-L-Homocystéine
- la (S)-N-[2-(mercaptométhyl)-1-oxo-3-phénylpropyl]-L-Homocystéine
- le 3-mercapto-N-(2-mercaptoéthyl)-propanamide
- le 3-Mercapto-N-mercaptométhyl-propionamide
- le 3-Mercapto-N-(3-mercapto-propyl)-propionamide
- le 3-Mercapto-N-(4-mercapto-butyl)-propionamide
- la N-(3-mercapto-1-oxopropyl)-L-Cystéine
- le 3-Mercapto-2-(3-mercapto-propionylamino)-propionamide
- le N-(1-Diméthylcarbamoyl-2-mercapto-éthyl)-3-mercapto-propionamide
- le 4-Mercapto-2-(3-mercapto-propionylamino)-butyric acid
- le 3-Mercapto-N-(2-mercapto-1,1-diméthyl-éthyl)-propionamide
- le 3-Mercapto-N-mercaptométhyl-2-méthyl-propionamide
- le 3-Mercapto-N-(2-mercaptoéthyl)-2-méthyl-propionamide
- le 3-Mercapto-N-(3-mercapto-propyl)-2-méthyl-propionamide
- le 3-Mercapto-N-(4-mercapto-butyl)- 2-méthyl-propionamide
- le 3-Mercapto-2-(3-mercapto-2-méthyl-propionylamino)-propionic acid
- le N-(1-Carbamoyl-2-mercapto-éthyl)-3-mercapto-2-méthyl-propionamide
- le N-(1-Diméthylcarbamoyl-2-mercapto-éthyl)-3-mercapto-2-méthyl-propionamide
- le 3-Mercapto-N-(2-mercapto-1,1-diméthyl-éthyl)- 2-méthyl-propionamide
- la N-(3-mercapto-2-méthyl-1-oxopropyl)-L-Homocystéine
- la N-[2-(mercaptométhyl)-1-oxo-3-phénylpropyl]-Cystéine
- le 4-[(1-carboxy-2-mercaptoéthyl)amino]-3-(mercaptométhyl)-4-oxo-Butanoate de méthyle
- le N-mercaptométhyl-3-mercaptométhyl-succinamic acid
- le N-(2-Mercapto-éthyl)-3-mercaptométhyl-succinamic acid
- le N-(3-Mercapto-propyl)-3-mercaptométhyl-succinamic acid
- le N-(4-Mercapto-butyl)-3-mercaptométhyl-succinamic acid
- le N-(1-Carboxy-2-mercapto-éthyl)-3-mercaptométhyl-succinamic acid
- le N-(1-Carboxy-3-mercapto-propyl)-3-mercaptométhyl-succinamic acid
- le N-(2-Mercapto-1,1-diméthyl-éthyl)-3-mercaptométhyl-succinamic acid
- le N-(2-Mercapto-1-éthyl)-3-mercaptométhyl-succinamic acid
- le 2-Mercapto-N-mercaptométhyl-2-méthyl-propionamide
- le 2-mercapto-N-(2-mercaptoéthyl)-2-méthyl-propanamide
- le 2-Mercapto-N-(3-mercapto-propyl)-2-méthyl-propionamide
- le 2-Mercapto-N-(4-mercapto-butyl)-2-méthyl-propionamide
- le 2-Mercapto-N-(5-mercapto-pentyl)-2-méthyl-propionamide
- le 2-Mercapto-N-(2-mercapto-1,1-diméthyl-éthyl)-2-méthyl-propionamid
- le 2-Mercapto-N-(2-mercapto-1-éthyl)-2-méthyl-propionamide
- la N-(2-mercapto-2-méthyl-1-oxopropyl)-D-Cystéine
- la N-(2-mercapto-2-méthyl-1-oxopropyl)-Cystéine
- la N-(2-mercapto-2-méthyl-1-oxopropyl)-L-Cystéine, monosel de sodium
- la N-(2-mercapto-2-méthyl-1-oxopropyl)-L-Cystéine
- le (R)-N-[2-amino-1-(mercaptométhyl)-2-oxoéthyl]-2-mercapto-2-méthyl-Propanami de
- le N-(2-mercapto-2-méthyl-1-oxopropyl)-L-Cystéinate de méthyle
- le (2R)-3-mercapto-2-[(2-mercapto-2-méthyl-1-oxopropyl)amino]-N,N-diméthyl-Propanamide
- la N2-[N-(2-mercapto-2-méthyl-1-oxopropyl)-L-cystéinyl]-L-Lysine
- la N-(2-mercapto-2-méthyl-1-oxopropyl)-Homocystéine
- la N-(2-éthyl-2-mercapto-1-oxobutyl)-L-Cystéine
- le 3-Mercapto-N-mercaptométhyl-3-méthyl-butyramide
- le 3-mercapto-N-(2-mercaptoéthyl)-3-méthyl-Butanamide
- le 3-Mercapto-N-(3-mercapto-propyl)-3-méthyl-butyramide
- le 3-Mercapto-N-(4-mercapto-butyl)-3-méthyl-butyramide
- la N-(3-mercapto-3-méthyl-1-oxobutyl)-L-Cystéine
- le N-(1-Carbamoyl-2-mercapto-éthyl)-3-mercapto-3-méthyl-butyramide
- le N-(1-Diméthylcarbamoyl-2-mercapto-éthyl)-3-mercapto-3-méthyl-butyramide
- le 4-Mercapto-2-(3-mercapto-3-méthyl-butyrylamino)-butyric acid
- le 3-Mercapto-N-(2-mercapto-1,1-diméthyl-éthyl)-3-méthyl-butyramide
- le 3-Mercapto-N-(2-mercapto-1-éthyl)-3-méthyl-butyramide
- le 3-Mercapto-N-mercaptométhyl-2,2-diméthyl-propionamide
- le 3-Mercapto-N-(2-mercaptoéthyl)-2,2-diméthyl-propionamide
- le 3-Mercapto-N-(3-mercapto-propyl)-2,2-diméthyl-propionamide
- le 3-Mercapto-N-(4-mercapto-butyl)- 2,2-diméthyl-propionamide
- le 3-Mercapto-N-(2-mercapto-1,1-diméthyl-éthyl)- 2,2-diméthyl-propionamide
- la N-(3-mercapto-2,2-diméthyl-1-oxopropyl)-D-Cystéine
- la N-(3-mercapto-2,2-diméthyl-1-oxopropyl)-L-Cystéine
- le N-(1-Carbamoyl-2-mercapto-éthyl)-3-mercapto-2,2-diméthyl-propionamide
- le N-(1-Diméthylcarbamoyl-2-mercapto-éthyl)-3-mercapto-2,2-diméthyl-propionamide
- le N-(3-mercapto-2,2-diméthyl-1-oxopropyl)-D-cystéinyl-Glycinamide
- la N6-[(1,1-diméthyléthoxy)carbonyl]-N2-[N-(3-mercapto-2,2-diméthyl-1-oxopropyl)-L-cystéinyl]-L-Lysine
- le N-[2-[(4-aminobutyl)amino]-1-(mercaptométhyl)-2-oxoéthyl]-3-mercapto-2,2-diméthyl-Propanamide, monochlorhydrate
- le N-[2-[(5-aminopentyl)amino]-1-(mercaptométhyl)-2-oxoéthyl]-3-mercapto-2,2-diméthyl-Propanamide, monochlorhydrate
- le N-[2-[(6-aminohexyl)amino]-1-(mercaptométhyl)-2-oxoéthyl]-3-mercapto-2,2-diméthyl-Propanamide, monochlorhydrate
- le (R)-N-[2-[(5-hydroxypentyl)amino]-1-(mercaptométhyl)-2-oxoéthyl]-3-mercapto-2,2-diméthyl-Propanamide
- la N2-[N-(3-mercapto-2,2-diméthyl-1-oxopropyl)-L-cystéinyl]-L-Lysine
- la N2-[N-(3-mercapto-2,2-diméthyl-1-oxopropyl)-L-cystéinyl]-N6,N6-diméthyl-L-Lysine
- la 3-mercapto-N-(3-mercapto-2,2-diméthyl-1-oxopropyl)-D-Valine
- la N-(3-mercapto-2,2-diméthyl-1-oxopropyl)-Homocystéine
- la N-[2-éthyl-2-(mercaptométhyl)-1-oxobutyl]-L-cystéine
- le 2-Amino-3-mercapto-N-mercaptométhyl-propionamide
- le (2R)- 2-amino-3-mercapto-N-(2-mercaptoéthyl)-Propanamide
- le (2S)-2-amino-3-mercapto-N-(2-mercaptoéthyl)-Propanamide
- le 2-Amino-3-mercapto-N-(3-mercapto-propyl)-propionamide
- le 2-Amino-3-mercapto-N-(4-mercapto-butyl)-propionamide
- la L-cystéinyl-L-Cystéine
- le Mercapto[(mercaptoacétyl)amino]-acétate de méthyle
- le L-cystéinyl-L-Cystéinate d'éthyle
- le 2-Amino-N-(1-carbamoyl-2-mercapto-éthyl)-3-mercapto-propionamide
- le 2-Amino-N-(1-carbamoyl-2-mercapto-éthyl)-3-mercapto-N,N-diméthyl-propionamide
- le 2-(2-Amino-3-mercapto-propionylamino)-4-mercapto-butyric acid
- le 2-Amino-3-mercapto-N-(2-mercapto-1,1-diméthyl-éthyl)-propionamide
- le 2-Amino-3-mercapto-N-(2-mercapto-1-éthyl)-propionamide
- le 2-Acétylamino-3-mercapto-N-mercaptométhyl-propionamide
- le (2R)-2-(acétylamino)-3-mercapto-N-(2-mercaptoéthyl)-Propanamide
- le 2-Acétylamino-3-mercapto-N-(3-mercapto-propyl)-propionamide
- le 2-Acétylamino-3-mercapto-N-(4-mercapto-butyl)-propionamide
- le 2-(2-Acétylamino-3-mercapto-propionylamino)-3-mercapto-3-méthyl-butyric acid
- le 2-Acétylamino-3-mercapto-N-(1-mercaptométhyl-propyl)-propionamide
- le 2-Acétylamino-N-(1-diméthylcarbamoyl-2-mercapto-éthyl)-3-mercapto-propionamide
- la N-acétyl-L-cystéinyl-L-Cystéine
- la N-(N-acétylcystéinyl)-L-Cystéine
- la N-(N-acétylcystéinyl)-DL-Cystéine
- le N-acétyl-L-cystéinyl-D-Cystéinate d'éthyle
- le N-acétyl-L-cystéinyl-L-Cystéinate d'éthyle
- le 2-acétamido-N-(1-carbamoyl-2-mercaptoéthyl)-3-mercapto-Propionamide
- le N-acétyl-L-cystéinyl-L-cystéinyl-Glycinate d'éthyle
- le 2-(2-Acétylamino-3-mercapto-propionylamino)-4-mercapto-butyric acid
- le 2-(carboxyméthylamino)-3-mercapto-N-mercaptométhyl-propionamide
- le 2-(carboxyméthylamino)-3-mercapto-N-(2-mercapto-éthyl)-propionamide
- le 2-(carboxyméthylamino)-3-mercapto-N-(3-mercapto-propyl)-propionamide
- le 2-(carboxyméthylamino)-3-mercapto-N-(4-mercapto-butyl)-propionamide
- le 2-(carboxyméthylamino)-N-(1-carbamoyl-2-mercapto-éthyl)-3-mercapto- propionamide
- le 2-[2-(Carboxyméthyl-amino)-3-mercapto-propionylamino]-3-mercapto-propionic acid
- le [1-(1-Carbamoyl-2-mercapto-éthylcarbamoyl)-2-mercapto-éthylamino]-acetic acid
- le 2-(carboxyméthylamino)-3-mercapto-N-(2-mercapto-1,1-diméthyl-éthyl)-propionamide
- la N-[N-[N-(carboxyméthyl)-L-cystéinyl]-L-cystéinyl]-D-Valine
- le 2-Amino-4-[2-mercapto-1-(mercaptométhyl-carbamoyl)-éthylcarbamoyl]-butyric acid
- le 2-Amino-4-[2-mercapto-1-(2-mercapto-éthylcarbamoyl)-éthylcarbamoyl]-butyric acid
- le 2-Amino-4- [2-mercapto-1-(3-mercapto-propylcarbamoyl)-éthylcarbamoyl]-butyric acid
- le 2-Amino-4-[2-mercapto-1-(4-mercapto-butylcarbamoyl)-éthylcarbamoyl]-butyric acid
- la L-.gamma.-glutamyl-L-cystéinyl-L-Cystéine
- le 2-[2-(4-Amino-4-carboxy-butyrylamino)-3-mercapto-propionylamino]-4-mercapto-butyric acid
- le 2-Amino-4-[1-(1-carbamoyl-2-mercapto-éthylcarbamoyl)-2-mercapto-éthylcarbamoyl]-butyric acid
- le 2-Amino-4-[1-(1-diméthylcarbamoyl-2-mercapto-éthylcarbamoyl)-2-mercapto-éthylcarbamoyl]-butyric acid
- le 2-Amino-4-[2-mercapto-1-(2-mercapto-1,1-diméthyl-éthylcarbamoyl)-éthylcarbamoyl]-butyric acid
- le 2-Amino-4-[2-mercapto-1-(2-mercapto-1-éthyl-éthylcarbamoyl)-éthylcarbamoyl]-butyric acid
- le 2-(2-Amino-acétylamino)-3-mercapto-N-mercaptométhyl-propionamide
- le 2-(2-Amino-acétylamino)-3-mercapto-N-(2-mercapto-éthyl)-propionamide
- le 2-(2-Amino-acétylamino)-3-mercapto-N-(3-mercapto-propyl)-propionamide
- le 2-(2-Amino-acétylamino)-3-mercapto-N-(4-mercapto-butyl)-propionamide
- le 2-(2-Amino-acétylamino)-3-mercapto-N-(5-mercapto-pentyl)- propionamide
- la glycyl-L-cystéinyl-L-Cystéine
- le 2-[2-(2-Amino-acétylamino)-3-mercapto-propionylamino]-4-mercapto-butyric acid
- le 2-(2-Amino-acétylamino)-3-mercapto-N-(2-mercapto-1,1-diméthyl-éthyl)-propionamide
- le 2-(2-Amino-acétylamino)-3-mercapto-N-(1-mercaptométhyl-propyl)-propionamide
- la 4-carboxy-L-.alpha.-glutamyl-L-cystéinyl-L-Cystéine
- la (S)-N-[N-(5-amino-5-carboxy-1-oxopentyl)-L-homocystéinyl]-L-Cystéine
- le 4-Mercapto-N-mercaptométhyl-butyramide
- le 4-Mercapto-N-(2-mercapto-éthyl)-butyramide
- le 4-Mercapto-N-(3-mercapto-propyl)-butyramide
- le N-(4-mercapto-1-oxobutyl)-L-Cystéine
- le 4-Mercapto-2-(3-mercapto-propionylamino)-butyramide
- le N-(1-Diméthylcarbamoyl-2-mercapto-éthyl)-4-mercapto-butyramide
- le N-(4-mercapto-1-oxobutyl)-L-homocystéine
- le 4-Mercapto-N-(2-mercapto-1,1-diméthyl-éthyl)-butyramide
- le 4-Mercapto-N-(1-mercaptométhyl-propyl)-butyramide
- le 5-Mercapto-pentanoic acid mercaptométhyl-amide
- le 5-Mercapto-pentanoic acid (2-mercapto-éthyl)-amide
- le N-(5-mercapto-1-oxopentyl)-L-Cystéine
- le 5-Mercapto-2-(3-mercapto-propionylamino)-pentanamide
- le N-(1-Diméthylcarbamoyl-2-mercapto-éthyl)-5-mercapto-pentanamide
- le 5-Mercapto-N-(2-mercapto-1,1-diméthyl-éthyl)-pentanamide
- le 5-Mercapto-N-(1-mercaptométhyl-propyl)-pentanamide
- le 6-Mercapto-hexanoic acid mercaptométhyl-amide.

3. Utilisation selon la revendication 2, **caractérisée en ce que** le dimercaptoamide de formule (I) est choisi parmi les composés suivants :
- le 2-mercapto-N-(mercaptométhyl)-acétamide
- le 2-mercapto-N-(2-mercaptoéthyl)-acétamide
- le 2-mercapto-N-(3-mercaptopropyl)-acétamide
- le 2-mercapto-N-(4-mercaptobùtyl)-acétamide
- le 2-mercapto-N-(5-mercaptopentyl)-acétamide
- le 3-mercapto-2-(2-mercapto-acétylamino)-propionamide
- le 3-mercapto-2-(2-mercapto-acetylamino)-N,N-diméthyl-propionamide
- le 2-mercapto-N-(2-mercapto-1,1-diméthyl-éthyl)-acétamide
- le 2-mercapto-N-(1-mercaptométhyl-propyl)-acétamide
- la N-(mercaptoacétyl)-L-cystéine
- la N-(mercaptoacétyl)-L-Homocystéine
- le 2-Mercapto-N-mercaptométhyl-propionamide
- le 2-Mercapto-N-(2-mercapto-éthyl)-propionamide
- le 2-Mercapto-N-(3-mercapto-propyl)-propionamide
- le 2-Mercapto-N-(4-mercapto-butyl)-propionamide
- le 2-Mercapto-N-(5-mercapto-pentyl)-propionamide
- l'acide mercapto[(2-mercapto-1-oxopropyl)amino]-acétique
- la 2-mercaptopropionyl-L-cystéine
- le N-(1-Carbamoyl-2-mercapto-éthyl)-2-mercapto-propionamide
- le N-(1-Diméthylcarbamoyl-2-mercapto-éthyl)-2-mercapto-propionamide
- le 4-Mercapto-2-(2-mercapto-propionylamino)-butyric acid
- le 2-Mercapto-N-(2-mercapto-1,1-diméthyl-éthyl)-propionamide
- le 2-Mercapto-butanoic acid mercaptométhyl-amide
- le 2-Mercapto-butanoic acid (2-mercapto-éthyl)-amide
- le 2-Mercapto-butanoic acid (3-mercapto-propyl)-amide
- le 2-Mercapto-butanoic acid (4-mercapto-butyl)-amide
- le 2-Mercapto-pentanoic acid mercaptométhyl-amide
- le 2-Mercapto-pentanoic acid (2-mercapto-éthyl)-amide
- le 2-Mercapto-pentanoic acid (3-mercapto-propyl)-amide
- le 3-mercapto-N-(2-mercaptoéthyl)-propanamide
- le 3-Mercapto-N-mercaptométhyl-propionamide
- le 3-Mercapto-N-(3-mercapto-propyl)-propionamide
- le 3-Mercapto-N-(4-mercapto-butyl)-propionamide
- la N-(3-mercapto-1-oxopropyl)-L-Cystéine
- le 3-Mercapto-2-(3-mercapto-propionylamino)-propionamide
- le N-(1-Diméthylcarbamoyl-2-mercapto-éthyl)-3-mercapto-propionamide
- le 4-Mercapto-2-(3-mercapto-propionylamino)-butyric acid
- le 3-Mercapto-N-(2-mercapto-1,1-diméthyl-éthyl)-propionamide
- le 3-Mercapto-N-mercaptométhyl-2-méthyl-propionamide
- le 3-Mercapto-N-(2-mercaptoéthyl)-2-méthyl-propionamide
- le 3-Mercapto-N-(3-mercapto-propyl)-2-méthyl-propionamide
- le 3-Mercapto-N-(4-mercapto-butyl)- 2-méthyl-propionamide
- le 3-Mercapto-2-(3-mercapto-2-méthyl-propionylamino)-propionic acid
- le N-(1-Carbamoyl-2-mercapto-éthyl)-3-mercapto-2-méthyl-propionamide
- le N-(1-Diméthylcarbamoyl-2-mercapto-éthyl)-3-mercapto-2-méthyl-propionamide
- le 3-Mercapto-N-(2-mercapto-1,1-diméthyl-éthyl)- 2-méthyl-propionamide
- la N-(3-mercapto-2-méthyl-1-oxopropyl)-L-Homocystéine
- le N-mercaptométhyl-3-mercaptométhyl-succinamic acid
- le N-(2-Mercapto-éthyl)-3-mercaptométhyl-succinamic acid
- le N-(3-Mercapto-propyl)-3-mercaptométhyl-succinamic acid
- le N-(4-Mercapto-butyl)-3-mercaptométhyl-succinamic acid
- le N-(1-Carboxy-2-mercapto-éthyl)-3-mercaptométhyl-succinamic acid
- le 2-Mercapto-N-mercaptométhyl-2-méthyl-propionamide
- le 2-mercapto-N-(2-mercaptoéthyl)-2-méthyl-propanamide
- le 2-Mercapto-N-(3-mercapto-propyl)-2-méthyl-propionamide
- le 2-Mercapto-N-(4-mercapto-butyl)-2-méthyl-propionamide
- le 2-Mercapto-N-(2-mercapto-1,1-diméthyl-éthyl)-2-méthyl-propionamide
- le 2-Mercapto-N-(2-mercapto-1-éthyl)-2-méthyl-propionamide
- la N-(2-mercapto-2-méthyl-1-oxopropyl)-L-Cystéine
- la N-(2-éthyl-2-mercapto-1-oxobutyl)-L-Cystéine
- le 3-Mercapto-N-mercaptométhyl-3-méthyl-butyramide
- le 3-mercapto-N-(2-mercaptoéthyl)-3-méthyl-Butanamide
- le 3-Mercapto-N-(3-mercapto-propyl)-3-méthyl-butyramide
- la N-(3-mercapto-3-méthyl-1-oxobutyl)-L-Cystéine
- le N-(1-Carbamoyl-2-mercapto-éthyl)-3-mercapto-3-méthyl-butyramide
- le N-(1-Diméthylcarbamoyl-2-mercapto-éthyl)-3-mercapto-3-méthyl-butyramide
- le 4-Mercapto-2-(3-mercapto-3-méthyl-butyrylamino)-butyric acid
- le 3-Mercapto-N-mercaptométhyl-2,2-diméthyl-propionamide
- le 3-Mercapto-N-(2-mercaptoéthyl)-2,2-diméthyl-propionamide
- le 3-Mercapto-N-(3-mercapto-propyl)-2,2-diméthyl-propionamide
- le 3-Mercapto-N-(4-mercapto-butyl)- 2,2-diméthyl-propionamide
- la N-(3-mercapto-2,2-diméthyl-1-oxopropyl)-L-Cystéine
- le N-(1-Carbamoyl-2-mercapto-éthyl)-3-mercapto-2,2-diméthyl-propionamide
- le N-(1-Diméthylcarbamoyl-2-mercapto-éthyl)-3-mercapto-2,2-diméthyl-propionamide
- la N-[2-éthyl-2-(mercaptométhyl)-1-oxobutyl]-L-cystéine
- le 2-Amino-3-mercapto-N-mercaptométhyl-propionamide
- le (2R)- 2-amino-3-mercapto-N-(2-mercaptoéthyl)-Propanamide
- le (2S)-2-amino-3-mercapto-N-(2-mercaptoéthyl)-Propanamide
- le 2-Amino-3-mercapto-N-(3-mercapto-propyl)-propionamide
- le 2-Amino-3-mercapto-N-(4-mercapto-butyl)-propionamide
- la L-cystéinyl-L-Cystéine
- le 2-Amino-N-(1-carbamoyl-2-mercapto-éthyl)-3-mercapto-propionamide
- le 2-Amino-N-(1-carbamoyl-2-mercapto-éthyl)-3-mercapto-N,N-diméthyl-propionamide
- le 2-(2-Amino-3-mercapto-propionylamino)-4-mercapto-butyric acid
- le 2-Amino-3-mercapto-N-(2-mercapto-1,1-diméthyl-éthyl)-propionamide
- le 2-Amino-3-mercapto-N-(2-mercapto-1-éthyl)-propionamide
- le 2-Acétylamino-3-mercapto-N-mercaptométhyl-propionamide
- la N-acétyl-L-cystéinyl-L-Cystéine
- le 2-(carboxyméthylamino)-3-mercapto-N-mercaptométhyl-propionamide
- le 2-(carboxyméthylamino)-3-mercapto-N-(2-mercapto-éthyl)-propionamide
- le 2-(carboxyméthylamino)-3-mercapto-N-(3-mercapto-propyl)-propionamide
- la L-.gamma.-glutamyl-L-cystéinyl-L-Cystéine
- le 2-(2-Amino-acétylamino)-3-mercapto-N-mercaptométhyl-propionamide
- le 2-(2-Amino-acétylamino)-3-mercapto-N-(2-mercapto-éthyl)-propionamide
- la glycyl-L-cystéinyl-L-Cystéine
- la 4-carboxy-L-.alpha.-glutamyl-L-cystéinyl-L-Cystéine
- le 4-Mercapto-N-mercaptométhyl-butyramide
- le 4-Mercapto-N-(2-mercapto-éthyl)-butyramide
- le 4-Mercapto-N-(3-mercapto-propyl)-butyramide
- le N-(4-mercapto-1-oxobutyl)-L-Cystéine
- le 4-Mercapto-2-(3-mercapto-propionylamino)-butyramide
- le N-(1-Diméthylcarbamoyl-2-mercapto-éthyl)-4-mercapto-butyramide
- le 4-Mercapto-N-(2-mercapto-1,1-diméthyl-éthyl)-butyramide
- le 4-Mercapto-N-(1-mercaptométhyl-propyl)-butyramide
- le 5-Mercapto-pentanoic acid mercaptométhyl-amide
- le 5-Mercapto-pentanoic acid (2-mercapto-éthyl)-amide
- le N-(5-mercapto-1-oxopentyl)-L-Cystéine
- le 5-Mercapto-2-(3-mercapto-propionylamino)-pentanamide
- le N-(1-Diméthylcarbamoyl-2-mercapto-éthyl)-5-mercapto-pentanamide
- le 5-Mercapto-N-(2-mercapto-1,1-diméthyl-éthyl)-pentanamide
- le 5-Mercapto-N-(1-mercaptométhyl-propyl)-pentanamide
- le 6-Mercapto-hexanoic acid mercaptométhyl-amide

4. Procédé de déformation permanente des fibres kératiniques, et notamment des fibres kératiniques humains telles que les cheveux, comprenant l'application sur les fibres d'une composition réductrice, puis d'une composition oxydante, **caractérisé en ce que** la composition réductrice comprend, en tant qu'agent réducteur, au moins un dimercaptoamide de formule générale (I) suivante:
HS-A-CO-NH-B-SH (I)
dans laquelle :
A représente un radical (CH2)n, n étant un nombre entier compris entre 1 et 5, éventuellement substitué par :
(i) un radical alkyle en C1-C5, linéaire ou ramifié ; phényle ; benzyle ; amino ; acétylamino ; NH-CO-CH2-NH2 ; NH-CO-CH2-CH2-CH(NH2)COOH ; NH-CO-CH2-CH2-CH(COOH)COOH ; CH2-COOH; CH2-COOCH3 ; ou CH2-COOCH2-CH3, ou
(ii) deux radicaux méthyle ou deux radicaux éthyle
et B représente un radical (CH₂)ₚ, p étant un nombre entier compris entre 1 et 5, éventuellement substitué par :
(i) un radical alkyle en C1-C5, linéaire ou ramifié ; carboxyle ; COOCH3 ; COOEt ; CONH2 ; CONH-CH3 ; CON(CH3)2 ; CONH-CH2-CH3 ; CON(CH2-CH3)2 ; CONH-CH2-CHOH-CH3 ; ; CO-NH-CH(COOH)-(CH2)4-NH2 ; CO-NH-CH(COOH)-(CH2)4-N(CH3)2 ; CO-NH-CH2-CH2-COOEt ; CO-NH-CH(COOH)-iPr ; CO-NH-CH2-R avec R = CO-NH2 ou (CH2)3-NH2 ou (CH2)4-NH2 ou (CH2)5-NH2 ou (CH2)4-OH, ou
(ii) 2 radicaux méthyle,
la somme n+p étant comprise entre 2 au minimum et 6 au maximum,
et leurs sels organiques et minéraux.

5. Procédé selon la revendication 4, **caractérisé en ce que** le dimercaptoamide de formule (I) est présent à une concentration comprise entre 0,05 et 35%, et de préférence entre 1 et 20% en poids du poids total de la composition réductrice.

6. Procédé selon la revendication 4 ou 5, **caractérisé en ce que** le pH de la composition réductrice est compris entre 4 et 11, et de préférence entre 6 et 10.

7. Procédé selon l'une quelconque des revendications 4 à 6, **caractérisé en ce que** la composition réductrice comprend en outre un ou plusieurs agents réducteurs choisis parmi l'acide thioglycolique, l'acide thiolactique, leurs dérivés esters et amides, la cystéamine et ses dérivés acylés en C1-C4, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides, les N-mono ou N,N-dialkylmercapto-4-butyramides, les aminomercaptoalkylamides, les dérivés des acides N-(mercaptoalkyl)succinamiques et des N-(mercaptoalkyl)succinimides, les alkylamino mercaptoalkylamides, le mélange azéotrope de thioglycolate de 2-hydroxypropyle et de thioglycolate de (2-hydroxy-1-méthyl)éthyle, les mercaptoalkylaminoamides, les N-mercaptoalkylalcanediamides, les dérivés d'acide formamidine sulfinique.

8. Procédé selon l'une quelconque des revendications 4 à 7, **caractérisé en ce que** la composition réductrice comprend en outre au moins un agent tensioactif non ionique, anionique, cationique ou amphotère, ou un mélange.

9. Procédé selon la revendication 8, **caractérisé en ce que** l'agent tensioactif est choisi parmi les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acide gras oxyéthylénés, les hydroxypropyléthers.

10. Procédé selon l'une quelconque des revendications 4 à 9, **caractérisé en ce que** la composition réductrice comprend en outre un composé choisi parmi les silicones volatiles ou non, linéaires ou cycliques et leurs mélanges, les polydiméthylsiloxanes, les polyorganosiloxanes quaternisés, les polyorganosiloxanes à groupements aminoalkyles modifiés par des groupements alcoxycarbonyalkyles, les polydiméthylsiloxanes à groupements terminaux stéaroxydiméthicone, les copolymères polydiméthylsiloxane-dialkylammonium acétate, les copolymères polydiméthylsiloxane-polyalkylbétaïne, les polysiloxanes organomodifiés par des groupements mercapto ou mercaptoalkyles, les silanes.

11. Procédé selon l'une quelconque des revendications 4 à 9, **caractérisé en ce que** la composition réductrice comprend en outre un composé choisi parmi les polymères cationiques du type ionène, les aminoacides basiques, l'acide glutamique, l'acide aspartique, les peptides et leurs dérivés, les hydrolysats de protéines, les cires, les agents de gonflement et de pénétration, le diméthylisosorbitol, l'urée et ses dérivés, la pyrrolidone, les N-alkyl-pyrrolidones, la thiamorpholinone, les alkyléthers d'alkylèneglycol ou de dialkylèneglycol, les alcanediols en C3-C6, l'imidazolidinone-2, les alcools gras, les dérivés de la lanoline, l'acide panthothénique, les agents anti-chute, les agents antipelliculaires, les épaississants, les agents de suspension, les agents séquestrants, les agents opacifiants, les colorants, les filtres solaires, les parfums et les conservateurs, les épaississants, les gélifiants.

12. Procédé selon l'une quelconque des revendications 4 à 11, **caractérisé en ce que** le composition oxydante comprend comme agent oxydant de l'eau oxygénée, un bromate alcalin, un persel, un polythionate, ou un mélange de bromate alcalin et de persel.

13. Composition réductrice, **caractérisée en ce qu'**elle comprend, en tant qu'agent réducteur, au moins un dimercaptoamide de formule générale (I) suivante :
HS-A-CO-NH-B-SH (I)
dans laquelle :
A représente un radical (CH2)n, n étant un nombre entier compris entre 1 et 5, éventuellement substitué par :
(i) un radical alkyle en C1-C5, linéaire ou ramifié ; phényle ; benzyle ; amino ; acétylamino ; NH-CO-CH2-NH2 ; NH-CO-CH2-CH2-CH(NH2)COOH ; NH-CO-CH2-CH2-CH(COOH)COOH ; CH2-COOH; CH2-COOCH3 ; ou CH2-COOCH2-CH3, ou
(ii) deux radicaux méthyle ou deux radicaux éthyle
et B représente un radical (CH₂)ₚ, p étant un nombre entier compris entre 1 et 5, éventuellement substitué par :
(i) un radical alkyle en C1-C5, linéaire ou ramifié ; carboxyle ; COOCH3; COOEt ; CONH2 ; CONH-CH3; -,- CON(CH3)2 ; CONH-CH2-CH3 ; CON(CH2-CH3)2 ; CONH-CH2-CHOH-CH3 ; ; CO-NH-CH(COOH)-(CH2)4-NH2 ; CO-NH-CH(COOH)-(CH2)4-N(CH3)2 ; CO-NH-CH2-CH2-COOEt ; CO-NH-CH(COOH)-iPr ; CO-NH-CH2-R avec R = CO-NH2 ou (CH2)3-NH2 ou (CH2)4-NH2 ou (CH2)5-NH2 ou (CH2)4-OH, ou
(ii) 2 radicaux méthyle,
la somme n+p étant comprise entre 2 et 6,
et leurs sels organiques et minéraux.

14. Composition selon la revendication 13, **caractérisée en ce que** le dimercatoamide de formule (I) est choisi parmi les composés mentionnés dans la revendication 2 ou 3.

15. Composition selon la revendication 13 ou 14, **caractérisée en ce que** le dimercaptoamide de formule (I) est présent à une concentration comprise entre 0,05 et 35%, et de préférence entre 1 et 20% en poids du poids total de la composition réductrice.

16. Composition selon l'une des revendications 13 à 15, **caractérisée en ce que** le pH de la composition est compris entre 4 et 11, et de préférence entre 6 et 10.

17. Composition selon l'une des revendications 13 à 16, **caractérisée en ce qu'**elle comprend en outre un ou plusieurs agents réducteurs choisis parmi l'acide thioglycolique, l'acide thiolactique, leurs dérivés esters et amides, la cystéamine et ses dérivés acylés en C1-C4, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides, les N-mono ou N,N-dialkylmercapto-4-butyramides, les aminomercaptoalkylamides, les dérivés des acides N-(mercaptoalkyl)succinamiques et des N-(mercaptoalkyl)succinimides, les alkylamino mercaptoalkylamides, le mélange azéotrope de thioglycolate de 2-hydroxypropyle et de thioglycolate de (2-hydroxy-1-méthyl)éthyle, les mercaptoalkylaminoamides, les N-mercaptoalkylalcanediamides, les dérivés d'acide formamidine sulfinique.

18. Composition selon l'une des revendications 13 à 17, **caractérisée en ce qu'**elle comprend en outre au moins un agent tensioactif non ionique, anionique, cationique ou amphotère, ou un mélange.

19. Composition selon la revendication 18, **caractérisée en ce que** l'agent tensioactif est choisi parmi les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acide gras oxyéthylénés, les hydroxypropyléthers.

20. Composition selon l'une des revendications 13 à 19, **caractérisée en ce qu'**elle comprend en outre un composé choisi parmi les silicones volatiles ou non, linéaires ou cycliques et leurs mélanges, les polydiméthylsiloxanes, les polyorganosiloxanes quaternisés, les polyorganosiloxanes à groupements aminoalkyles modifiés par des groupements alcoxycarbonyalkyles, les polydiméthylsiloxanes à groupements terminaux stéaroxydiméthicone, les copolymères polydiméthylsiloxane-dialkylammonium acétate, les copolymères polydiméthylsiloxane-polyalkylbétaïne, les polysiloxanes organomodifiés par des groupements mercapto ou mercaptoalkyles, les silanes.

21. Composition selon l'une des revendications 13 à 20, **caractérisé en ce qu'**elle comprend en outre un composé choisi parmi les polymères cationiques du type ionène, les aminoacides basiques, l'acide glutamique, l'acide aspartique, les peptides et leurs dérivés, les hydrolysats de protéines, les cires, les agents de gonflement et de pénétration, le diméthylisosorbitol, l'urée et ses dérivés, la pyrrolidorie, les N-alkyl-pyrrolidones, la thiamorpholinone, les alkyléthers d'alkylèneglycol ou de dialkylèneglycol, les alcanediols en C3-C6, l'imidazolidinone-2, les alcools gras, les dérivés de la lanoline, l'acide panthothénique, les agents anti-chute, les agents antipelliculaires, les épaississants, les agents de suspension, les agents séquestrants, les agents opacifiants, les colorants, les filtres solaires, les parfums et les conservateurs.

22. Kit pour la déformation permanente des cheveux comprenant, dans un premier compartiment, en tant que composition réductrice, une composition selon l'une des revendications 13 à 21, et dans un second compartiment, une composition oxydante.

23. Dimercaptoamide répondant à la formule générale (II) suivante :
HS-A-CO-NH-B-SH (II)
dans laquelle :
(i) A représente un radical CH2 et B représente un radical (CH2)x dans lequel x = 2, 3, 4 ou 5 ; un radical C(CH3)2-CH2 ; un radical CH(Et)-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(ii) A représente un radical CH(CH3) et B représente un radical (CH2)x dans lequel x = 1, 2, 3, 4 ou 5 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(iii) A représente un radical CH-R3 dans lequel R3 désigne un radical alkyle, linéaire ou ramifié, en C2-C5 et B représente un radical (CH2)x dans lequel x = 1, 2, 3, 4 ou 5 ; un radical C(CH3)2-CH2 ; un radical CH(Et)-CH2 ; un radical CH(COOH)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(iv) A représente un radical CH2-CH2 ou C(CH3)2-CH2 et B représente un radical (CH2)x dans lequel x = 1, 3 ou 4; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(v) A représente un radical CH2-CH(CH3) et B représente un radical (CH2)x dans lequel x = 1, 2, 3 ou 4 ; un radical C(CH3)2-CH2 ; un radical CH(Et)-CH2 ; un radical CH(COOH)-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(vi) A représente un radical CH2-CH-R4 dans lequel R4 désigne un radical phényle ou COO-CH3 ou COO-CH2-CH3 et B représente un radical (CH2)x dans lequel x = 1, 2, 3 ou 4 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(vii) A représente un radical CH2-CH(CH2-COOH) ou CH2-CH(NH-CH2-COOH) et B représente un radical (CH2)x dans lequel x = 1, 2, 3 ou 4 ; un radical C(CH3)2-CH2 ; un radical CH(Et)-CH2 ; un radical CH(COOH)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(viii) A représente un radical C(CH3)2 et B représente un radical (CH2)x dans lequel x = 1, 3, 4 ou 5 ; un radical C(CH3)2-CH2 ; un radical CH(Et)-CH2 ; un radical CH(CO-NHCH3)-CH2 ; un radical CH(CO-NHEt)-CH2 ; un radical CH(CO-NH-CH2-CHOH-CH3)-CH2 ; ou CH(CO-NR1R2)-CH2-CH2 dans lequel R1 et R2 représentent, indépendamment l'un de l'autre, un atome d'hydrogène ou un radical méthyle, éthyle ou 2-hydroxypropyle
(ix) A représente un radical C(Et)2 et B représente un radical (CH2)x dans lequel x = 1, 2, 3, 4 ou 5 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(x) A représente un radical CH2-C(CH3)2 ou CH(CH2-Ph) et B représente un radical (CH2)x dans lequel x = 1, 2, 3 ou 4 ; un radical C(CH3)2-CH2 ; un radical CH(Et)-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xi) A représente un radical CH2-C(Et)2 et B représente un radical (CH2)x dans lequel x = 1, 2, 3 ou 4 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xii) A représente un radical CH2-CH(NH2) et B représente un radical (CH2)x dans lequel x = 1, 3 ou 4 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xiii) A représente un radical CH2-CH(NHAc) et B représente un radical (CH2)x dans lequel x = 1, 3 ou 4 ; un radical C(CH3)2-CH2 ; un radical CH(Et)-CH2 ; un radical CH(COOH)-CH2-CH2 ; un radical CH(CO-NH-CH3)-CH2 ; un radical CH(CO-NH-Et)-CH2 ; un radical CH(CO-N(CH3)2)-CH2 ; un radical CH(CO-NR1R2)-CH2-CH2 dans lequel R1 et R2 représentent, indépendamment l'un de l'autre, un atome d'hydrogène ou un radical méthyle, éthyle ou 2-hydroxypropyle
(xiv) A représente un radical CH2-CH(NH-CO-CH2-CH2-CH(NH2)COOH) ou CH2-CH(NH-CO-CH2-NH2) ou CH2-CH(NH-CO-CH(NH2)-CH2-CH(COOH)2 ou CH2-CH(NH-CO-CH2-CH2-CH2-CH(NH2)COOH) et B représente un radical (CH2)x dans lequel x = 1, 2, 3 ou 4 ; un radical C(CH3)2-CH2 ; un radical CH(Et)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xv) A représente un radical (CH2)3 et B représente un radical (CH2)x dans lequel x = 1, 2 ou 3; un radical C(CH3)2-CH2 ; un radical CH(Et)-CH2 ; un radical CH(COOH)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xvi) A représente un radical (CH2)4 et B représente un radical (CH2)x dans lequel x = 1 ou 2; un radical C(CH3)2-CH2 ; un radical CH(Et)-CH2 ; un radical CH(COOH)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
(xvii) A représente un radical (CH2)5 et B représente un radical CH2
(xviii) A représente un radical CH-Ph et B représente un radical (CH2)x dans lequel x = 1, 2, 3,4 ou 5; un radical C(CH3)2-CH2 ; un radical CH(Et)-CH2 ; un radical CH(COOH)-CH2 ; un radical CH(COOH)-CH2-CH2 ; un radical CH(CO-NR1R2)-CH2 ou CH(CO-NR1R2)-CH2-CH2 dans lesquels R1 et R2 représentent, indépendamment l'un de l'autre, un atome d'hydrogène ou un radical méthyle, éthyle ou 2-hydroxypropyle
et les sels organiques et minéraux desdits composés de formule (II).

24. Dimercaptoamide selon la revendication 23, **caractérisé en ce qu'**il est choisi parmi les composés suivants :
- le 2-Mercapto-N-(2-mercapto-éthyl)-acétamide
- le 2-Mercapto-N-(3-mercapto-propyl)-acétamide
- le 2-Mercapto-N-(4-mercapto-butyl)-acétamide
- le 2-Mercapto-N-(5-mercapto-pentyl)-acétamide
- le 3-Mercapto-2-(2-mercapto-acétylamino)-propionamide
- le 3-Mercapto-2-(2-mercapto-acétylamino)-N,N-diméthyl-propionamide
- le 2-Mercapto-N-(2-mercapto-1,1-diméthyl-éthyl)-acétamide
- le 2-Mercapto-N-(1-mercaptométhyl-propyl)-acétamide
- le 2-Mercapto-N-mercaptométhy)-propionamide
- le 2-Mercapto-N-(2-mercapto-éthyl)-propionamide
- le 2-Mercapto-N-(3-rmercapto-propyl)-propionamide
- le 2-Mercapto-N-(4-mercapto-butyl)-propionamide
- le 2-Mercapto-N-(5-mercapto-pentyl)-propionamide
- le N-(1-Carbamoyl-2-mercapto-éthyl)-2-mercapto-propionamide
- le N-(1-Diméthylcarbamoyl-2-mercapto-éthyl)-2-mercapto-propionamide
- le 4-Mercapto-2-(2-mercapto-propionylamino)-butyric acid
- le 2-Mercapto-N-(2-mercapto-1,1-diméthyl-éthyl)-propionamide
- le 2-Mercapto-butanoic acid mercaptométhyl-amide
- le 2-Mercapto-butanoic acid (2-mercapto-éthyl)-amide
- le 2-Mercapto-butanoic acid (3-mercapto-propyl)-amide
- le 2-Mercapto-butanoic acid (4-mercapto-butyl)-amide
- le 2-Mercapto-butanoic acid (5-mercapto-pentyl)-amide
- le 2-Mercapto-butanoic acid (1-carbamoyl-2-mercapto-éthyl)-amide
- le N-(1-Diméthylcarbamoyl-2-mercapto-éthyl)-2-mercapto-butyramide
- le 2-Mercapto-butanoic acid (1-carbamoyl-3-mercapto-propyl)-amide
- le 2-Mercapto-butanoic acid (2-mercapto-1,1-diméthyl-éthyl)-amide
- le 2-Mercapto-pentanoic acid mercaptométhyl-amide
- le 2-Mercapto-pentanoic acid (2-mercapto-éthyl)-amide
- le 2-Mercapto-pentanoic acid (3-mercapto-propyl)-amide
- le 2-Mercapto-pentanoic acid (4-mercapto-butyl)-amide
- le 2-Mercapto-pentanoic acid (5-mercapto-pentyl)-amide
- le 3-Mercapto-2-(2-mercapto-pentanoylamino)-propionic acid
- le 2-Mercapto-pentanoic acid (1-carbamoyl-2-mercapto-éthyl)-amide
- le 2-Mercapto-pentanoic acid (1-diméthylcarbamoyl-2-mercapto-éthyl)-amide
- le 2-Mercapto-pentanoic acid (1-carbamoyl-3-mercapto-propyl)-amide
- le 2-Mercapto-pentanoic acid (2-mercapto-1,1-diméthyl-éthyl)-amide
- le 3-Mercapto-N-mercaptométhyl-propionamide
- le 3-Mercapto-N-(3-mercapto-propyl)-propionamide
- le 3-Mercapto-N-(4-mercapto-butyl)-propionamide
- le 3-Mercapto-2-(3-mercapto-propionylamino)-propionamide
- le N-(1-Diméthylcarbamoyl-2-mercapto-éthyl)-3-mercapto-propionamide
- le 4-Mercapto-2-(3-mercapto-propionylamino)-butyric acid
- le 3-Mercapto-N-(2-mercapto-1,1-diméthyl-éthyl)-propionamide
- le 3-Mercapto-N-mercaptométhyl-2-méthyl-propionamide
- le 3-Mercapto-N-(2-mercaptoéthyl)-2-méthyl-propionamide
- le 3-Mercapto-N-(3-mercapto-propyl)-2-méthyl-propionamide
- le 3-Mercapto-N-(4-mercapto-butyl)- 2-méthyl-propionamide
- le 3-Mercapto-2-(3-mercapto-2-méthyl-propionylamino)-propionic acid
- le N-(1-Carbamoyl-2-mercapto-éthyl)-3-mercapto-2-méthyl-propionamide
- le N-(1-Diméthylcarbamoyl-2-mercapto-éthyl)-3-mercapto-2-méthyl-propionamide
- le 3-Mercapto-N-(2-mercapto-1,1-diméthyl-éthyl)- 2-méthyl-propionamide
- le N-mercaptométhyl-3-mercaptométhyl-succinamic acid
- le N-(2-Mercapto-éthyl)-3-mercaptométhyl-succinamic acid
- le N-(3-Mercapto-propyl)-3-mercaptométhyl-succinamic acid
- le N-(4-Mercapto-butyl)-3-mercaptométhyl-succinamic acid
- le N-(1-Carboxy-2-mercapto-éthyl)-3-mercaptométhyl-succinamic acid
- le N-(1-Carboxy-3-mercapto-propyl)-3-mercaptométhyl-succinamic acid
- le. N-(2-Mercapto-1,1-diméthyl-éthyl)-3-mercaptométhyl-succinamic acid
- le N-(2-Mercapto-1-éthyl)-3-mercaptométhyl-succinamic acid
- le 2-Mercapto-N-mercaptométhyl-2-méthyl-propionamide
- le 2-Mercapto-N-(3-mercapto-propyl)-2-méthyl-propionamide
- le 2-Mercapto-N-(4-mercapto-butyl)-2-méthyl-propionamide
- le 2-Mercapto-N-(5-mercapto-pentyl)-2-méthyl-propionamide
- le 2-Mercapto-N-(2-mercapto-1,1-diméthyl-éthyl)-2-méthyl-propionamide
- le 2-Mercapto-N-(2-mercapto-1-éthyl)-2-méthyl-propionamide
- le 3-Mercapto-N-mercaptométhyl-3-méthyl-butyramide
- le 3-Mercapto-N-(3-mercapto-propyl)-3-méthyl-butyramide
- le 3-Mercapto-N-(4-mercapto-butyl)-3-méthyl-butyramide
- le N-(1-Carbamoyl-2-mercapto-éthyl)-3-mercapto-3-méthyl-butyramide
- le N-(1-Diméthylcarbamoyl-2-mercapto-éthyl)-3-mercapto-3-méthyl-butyramide
- le 4-Mercapto-2-(3-mercapto-3-méthyl-butyrylamino)-butyric acid
- le 3-Mercapto-N-(2-mercapto-1,1-diméthyl-éthyl)-3-méthyl-butyramide
- le 3-Mercapto-N-(2-mercapto-1-éthyl)-3-méthyl-butyramide
- le 3-Mercapto-N-mercaptométhyl-2,2-diméthyl-propionamide
- le 3-Mercapto-N-(2-mercaptoéthyl)-2,2-diméthyl-propionamide
- le 3-Mercapto-N-(3-mercapto-propyl)-2,2-diméthyl-propionamide
- le 3-Mercapto-N-(4-mercapto-butyl)- 2,2-diméthyl-propionamide
- le 3-Mercapto-N-(2-mercapto-1,1-diméthyl-éthyl)- 2,2-diméthyl-propionamide
- le N-(1-Carbamoyl-2-mercapto-éthyl)-3-mercapto-2,2-diméthyl-propionamide
- le N-(1-Diméthylcarbamoyl-2-mércapto-éthyl)-3-mercapto-2,2-diméthyl-propionamide
- le 2-Amino-3-mercapto-N-mercaptométhyl-propionamide
- le 2-Amino-3-mercapto-N-(3-mercapto-propyl)-propionamide
- le 2-Amino-3-mercapto-N-(4-mercapto-butyl)-propionamide
- le 2-Amino-N-(1-carbamoyl-2-mercapto-éthyl)-3-mercapto-propionamide
- le 2-Amino-N-(1-carbamoyl-2-mercapto-éthyl)-3-mercapto-N,N-diméthyl-propionamide
- le 2-(2-Amino-3-mercapto-propionylamino)-4-mercapto-butyric acid
- le 2-Amino-3-mercapto-N-(2-mercapto-1,1-diméthyl-éthyl)-propionamide
- le 2-Amino-3-mercapto-N-(2-mercapto-1-éthyl)-propionamide
- le 2-Acétylamino-3-mercapto-N-mercaptométhyl-propionamide
- le 2-Acétylamino-3-mercapto-N-(3-mercapto-propyl)-propionamide
- le 2-Acétylamino-3-mercapto-N-(4-mercapto-butyl)-propionamide
- le 2-(2-Acétylamino-3-mercapto-propionylamino)-3-mercapto-3-méthyl-butyric acid
- le 2-Acétylamino-3-m,ercapto-N-(1-mercaptométhyl-propyl)-propionamide
- le 2-Acétylamino-N-(1-diméthylcarbamoyl-2-mercapto-éthyl)-3-mercapto-propionamide
- le 2-(2-Acétylamino-3-mercapto-propionylamino)-4-mercapto-butyric acid
- le 2-(carboxyméthylamino)-3-mercapto-N-mercaptométhyl-propionamide
- le 2-(carboxyméthylamino)-3-mercapto-N-(2-mercapto-éthyl)-propionamide
- le 2-(carboxyméthylamino)-3-mercapto-N-(3-mercapto-propyl)-propionamide
- le 2-(carboxyméthylamino)-3-mercapto-N-(4-mercapto-butyl)-propionamide
- le 2-(carboxyméthylamino)-N-(1-carbamoyl-2-mercapto-éthyl)-3-mercapto-propionamide
- le 2-[2-(Carboxyméthyl-amino)-3-mercapto-propionylamino]-3-mercapto-propionic acid
- le [1-(1-Carbamoyl-2-mercapto-éthylcarbamoyl)-2-mercapto-éthylamino]-acetic acid
- le 2-(carboxyméthylamino)-3-mercapto-N-(2-mercapto-1,1-diméthyl-éthyl)-propionamide
- le 2-Amino-4-[2-mercapto-1-(mercaptométhyl-carbamoyl)-éthylcarbamoyl]-butyric acid
- le 2-Amino-4-[2-mercapto-1-(2-mercapto-éthylcarbamoyl)-éthylcarbamoyl]-butyric acid
- le 2-Amino-4-[2-mercapto-1-(3-mercapto-propylcarbamoyl)-éthylcarbamoyl]-butyric acid
- le 2-Amino-4-[2-mercapto-1-(4-mercapto-butylcarbamoyl)-éthylcarbamoyl]-butyric acid
- le 2-[2-(4-Amino-4-carboxy-butyrylamino)-3-mercapto-propionylamino]-4-mercapto-butyric acid
- le 2-Amino-4-[1-(1-carbamoyl-2-mercapto-éthylcarbamoyl)-2-mercapto-éthylcarbamoyl]-butyric acid
- le 2-Amino-4-[1-(1-diméthylcarbamoyl-2-mercapto-éthylcarbamoyl)-2-mercapto-éthylcarbamoyl]-butyric acid
- le 2-Amino-4-[2-mercapto-1-(2-mercapto-1,1-diméthyl-éthylcarbamoyl)-éthylcarbamoyl]-butyric acid
- le 2-Amino-4-[2-mercapto-1-(2-mercapto-1-éthyl-éthylcarbamoyl)-éthylcarbamoyl]-butyric acid
- le 2-(2-Amino-acétylamino)-3-mercapto-N-mercaptométhyl-propionamide
- le 2-(2-Amino-acétylamino)-3-mercapto-N-(2-mercapto-éthyl)-propionamide
- le 2-(2-Amino-acétylamino)-3-mercapto-N-(3-mercapto-propyl)-propionamide
- le 2-(2-Amino-acétylamino)-3-mercapto-N-(4-mercapto-butyl)-propionamide
- le 2-(2-Amino-acétylamino)-3-mercapto-N-(5-mercapto-pentyl)- propionamide
- le 2- [2-(2-Amino-acétylamino)-3 -mercapto-propionylamino] - 4-mercapto-butyric acid
- le 2-(2-Amino-acétylamino)-3-mercapto-N-(2-mercapto-1,1-diméthyl-éthyl)-propionamide
- le 2-(2-Amino-acétylamino)-3-mercapto-N-(1-mercaptométhyl-propyl)-propionamide
- le 4-Mercapto-N-mercaptométhyl-butyramide
- le 4-Mercapto-N-(2-mercapto-éthyl)-butyramide
- le 4-Mercapto-N-(3-mercapto-propyl)-butyramide
- le N-(4-mercapto-1-oxobutyl)-L-Cystéine
- le 4-Mercapto-2-(3-mercapto-propionylamino)-butyramide
- le N-(1-Diméthylcarbamoyl-2-mercapto-éthyl)-4-mercapto-butyramide
- le N-(4-mercapto-1-oxobutyl)-L-homocystéine
- le 4-Mercapto-N-(2-mercapto-1,1-diméthyl-éthyl)-butyramide
- le 4-Mercapto-N-(1-mercaptométhyl-propyl)-butyramide
- le 5-Mercapto-pentanoic acid mercaptométhyl-amide
- le 5-Mercapto-pentanoic acid (2-mercapto-éthyl)-amide
- le N-(5-mercapto-1-oxopentyl)-L-Cystéine
- le 5-Mercapto-2-(3-mercapto-propionylamino)-pentanamide
- le N-(1-Diméthylcarbamoyl-2-mercapto-éthyl)-5-mercapto-pentanamide
- le 5-Mercapto-N-(2-mercapto-1,1-diméthyl-éthyl)-pentanamide
- le 5-Mercapto-N-(1-mercaptométhyl-propyl)-pentanamide
- le 6-Mercapto-hexanoic acid mercaptométhylamide

25. Dimercaptoamide selon la revendication 23, **caractérisé en ce qu'**il est choisi parmi les composés suivants :
- le 2-Mercapto-N-(2-mercapto-éthyl)-acétamide
- le 2-Mercapto-N-(3-mercapto-propy_1)-acétamide
- le 2-Mercapto-N-(4-mercapto-butyl)-acétamide
- le 2-Mercapto-N-(5-mercapto-pentyl)-acétamide
- le 3-Mercapto-2-(2-mercapto-acétylamino)-propionamide
- le 3-Mercapto-2-(2-mercapto-acétylamino)-N,N-diméthyl-propionamide
- le 2-Mercapto-N-(2-mercapto-1,1-diméthyl-éthyl)-acétamide
- le 2-Mercapto-N-(1-mercaptométhyl-propyl)-acétamide
- le 2-Mercapto-N-mercaptométhyl-propionamide
- le 2-Mercapto-N-(2-mercapto-éthyl)-propionamide
- le 2-Mercapto-N-(3-mercapto-propyl)-propionamide
- le 2-Mercapto-N-(4-mercapto-butyl)-propionamide
- le 2-Mercapto-N-(5-mercapto-pentyl)-propionamide
- le N-(1-Carbamoyl-2-mercapto-éthyl)-2-mercapto-propionamide
- le N-(1-Diméthylcarbamoyl-2-mercapto-éthyl)-2-mercapto-propionamide
- le 4-Mercapto-2-(2-mercapto-propionylamino)-butyric acid
- le 2-Mercapto-N-(2-mercapto-1,1-diméthyl-éthyl)-propionamide
- le 2-Mercapto-butanoic acid mercaptométhyl-amide
- le 2-Mercapto-butanoic acid (2-mercapto-éthyl)-amide
- le 2-Mercapto-butanoic acid (3-mercapto-propyl)-amide
- le 2-Mercapto-butanoic acid (4-mercapto-butyl)-amide
- le 2-Mercapto-pentanoic acid mercaptométhyl-amide
- le 2-Mercapto-pentanoic acid (2-mercapto-éthyl)-amide
- le 2-Mercapto-pentanoic acid (3-mercapto-propyl)-amide
- le 3-Mercapto-N-mercaptométhyl-propionamide
- le 3-Mercapto-N-(3-mercapto-propyl)-propionamide
- le 3-Mercapto-2-(3-mercapto-propionylamino)-propionamide
- le N-(1-Diméthylcarbamoyl-2-mercapto-éthyl)-3-mercapto-propionamide
- le 4-Mercapto-2-(3-mercapto-propionylamino)-butyric acid
- le 3-Mercapto-N-(2-mercapto-1,1-diméthyl-éthyl)-propionamide
- le 3-Mercapto-N-mercaptométhyl-2-méthyl-propionamide
- le 3-Mercapto-N-(2-mercaptoéthyl)-2-méthyl-propionamide
- le 3-Mercapto-N-(3-mercapto-propyl)-2-méthyl-propionamide
- le 3-Mercapto-N-(4-mercapto-butyl)- 2-méthyl-propionamide
- le 3-Mercapto-2-(3-mercapto-2-méthyl-propionylamino)-propionic acid
- le N-(1-Carbamoyl-2-mercapto-éthyl)-3-mercapto-2-méthyl-propionamide
- le N-(1-Diméthylcarbamoyl-2-mercapto-éthyl)-3-mercapto-2-méthyl-propionamide
- le 3-Mercapto-N-(2-mercapto-1,1-diméthyl-éthyl)- 2-méthyl-propionamide
- le N-mercaptométhyl-3-mercaptométhyl-succinamic acid
- le N-(2-Mercapto-éthyl)-3-mercaptométhyl-succinamic acid
- le N-(3-Mercapto-propyl)-3-mercaptométhyl-succinamic acid
- le N-(4-Mercapto-butyl)-3-mercaptométhyl-succinamic acid
- le N-(1-Carboxy-2-mercapto-éthyl)-3-mercaptométhyl-succinamic acid
- le 2-Mercapto-N-mercaptométhyl-2-méthyl-propionamide
- le 2-Mercapto-N-(3-mercapto-propyl)-2-méthyl-propionamide
- le 2-Mercapto-N-(4-mercapto-butyl)-2-méthyl-propionamide
- le 2-Mercapto-N-(2-mercapto-1,1-diméthyl-éthyl)-2-méthyl-propionamide
- le 2-Mercapto-N-(2-mercapto-1-éthyl)-2-méthyl-propionamide
- le 3-Mercapto-N-mercaptométhyl-3-méthyl-butyramide
- le 3-Mercapto-N-(3-mercapto-propyl)-3-méthyl-butyramide
- le N-(1-Carbamoyl-2-mercapto-éthyl)-3-mercapto-3-méthyl-butyramide
- le N-(1-Diméthylcarbamoyl-2-mercapto-éthyl)-3-mercapto-3-méthyl-butyramide
- le 4-Mercapto-2-(3-mercapto-3-méthyl-butyrylamino)-butyric acid
- le 3-Mercapto-N-mercaptométhyl-2,2-diméthyl-propionamide
- le 3-Mercapto-N-(2-mercaptoéthyl)-2,2-diméthyl-propionamide
- le 3-Mercapto-N-(3-mercapto-propyl)-2,2-diméthyl-propionamide
- le 3-Mercapto-N-(4-mercapto-butyl)- 2,2-diméthyl-propionamide
- le N-(1-Carbamoyl-2-mercapto-éthyl)-3-mercapto-2,2-diméthyl-propionamide
- le N-(1-Diméthylcarbamoyl-2-mercapto-éthyl)-3-mercapto-2,2-diméthyl-propionamide
- le 2-Amino-3-mercapto-N-mercaptométhyl-propionamide
- le 2-Amino-3-mercapto-N-(3-mercapto-propyl)-propionamide
- le 2-Amino-3-mercapto-N-(4-mercapto-butyl)-propionamide
- le 2-Amino-N-(1-carbamoyl-2-mercapto-éthyl)-3-mercapto-propionamide
- le 2-Amino-N-(1-carbamoyl-2-mercapto-éthyl)-3-mercapto-N,N-diméthyl-propionamide
- le 2-(2-Amino-3-mercapto-propionylamino)-4-mercapto-butyric acid
- le 2-Amino-3-mercapto-N-(2-mercapto-1,1-diméthyl-é.thyl)-propionamide
- le.2-Amino-3-inercapto-N-(2-mercapto-1-éthyl)-propionamide
- le 2-Acétylamino-3-mercapto-N-mercaptométhyl-propionamide
- le 2-(carboxyméthylamino)-3-mercapto-N-mercaptométhyl-propionamide
- le 2-(carboxyméthylamino)-3-mercapto-N-(2-mercapto-éthyl)-propionamide
- le 2-(carboxyméthylamino)-3-inercapto-N-(3-mercapto-propyl)-propionamide
- le 2-(2-Amino-acétylamino)-3-mercapto-N-mercaptométhyl-propionamide
- le 2-(2-Amino-acétylamino)-3-mercapto-N-(2-mercapto-éthyl)-propionamide
- le 4-Mercapto-N-mercaptométhyl-butyramide
- le 4-Mercapto-N-(2-mercapto-éthyl)-butyramide
- le 4-Mercapto-N-(3-mercapto-propyl)-butyramide
- le N-(4-mercapto-1-oxobutyl)-L-Cystéine
- le 4-Mercapto-2-(3-mercapto-propionylamino)-butyramide
- le N-(1-Diméthylcarbamoyl-2-mercapto-éthyl)-4-mercapto-butyramide
- le 4-Mercapto-N-(2-mercapto-1,1-diméthyl-éthyl)-butyramide
- le 4-Mercapto-N-(1-mercaptométhyl-propyl)-butyramide
- le 5-Mercapto-pentanoic acid mercaptométhyl-amide
- le 5-Mercapto-pentanoic acid (2-mercapto-éthyl)-amide
- le N-(5-mercapto-1-oxopentyl)-L-Cystéine
- le 5-Mercapto-2-(3-mercapto-propionylamino)-pentanamide
- le N-(1-Diméthylcarbamoyl-2-mercapto-éthyl)-5-mercapto-pentanamide
- le 5-Mercapto-N-(2-mercapto-1,1-diméthyl-éthyl)-pentanamide
- le 5-Mercapto-N-(1-mercaptométhyl-propyl)-pentanamide
- le 6-Mercapto-hexanoic acid mercaptométhyl-amide.

26. Composition réductrice, **caractérisée en ce qu'**elle comprend au moins un dimercaptoamide selon l'une des revendications 23 à 25.

27. Procédé de préparation d'un dimercaptoamide de formule (II) selon la revendication 23, **caractérisé en ce qu'**il comprend la réaction d'un mercapto halogénure d'acide protégé de formule P-S-A-CO-X, avec un aminothiol protégé de formule H₂N-B-S-P, A et B ayant la même signification que dans la revendication 23, P étant un groupe protecteur de la fonction thiol, et X étant le chlore ou le brome, dans un solvant aprotique, en présence d'un agent destiné à capter l'acide chlorhydrique ou bromhydrique libéré, tel que par exemple une amine tertiaire, de façon à former un dipercaptoamide protégé de formule P-S-A-CO-NH-B-S-P, puis la déprotection du dipercaptoamide protégé, par hydrolyse ou par réduction.

28. Procédé de préparation d'un dimercaptoamide de formule (II) selon la revendication 23, **caractérisé en ce qu'**il comprend la réaction d'un mercaptoacide de formule HS-A-COOH avec un aminothiol de formule H₂N-B-SH, A et B ayant la même signification que dans la revendication 23, en présence d'un agent de couplage et/ou de déshydratation, dans un solvant aprotique.

29. Procédé de préparation d'un dimercaptoamide de formule (II) selon la revendication 23, **caractérisé en ce qu'**il comprend la réaction d'un mercaptoester de formule HS-A-COOR, R étant un radical méthyle ou éthyle, avec un aminothiol de formule H₂N-B-SH, A et B ayant la même signification que dans la revendication 23, à une température comprise entre 30°C et 180°C.

30. Procédé de préparation d'un dimercaptoamide de formule (II) selon la revendication 23, **caractérisé en ce qu'**il comprend :
a) la réaction d'un halogénure d'acide halogéné de formule X-A-CO-Cl avec une halogénoamine de formule H₂N-B-X, X représentant un atome de chlore ou de brome, A et B ayant la même signification que dans la revendication 23, dans un solvant aprotique et en présence d'un agent destiné à capter l'acide chlorhydrique ou bromhydrique libéré, de façon à former un dihalogénoamide de formule X-A-CO-NH-B-X, puis
b) la transformation du dihalogénoamide obtenu en dimercaptoamide de formule (II), soit par réaction directe avec de l'hydrogène sulfuré dans un solvant approprié, soit par réaction avec l'acide thioacétique, de préférence sous forme salifiée, suivie d'une hydrolyse acide ou alcaline du di(S-acétyl)mercaptoamide, soit encore par réaction avec la thiourée suivie d'une hydrolyse du di sel d'isothiouronium.

31. Procédé de préparation d'un dimercaptoamide de formule (II) selon la revendication 23, **caractérisé en ce qu'**il comprend la réaction d'une thiolactone de formule 11 avec un aminothiol de formule 5, dans un solvant aprotique, selon la réaction suivante : 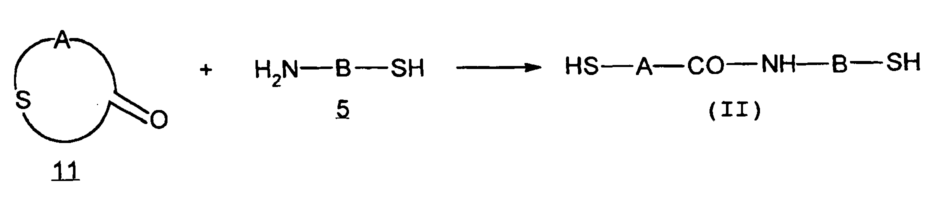 A et B ayant la même signification que dans la revendication 23.

32. Procédé de préparation d'un dimercaptoamide de formule (II) selon la revendication 23, **caractérisé en ce qu'**il comprend la réaction d'un mercaptoamide de formule HS-A-CO-NH₂ avec un sel d'aminothiol de formule HX, H₂N-B-SH, X représentant un atome de chlore ou brome, A et B ayant la même signification que dans la revendication 23, à une température comprise entre 60 et 200°C.

## Claims

1. Use, as a reducing agent for permanently reshaping keratin fibres, and in particular human keratin fibres such as the hair, of a dimercaptoamide of general formula (I) below:
HS-A-CO-NH-B-SH (I)
in which:
A represents a radical (CH₂)ₙ, n being an integer between 1 and 5, optionally substituted with:
(i) a linear or branched C₁-C₅ alkyl radical; phenyl; benzyl; amino; acetylamino; NH-CO-CH₂-NH₂; NH-CO-CH₂-CH₂-CH (NH₂) COOH; NH-CO-CH₂-CH₂-CH(COOH) COOH; CH₂-COOH; CH₂-COOCH₃; or CH₂-COOCH₂-CH₃, or
(ii) two methyl radicals or two ethyl radicals,
and B represents a radical (CH₂)_{P}, p being an integer between 1 and 5, optionally substituted with:
(i) a linear or branched C₁-C₅ alkyl radical; carboxyl; COOCH₃; COOEt; CONH₂; CONH-CH₃; CON (CH₃)₂; CONH-CH₂-CH₃; CON (CH₂-CH₃)₂; CONH-CH₂-CHOH-CH₃; CO-NH-CH(COOH)-(CH₂)₄-NH₂; CO-NH-CH(COOH)-(CH₂)₄-N(CH₃)₂; CO-NH-CH₂-CH₂-COOEt; CO-NH-CH(COOH)-iPr; CO-NH-CH₂-R with R = CO-NH₂ or (CH₂)₃-NH₂ or (CH₂)₄-NH₂ or (CH₂)₅-NH₂ or (CH₂)₄-OH, or
(ii) 2 methyl radicals,
the sum of n+p being between 2 and 6, and their organic and inorganic salts.

2. Use according to Claim 1, **characterized in that** the dimercaptoamide of formula (I) is chosen from the following compounds:
- 2-mercapto-N-(mercaptomethyl)acetamide
- 2-mercapto-N-(2-mercaptoethyl)acetamide
- 2-mercapto-N-(3-mercaptopropyl)acetamide
- 2-mercapto-N-(4-mercaptobutyl)acetamide
- 2-mercapto-N-(5-mercaptopentyl)acetamide
- 3-mercapto-2-(2-mercaptoacetylamino)propionamide
- 3-mercapto-2-(2-mercaptoacetylamino)-N,N-dimethyl-propionamide
- 2-mercapto-N-(2-mercapto-1,1-dimethylethyl)acetamide
- 2-mercapto-N-(1-mercaptomethylpropyl)acetamide
- ethyl mercapto[(mercaptoacetyl)amino]acetate
- N-(mercaptoacetyl)-L-cysteine
- N-(mercaptoacetyl)-L-homocysteine
- N-(mercaptoacetyl)-L-homocysteine, sodium salt
- 2-mercapto-N-mercaptomethylpropionamide
- 2-mercapto-N-(2-mercaptoethyl)propionamide
- 2-mercapto-N-(3-mercaptopropyl)propionamide
- 2-mercapto-N-(4-mercaptobutyl)propionamide
- 2-mercapto-N-(5-mercaptopentyl)propionamide
- mercapto-[(2-mercapto-1-oxopropyl)amino]acetic acid
- N-(2-mercapto-1-oxopropyl)-DL-cysteine
- (R)-N-(2-mercapto-1-oxopropyl)-L-cysteine
- (S)-N-(2-mercapto-1-oxopropyl)-L-cysteine
- 2-mercaptopropionyl-L-cysteine
- N-(1-carbamoyl-2-mercaptoethyl)-2-mercaptopropionamide
- N-(1-dimethylcarbamoyl-2-mercaptoethyl)-2-mercaptopropionamide
- 4-mercapto-2-(2-mercaptopropionylamino)butyric acid
- 2-mercapto-N-(2-mercapto-1,1-dimethylethyl)propionamide
- 2-mercaptobutanoic acid mercaptomethylamide
- 2-mercaptobutanoic acid (2-mercaptoethyl)amide
- 2-mercaptobutanoic acid (3-mercaptopropyl)amide
- 2-mercaptobutanoic acid (4-mercaptobutyl)amide
- 2-mercaptobutanoic acid (5-mercaptopentyl)amide
- 2-mercaptobutanoic acid (1-carbamoyl-2-mercaptoethyl)amide
- N-(1-dimethylcarbamoyl-2-mercaptoethyl)-2-mercaptobutyramide
- 2-mercaptobutanoic acid (1-carbamoyl-3-mercaptopropyl)amide
- 2-mercaptobutanoic acid (2-mercapto-1,1-dimethylethyl)amide
- 2-mercaptopentanoic acid mercaptomethylamide
- 2-mercaptopentanoic acid (2-mercaptoethyl)amide
- 2-mercaptopentanoic acid (3-mercaptopropyl)amide
- 2-mercaptopentanoic acid (4-mercaptobutyl)amide
- 2-mercaptopentanoic acid (5-mercaptopentyl)amide
- 3-mercapto-2-(2-mercaptopentanoylamino)propionic acid
- 2-mercaptopentanoic acid (1-carbamoyl-2-mercaptoethyl)amide
- 2-mercaptopentanoic acid (1-dimethylcarbamoyl-2-mercaptoethyl)amide
- 2-mercaptopentanoic acid (1-carbamoyl-3-mercaptopropyl)amide
- 2-mercaptopentanoic acid (2-mercapto-1,1-dimethylethyl)amide
- ethyl mercapto[(mercaptophenylacetyl)amino]acetate
- (R)-N-[2-(mercaptomethyl)-1-oxo-3-phenylpropyl]-L-cysteine
- (R)-N-[2-(mercaptomethyl)-1-oxo-3-phenylpropyl]-L-homocysteine
- (S)-N-[2-(mercaptomethyl)-1-oxo-3-phenylpropyl]-L-homocysteine
- 3-mercapto-N-(2-mercaptoethyl)propanamide
- 3-mercapto-N-mercaptomethylpropionamide
- 3-mercapto-N-(3-mercaptopropyl)propionamide
- 3-mercapto-N-(4-mercaptobutyl)propionamide
- N-(3-mercapto-1-oxopropyl)-L-cysteine
- 3-mercapto-2-(3-mercaptopropionylamino)propionamide
- N-(1-dimethylcarbamoyl-2-mercaptoethyl)-3-mercaptopropionamide
- 4-mercapto-2-(3-mercaptopropionylamino)butyric acid
- 3-mercapto-N-(2-mercapto-1,1-dimethylethyl)propionamide
- 3-mercapto-N-mercaptomethyl-2-methylpropionamide
- 3-mercapto-N-(2-mercaptoethyl)-2-methylpropionamide
- 3-mercapto-N-(3-mercaptopropyl)-2-methylpropionamide
- 3-mercapto-N-(4-mercaptobutyl)-2-methylpropionamide
- 3-mercapto-2-(3-mercapto-2-methylpropionylamino)-propionic acid
- N-(1-carbamoyl-2-mercaptoethyl)-3-mercapto-2-methylpropionamide
- N-(1-dimethylcarbamoyl-2-mercaptoethyl)-3-mercapto-2-methylpropionamide
- 3-mercapto-N-(2-mercapto-1,1-dimethylethyl)-2-methylpropionamide
- N-(3-mercapto-2-methyl-l-oxopropyl)-L-homocysteine
- N-[2-(mercaptomethyl)-1-oxo-3-phenylpropyl]cysteine
- methyl 4-[(1-carboxy-2-mercaptoethyl)amino]-3-(mercaptomethyl)-4-oxobutanoate
- N-mercaptomethyl-3-mercaptomethylsuccinamic acid
- N-(2-mercaptoethyl)-3-mercaptomethylsuccinamic acid
- N-(3-mercaptopropyl)-3-mercaptomethylsuccinamic acid
- N-(4-mercaptobutyl)-3-mercaptomethylsuccinamic acid
- N-(1-carboxy-2-mercaptoethyl)-3-mercaptomethylsuccinamic acid
- N-(1-carboxy-3-mercaptopropyl)-3-mercaptomethylsuccinamic acid
- N-(2-mercapto-1,1-dimethylethyl)-3-mercaptomethylsuccinamic acid
- N-(2-mercapto-1-ethyl)-3-mercaptomethylsuccinamic acid
- 2-mercapto-N-mercaptomethyl-2-methylpropionamide
- 2-mercapto-N-(2-mercaptoethyl)-2-methylpropanamide
- 2-mercapto-N-(3-mercaptopropyl)-2-methylpropionamide
- 2-mercapto-N-(4-mercaptobutyl)-2-methylpropionamide
- 2-mercapto-N-(5-mercaptopentyl)-2-methylpropionamide
- 2-mercapto-N-(2-mercapto-1,1-dimethylethyl)-2-methylpropionamide
- 2-mercapto-N-(2-mercapto-1-ethyl)-2-methylpropionamide
- N-(2-mercapto-2-methyl-1-oxopropyl)-D-cysteine
- N-(2-mercapto-2-methyl-1-oxopropyl)cysteine
- N-(2-mercapto-2-methyl-1-oxopropyl)-L-cysteine, monosodium salt
- N-(2-mercapto-2-methyl-1-oxopropyl)-L-cysteine
- (R)-N-[2-amino-1-(mercaptomethyl)-2-oxoethyl]-2-mercapto-2-methylpropanamide
- methyl N-(2-mercapto-2-methyl-1-oxopropyl)-L-cysteinate
- (2R)-3-mercapto-2-[(2-mercapto-2-methyl-1-oxopropyl)amino]-N,N-dimethylpropanamide
- N2-[N-(2-mercapto-2-methyl-1-oxopropyl)-L-cysteinyl]-L-lysine
- N-(2-mercapto-2-methyl-1-oxopropyl)homocysteine
- N-(2-ethyl-2-mercapto-1-oxobutyl)-L-cysteine
- 3-mercapto-N-mercaptomethyl-3-methylbutyramide
- 3-mercapto-N-(2-mercaptoethyl)-3-methylbutanamide
- 3-mercapto-N-(3-mercaptopropyl)-3-methylbutyramide
- 3-mercapto-N-(4-mercaptobutyl)-3-methylbutyramide
- N-(3-mercapto-3-methyl-1-oxobutyl)-L-cysteine
- N-(1-carbamoyl-2-mercaptoethyl)-3-mercapto-3-methylbutyramide
- N-(1-dimethylcarbamoyl-2-mercaptoethyl)-3-mercapto-3-methylbutyramide
- 4-mercapto-2-(3-mercapto-3-methylbutyrylamino)butyric acid
- 3-mercapto-N-(2-mercapto-1,1-dimethylethyl)-3-methylbutyramide
- 3-mercapto-N-(2-mercapto-1-ethyl)-3-methylbutyramide
- 3-mercapto-N-mercaptomethyl-2,2-dimethylpropionamide
- 3-mercapto-N-(2-mercaptoethyl)-2,2-dimethylpropionamide
- 3-mercapto-N-(3-mercaptopropyl)-2,2-dimethylpropionamide
- 3-mercapto-N-(4-mercaptobutyl)-2,2-dimethylpropionamide
- 3-mercapto-N-(2-mercapto-1,1-dimethylethyl)-2,2-dimethylpropionamide
- N-(3-mercapto-2,2-dimethyl-1-oxopropyl)-D-cysteine
- N-(3-mercapto-2,2-dimethyl-1-oxopropyl)-L-cysteine
- N-(1-carbamoyl-2-mercaptoethyl)-3-mercapto-2,2-dimethylpropionamide
- N-(1-dimethylcarbamoyl-2-mercaptoethyl)-3-mercapto-2,2-dimethylpropionamide
- N-(3-mercapto-2,2-dimethyl-1-oxopropyl)-D-cysteinylglycinamide
- N6-[(1,1-dimethylethoxy)carbonyl]-N2-[N-(3-mercapto-2,2-dimethyl-1-oxopropyl)-L-cysteinyl]-L-lysine
- N-[2-[(4-aminobutyl)amino]-1-(mercaptomethyl)-2-oxoethyl]-3-mercapto-2,2-dimethylpropanamide, monohydrochloride
- N-[2-[(5-aminopentyl)amino]-1-(mercaptomethyl)-2-oxoethyl]-3-mercapto-2,2-dimethylpropanamide, monohydrochloride
- N-[2-[(6-aminohexyl)amino]-1-(mercaptomethyl)-2-oxoethyl]-3-mercapto-2,2-dimethylpropanamide, monohydrochloride
- (R)-N-[2-[(5-hydroxypentyl)amino]-1-(mercaptomethyl)-2-oxoethyl]-3-mercapto-2,2-dimethylpropanamide
- N2-[N-(3-mercapto-2,2-dimethyl-1-oxopropyl)-L-cysteinyl]-L-lysine
- N2-[N-(3-mercapto-2,2-dimethyl-1-oxopropyl)-L-cysteinyl]-N6,N6-dimethyl-L-lysine
- 3-mercapto-N-(3-mercapto-2,2-dimethyl-1-oxopropyl)-D-valine
- N-(3-mercapto-2,2-dimethyl-1-oxopropyl)homocysteine
- N-[2-ethyl-2-(mercaptomethyl)-1-oxobutyl]-L-cysteine
- 2-amino-3-mercapto-N-mercaptomethylpropionamide
- (2R)-2-amino-3-mercapto-N-(2-mercaptoethyl)propanamide
- (2S)-2-amino-3-mercapto-N-(2-mercaptoethyl)propanamide
- 2-amino-3-mercapto-N-(3-mercaptopropyl)propionamide
- 2-amino-3-mercapto-N-(4-mercaptobutyl)propionamide
- L-cysteinyl-L-cysteine
- methyl mercapto[(mercaptoacetyl)amino]acetate
- ethyl L-cysteinyl-L-cysteinate
- 2-amino-N-(1-carbamoyl-2-mercaptoethyl)-3-mercaptopropionamide
- 2-amino-N-(1-carbamoyl-2-mercaptoethyl)-3-mercapto-N,N-dimethylpropionamide
- 2-(2-amino-3-mercaptopropionylamino)-4-mercaptobutyric acid
- 2-amino-3-mercapto-N-(2-mercapto-1,1-dimethylethyl)-propionamide
- 2-amino-3-mercapto-N-(2-mercapto-1-ethyl)propionamide
- 2-acetylamino-3-mercapto-N-mercaptomethylpropionamide
- (2R)-2-(acetylamino)-3-mercapto-N-(2-mercaptoethyl)-propanamide
- 2-acetylamino-3-mercapto-N-(3-mercaptopropyl)propionamide
- 2-acetylamino-3-mercapto-N-(4-mercaptobutyl)propionamide
- 2-(2-acetylamino-3-mercaptopropionylamino)-3-mercapto-3-methylbutyric acid
- 2-acetylamino-3-mercapto-N-(1-mercaptomethylpropyl)-propionamide
- 2-acetylamino-N-(1-dimethylcarbamoyl-2-mercaptoethyl)-3-mercaptopropionamide
- N-acetyl-L-cysteinyl-L-cysteine
- N-(N-acetylcysteinyl)-L-cysteine
- N-(N-acetylcysteinyl)-DL-cysteine
- ethyl N-acetyl-L-cysteinyl-D-cysteinate
- ethyl N-acetyl-L-cysteinyl-L-cysteinate
- 2-acetamido-N-(1-carbamoyl-2-mercaptoethyl)-3-mercaptopropionamide
- ethyl N-acetyl-L-cysteinyl-L-cysteinyl-glycinate
- 2-(2-acetylamino-3-mercaptopropionylamino)-4-mercaptobutyric acid
- 2-(carboxymethylamino)-3-mercapto-N-mercaptomethylpropionamide
- 2-(carboxymethylamino)-3-mercapto-N-(2-mercaptoethyl)propionamide
- 2-(carboxymethylamino)-3-mercapto-N-(3-mercaptopropyl)propionamide
- 2-(carboxymethylamino)-3-mercapto-N-(4-mercaptobutyl)propionamide
- 2-(carboxymethylamino)-N-(1-carbamoyl-2-mercaptoethyl)-3-mercaptopropionamide
- 2-[2-(carboxymethylamino)-3-mercaptopropionylamino]-3-mercaptopropionic acid
- [1-(1-carbamoyl-2-mercaptoethylcarbamoyl)-2-mercaptoethylamino]acetic acid
- 2-(carboxymethylamino)-3-mercapto-N-(2-mercapto-1,1-dimethylethyl)propionamide
- N-[N-[N-(carboxymethyl)-L-cysteinyl]-L-cysteinyl]-D-valine
- 2-amino-4-[2-mercapto-1-(mercaptomethylcarbamoyl)-ethylcarbamoyl]butyric acid
- 2-amino-4-[2-mercapto-1-(2-mercaptoethylcarbamoyl)-ethylcarbamoyl]butyric acid
- 2-amino-4-[2-mercapto-1-(3-mercaptopropylcarbamoyl)-ethylcarbamoyl]butyric acid
- 2-amino-4-[2-mercapto-1-(4-mercaptobutylcarbamoyl)-ethylcarbamoyl]butyric acid
- L-.gamma.-glutamyl-L-cysteinyl-L-cysteine
- 2-[2-(4-amino-4-carboxybutyrylamino)-3-mercapto-propionylamino]-4-mercaptobutyric acid
- 2-amino-4-[1-(1-carbamoyl-2-mercaptoethylcarbamoyl)-2-mercaptoethylcarbamoyl]butyric acid
- 2-amino-4-[1-(1-dimethylcarbamoyl-2-mercaptoethylcarbamoyl)-2-mercaptoethylcarbamoyl]butyric acid
- 2-amino-4-[2-mercapto-l-(2-mercapto-1,1-dimethylethylcarbamoyl)ethylcarbamoyl]butyric acid
- 2-amino-4-[2-mercapto-1-(2-mercapto-1-ethylethylcarbamoyl)ethylcarbamoyl]butyric acid
- 2-(2-aminoacetylamino)-3-mercapto-N-mercaptomethylpropionamide
- 2-(2-aminoacetylamino)-3-mercapto-N-(2-mercaptoethyl)propionamide
- 2-(2-aminoacetylamino)-3-mercapto-N-(3-mercaptopropyl)propionamide
- 2-(2-aminoacetylamino)-3-mercapto-N-(4-mercaptobutyl)propionamide
- 2-(2-aminoacetylamino)-3-mercapto-N-(5-mercaptopentyl)propionamide
- glycyl-L-cysteinyl-L-cysteine
- 2-[2-(2-aminoacetylamino)-3-mercaptopropionylamino]-4-mercaptobutyric acid
- 2-(2-aminoacetylamino)-3-mercapto-N-(2-mercapto-1,1-dimethylethyl)propionamide
- 2-(2-aminoacetylamino)-3-mercapto-N-(1-mercaptomethylpropyl)propionamide
- 4-carboxy-L-.alpha.-glutamyl-L-cysteinyl-L-cysteine
- (S)-N-[N-(5-amino-5-carboxy-1-oxopentyl)-L-homocysteinyl]-L-cysteine
- 4-mercapto-N-mercaptomethylbutyramide
- 4-mercapto-N-(2-mercaptoethyl)butyramide
- 4-mercapto-N-(3-mercaptopropyl)butyramide
- N-(4-mercapto-1-oxobutyl)-L-cysteine
- 4-mercapto-2-(3-mercaptopropionylamino)butyramide
- N-(1-dimethylcarbamoyl-2-mercaptoethyl)-4-mercaptobutyramide
- N-(4-mercapto-1-oxobutyl)-L-homocysteine
- 4-mercapto-N-(2-mercapto-1,1-dimethylethyl)butyramide
- 4-mercapto-N-(1-mercaptomethylpropyl)butyramide
- 5-mercaptopentanoic acid mercaptomethylamide
- 5-mercaptopentanoic acid (2-mercaptoethyl)amide
- N-(5-mercapto-1-oxopentyl)-L-cysteine
- 5-mercapto-2-(3-mercaptopropionylamino)pentanamide
- N-(1-dimethylcarbamoyl-2-mercaptoethyl)-5-mercaptopentanamide
- 5-mercapto-N-(2-mercapto-1,1-dimethylethyl)pentanamide
- 5-mercapto-N-(1-mercaptomethylpropyl)pentanamide
- 6-mercaptohexanoic acid mercaptomethylamide

3. Use according to Claim 2, **characterized in that** the dimercaptoamide of formula (I) is chosen from the following compounds:
- 2-mercapto-N-(mercaptomethyl)acetamide
- 2-mercapto-N-(2-mercaptoethyl)acetamide
- 2-mercapto-N-(3-mercaptopropyl)acetamide
- 2-mercapto-N-(4-mercaptobutyl)acetamide
- 2-mercapto-N-(5-mercaptopentyl)acetamide
- 3-mercapto-2-(2-mercaptoacetylamino)propionamide
- 3-mercapto-2-(2-mercaptoacetylamino)-N,N-dimethylpropionamide
- 2-mercapto-N-(2-mercapto-1,1-dimethylethyl)acetamide
- 2-mercapto-N-(1-mercaptomethylpropyl)acetamide
- N-(mercaptoacetyl)-L-cysteine
- N-(mercaptoacetyl)-L-homocysteine
- 2-mercapto-N-mercaptomethylpropionamide
- 2-mercapto-N-(2-mercaptoethyl)propionamide
- 2-mercapto-N-(3-mercaptopropyl)propionamide
- 2-mercapto-N-(4-mercaptobutyl)propionamide
- 2-mercapto-N-(5-mercaptopentyl)propionamide
- mercapto-[(2-mercapto-1-oxopropyl)amino]acetic acid
- 2-mercaptopropionyl-L-cysteine
- N-(1-carbamoyl-2-mercaptoethyl)-2-mercaptopropionamide
- N-(1-dimethylcarbamoyl-2-mercaptoethyl)-2-mercaptopropionamide
- 4-mercapto-2-(2-mercaptopropionylamino)butyric acid
- 2-mercapto-N-(2-mercapto-1,1-dimethylethyl)propionamide
- 2-mercaptobutanoic acid mercaptomethylamide
- 2-mercaptobutanoic acid (2-mercaptoethyl)amide
- 2-mercaptobutanoic acid (3-mercaptopropyl)amide
- 2-mercaptobutanoic acid (4-mercaptobutyl)amide
- 2-mercaptopentanoic acid mercaptomethylamide
- 2-mercaptopentanoic acid (2-mercaptoethyl)amide
- 2-mercaptopentanoic acid (3-mercaptopropyl)amide
- 3-mercapto-N-(2-mercaptoethyl)propanamide
- 3-mercapto-N-mercaptomethylpropionamide
- 3-mercapto-N-(3-mercaptopropyl)propionamide
- 3-mercapto-N-(4-mercaptobutyl)propionamide
- N-(3-mercapto-1-oxopropyl)-L-cysteine
- 3-mercapto-2-(3-mercaptopropionylamino)propionamide
- N-(1-dimethylcarbamoyl-2-mercaptoethyl)-3-mercaptopropionamide
- 4-mercapto-2-(3-mercaptopropionylamino)butyric acid
- 3-mercapto-N-(2-mercapto-1,1-dimethylethyl)propionamide
- 3-mercapto-N-mercaptomethyl-2-methylpropionamide
- 3-mercapto-N-(2-mercaptoethyl)-2-methylpropionamide
- 3-mercapto-N-(3-mercaptopropyl)-2-methylpropionamide
- 3-mercapto-N-(4-mercaptobutyl)-2-methylpropionamide
- 3-mercapto-2-(3-mercapto-2-methylpropionylamino)-propionic acid
- N-(1-carbamoyl-2-mercaptoethyl)-3-mercapto-2-methylpropionamide
- N-(1-dimethylcarbamoyl-2-mercaptoethyl)-3-mercapto-2-methylpropionamide
- 3-mercapto-N-(2-mercapto-1,1-dimethylethyl)-2-methylpropionamide
- N-(3-mercapto-2-methyl-1-oxopropyl)-L-homocysteine
- N-mercaptomethyl-3-mercaptomethylsuccinamic acid
- N-(2-mercaptoethyl)-3-mercaptomethylsuccinamic acid
- N-(3-mercaptopropyl)-3-mercaptomethylsuccinamic acid
- N-(4-mercaptobutyl)-3-mercaptomethylsuccinamic acid
- N-(1-carboxy-2-mercaptoethyl)-3-mercaptomethylsuccinamic acid
- 2-mercapto-N-mercaptomethyl-2-methylpropionamide
- 2-mercapto-N-(2-mercaptoethyl)-2-methylpropanamide
- 2-mercapto-N-(3-mercaptopropyl)-2-methylpropionamide
- 2-mercapto-N-(4-mercaptobutyl)-2-methylpropionamide
- 2-mercapto-N-(2-mercapto-1,1-dimethylethyl)-2-methylpropionamide
- 2-mercapto-N-(2-mercapto-1-ethyl)-2-methylpropionamide
- N-(2-mercapto-2-methyl-1-oxopropyl)-L-cysteine
- N-(2-ethyl-2-mercapto-1-oxobutyl)-L-cysteine
- 3-mercapto-N-mercaptomethyl-3-methylbutyramide
- 3-mercapto-N-(2-mercaptoethyl)-3-methylbutanamide
- 3-mercapto-N-(3-mercaptopropyl)-3-methylbutyramide
- N-(3-mercapto-3-methyl-1-oxobutyl)-L-cysteine
- N-(1-carbamoyl-2-mercaptoethyl)-3-mercapto-3-methylbutyramide
- N-(1-dimethylcarbamoyl-2-mercaptoethyl)-3-mercapto-3-methylbutyramide
- 4-mercapto-2-(3-mercapto-3-methylbutyrylamino)butyric acid
- 3-mercapto-N-mercaptomethyl-2,2-dimethylpropionamide
- 3-mercapto-N-(2-mercaptoethyl)-2,2-dimethylpropionamide
- 3-mercapto-N-(3-mercaptopropyl)-2,2-dimethylpropionamide
- 3-mercapto-N-(4-mercaptobutyl)-2,2-dimethylpropionamide
- N-(3-mercapto-2,2-dimethyl-1-oxopropyl)-L-cysteine
- N-(1-carbamoyl-2-mercaptoethyl)-3-mercapto-2,2-dimethylpropionamide
- N-(1-dimethylcarbamoyl-2-mercaptoethyl)-3-mercapto-2,2-dimethylpropionamide
- N-[2-ethyl-2-(mercaptomethyl)-1-oxobutyl]-L-cysteine
- 2-amino-3-mercapto-N-mercaptomethylpropionamide
- (2R)-2-amino-3-mercapto-N-(2-mercaptoethyl)propanamide
- (2S)-2-amino-3-mercapto-N-(2-mercaptoethyl)propanamide
- 2-amino-3-mercapto-N-(3-mercaptopropyl)propionamide
- 2-amino-3-mercapto-N-(4-mercaptobutyl)propionamide
- L-cysteinyl-L-cysteine
- 2-amino-N-(1-carbamoyl-2-mercaptoethyl)-3-mercaptopropionamide
- 2-amino-N-(1-carbamoyl-2-mercaptoethyl)-3-mercapto-N,N-dimethylpropionamide
- 2-(2-amino-3-mercaptopropionylamino)-4-mercaptobutyric acid
- 2-amino-3-mercapto-N-(2-mercapto-1,1-dimethylethyl)-propionamide
- 2-amino-3-mercapto-N-(2-mercapto-1-ethyl)propionamide
- 2-acetylamino-3-mercapto-N-mercaptomethylpropionamide
- N-acetyl-L-cysteinyl-L-cysteine
- 2-(carboxymethylamino)-3-mercapto-N-mercaptomethylpropionamide
- 2-(carboxymethylamino)-3-mercapto-N-(2-mercaptoethyl)propionamide
- 2-(carboxymethylamino)-3-mercapto-N-(3-mercaptopropyl)propionamide
- L-.gamma.-glutamyl-L-cysteinyl-L-cysteine
- 2-(2-aminoacetylamino)-3-mercapto-N-mercaptomethylpropionamide
- 2-(2-aminoacetylamino)-3-mercapto-N-(2-mercaptoethyl)propionamide
- glycyl-L-cysteinyl-L-cysteine
- 4-carboxy-L-.alpha.-glutamyl-L-cysteinyl-L-cysteine
- 4-mercapto-N-mercaptomethylbutyramide
- 4-mercapto-N-(2-mercaptoethyl)butyramide
- 4-mercapto-N-(3-mercaptopropyl)butyramide
- N-(4-mercapto-1-oxobutyl)-L-cysteine
- 4-mercapto-2-(3-mercaptopropionylamino)butyramide
- N-(1-dimethylcarbamoyl-2-mercaptoethyl)-4-mercaptobutyramide
- 4-mercapto-N-(2-mercapto-1,1-dimethylethyl)butyramide
- 4-mercapto-N-(1-mercaptomethylpropyl)butyramide
- 5-mercaptopentanoic acid mercaptomethylamide
- 5-mercaptopentanoic acid (2-mercaptoethyl)amide
- N-(5-mercapto-1-oxopentyl)-L-cysteine
- 5-mercapto-2-(3-mercaptopropionylamino)pentanamide
- N-(1-dimethylcarbamoyl-2-mercaptoethyl)-5-mercaptopentanamide
- 5-mercapto-N-(2-mercapto-1,1-dimethylethyl)pentanamide
- 5-mercapto-N-(1-mercaptomethylpropyl)pentanamide
- 6-mercaptohexanoic acid mercaptomethylamide.

4. Process for permanently reshaping fibres, and in particular human keratin fibres such as the hair, comprising the application, to the fibres, of a reducing composition, and then of an oxidizing composition, **characterized in that** the reducing composition comprises, as a reducing agent, at least one dimercaptoamide of general formula (I) below:
HS-A-CO-NH-B-SH (I)
in which:
A represents a radical (CH₂)ₙ, n being an integer between 1 and 5, optionally substituted with:
(i) a linear or branched C₁-C₅ alkyl radical; phenyl; benzyl; amino; acetylamino; NH-CO-CH₂-NH₂; NH-CO-CH₂-CH₂-CH(NH₂)COOH; NH-CO-CH₂-CH₂-CH(COOH)COOH; CH₂-COOH; CH₂-COOCH₃; or CH₂-COOCH₂-CH₃, or
(ii) two methyl radicals or two ethyl radicals,
and B represents a radical (CH₂)p, p being an integer between 1 and 5, optionally substituted with:
(i) a linear or branched C₁-C₅ alkyl radical; carboxyl; COOCH₃; COOEt; CONH₂; CONH-CH₃; CON(CH₃)₂; CONH-CH₂-CH₃; CON (CH₂-CH₃)₂; CONH-CH₂-CHOH-CH₃; CO-NH-CH(COOH)-(CH₂)₄-NH₂; CO-NH-CH(COOH)-(CH₂)₄-N(CH₃)₂; CO-NH-CH₂-CH₂-COOEt ; CO-NH-CH(COOH)-iPr; CO-NH-CH₂-R with R = CO-NH₂ or (CH₂)₃-NH₂ or (CH₂)₄-NH₂ or (CH₂)₅-NH₂ or (CH₂)₄-OH, or
(ii) 2 methyl radicals,
the sum of n+p being between at least 2 and at most 6, and their organic and inorganic salts.

5. Process according to Claim 4, **characterized in that** the dimercaptoamide of formula (I) is present at a concentration of between 0.05 and 35%, and preferably of between 1 and 20% by weight of the total weight of the reducing composition.

6. Process according to Claim 4 or 5, **characterized in that** the pH of the reducing composition is between 4 and 11, and preferably between 6 and 10.

7. Process according to any one of Claims 4 to 6, **characterized in that** the reducing composition also comprises one or more reducing agents chosen from thioglycolic acid, thiolactic acid, their ester and amide derivatives, cysteamine and its C₁-C₄ acylated derivatives, cysteine, N-acetylcysteine, thiomalic acid, pantethein, 2,3-dimercaptosuccinic acid, alkali metal or alkaline-earth metal sulphites or bisulphites, N-(mercaptoalkyl)-ω-hydroxyalkylamides, N-mono- or N,N-dialkylmercapto-4-butyramides, aminomercaptoalkylamides, N-(mercaptoalkyl)succinamic acid and N-(mercaptoalkyl)succinimide derivatives, alkylaminomercaptoalkylamides, the azeotropic mixture of 2-hydroxypropyl thioglycolate and of (2-hydroxy-1-methyl)ethyl thioglycolate, mercapto alkylaminoamides, N-mercaptoalkylalkanediamides, and formamidinesulphinic acid derivatives.

8. Process according to any one of Claims 4 to 7, **characterized in that** the reducing composition also comprises at least one nonionic, anionic, cationic or amphoteric surfactant, or a mixture.

9. Process according to Claim 8, **characterized in that** the surfactant is chosen from alkyl sulphates, alkylbenzene sulphates, alkyl ether sulphates, alkylsulphonates, quaternary ammonium salts, alkylbetaines, oxyethylenated alkylphenols, fatty acid alkanolamides, oxyethylenated fatty acid esters, and hydroxypropyl ethers.

10. Process according to any one of Claims 4 to 9, **characterized in that** the reducing composition also comprises a compound chosen from linear or cyclic, volatile or nonvolatile silicones and mixtures thereof, polydimethylsiloxanes, quaternized polyorganosiloxanes, polyorganosiloxanes containing aminoalkyl groups modified with alkoxycarbonylalkyl groups, polydimethylsiloxanes containing stearoxydimethicone end groups, polydimethylsiloxane-dialkylammonium acetate copolymers, polydimethylsiloxane-polyalkylbetaine copolymers, polysiloxanes organomodified with mercapto or mercaptoalkyl groups, and silanes.

11. Process according to any one of Claims 4 to 9, **characterized in that** the reducing composition also comprises a compound chosen from cationic polymers of the ionene type, basic amino acids, glutamic acid, aspartic acid, peptides and derivatives thereof, protein hydrolysates, waxes, swelling agents and penetrating agents, dimethylisosorbitol, urea and its derivatives, pyrrolidone, N-alkylpyrrolidones, thiamorpholinone, alkylene glycol alkyl ethers or dialkylene glycol alkyl ethers, C₃-C₆ alkane diols, 2-imidazolidinone, fatty alcohols, lanolin derivatives, pantothenic acid, agents for preventing hair loss, antidandruff agents, thickeners, suspending agents, sequestering agents, opacifiers, colorants, sunscreens, fragrances and preserving agents, thickeners and gelling agents.

12. Process according to any one of Claims 4 to 11, **characterized in that** the oxidizing composition comprises, as an oxidizing agent, aqueous hydrogen peroxide, an alkali metal bromate, a persalt, a polythionate, or a mixture of alkali metal bromate and of persalt.

13. Reducing composition, **characterized in that** it comprises, as a reducing agent, at least one dimercaptoamide of general formula (I) below:
HS-A-CO-NH-B-SH (I)
in which:
A represents a radical (CH₂)ₙ, n being an integer between 1 and 5, optionally substituted with:
(i) a linear or branched C₁-C₅ alkyl radical; phenyl; benzyl; amino; acetylamino; NH-CO-CH₂-NH₂; NH-CO-CH₂-CH₂-CH(NH₂)COOH; NH-CO-CH₂-CH₂-CH(COOH)COOH; CH₂-COOH; CH₂-COOCH₃; or CH₂-COOCH₂-CH₃, or
(ii) two methyl radicals or two ethyl radicals,
and B represents a radical (CH₂)ₚ, p being an integer between 1 and 5, optionally substituted with:
(i) a linear or branched C₁-C₅ alkyl radical; carboxyl; COOCH₃; COOEt; CONH₂; CONH-CH₃; CON(CH₃)₂; CONH-CH₂-CH₃; CON(CH₂-CH₃)₂; CONH-CH₂-CHOH-CH₃; CO-NH-CH(COOH)-(CH₂)₄-NH₂; CO-NH-CH(COOH)-(CH₂)₄-N(CH₃)₂; CO-NH-CH₂-CH₂-COOEt; CO-NH-CH(COOH)-iPr; CO-NH-CH₂-R with R = CO-NH₂ or (CH₂)₃-NH₂ or (CH₂)₄-NH₂ or (CH₂)₅-NH₂ or (CH₂)₄-OH, or
(ii) 2 methyl radicals,
the sum of n+p being between 2 and 6,
and their organic and inorganic salts.

14. Composition according to Claim 13, **characterized in that** the dimercaptoamide of formula (I) is chosen from the compounds mentioned in Claim 2 or 3.

15. Composition according to Claim 13 or 14, **characterized in that** the dimercaptoamide of formula (I) is present at a concentration of between 0.05 and 35%, and preferably of between 1 and 20% by weight of the total weight of the reducing composition.

16. Composition according to one of Claims 13 to 15, **characterized in that** the pH of the composition is between 4 and 11, and preferably between 6 and 10.

17. Composition according to one of Claims 13 to 16, **characterized in that** it also comprises one or more reducing agents chosen from thioglycolic acid, thiolactic acid, their ester and amide derivatives, cysteamine and its C₁-C₄ acylated derivatives, cysteine, N-acetylcysteine, thiomalic acid, pantethein, 2,3-dimercaptosuccinic acid, alkali metal or alkaline-earth metal sulphites or bisulphites, N-(mercaptoalkyl)-ω-hydroxyalkylamides, N-mono- or N,N-dialkylmercapto-4-butyramides, aminomercaptoalkylamides, N-(mercaptoalkyl)succinamic acid and N-(mercaptoalkyl)succinimide derivatives, alkylaminomercaptoalkylamides, the azeotropic mixture of 2-hydroxypropyl thioglycolate and of (2-hydroxy-1-methyl)ethyl thioglycolate, mercapto alkylaminoamides, N-mercaptoalkylalkanediamides, and formamidinesulphinic acid derivatives.

18. Composition according to one of Claims 13 to 17, **characterized in that** it also comprises at least one nonionic, anionic, cationic or amphoteric surfactant, or a mixture.

19. Composition according to Claim 18, **characterized in that** the surfactant is chosen from alkyl sulphates, alkylbenzene sulphates, alkyl ether sulphates, alkylsulphonates, quaternary ammonium salts, alkylbetaines, oxyethylenated alkylphenols, fatty acid alkanolamides, oxyethylenated fatty acid esters, and hydroxypropyl ethers.

20. Composition according to one of Claims 13 to 19, **characterized in that** it also comprises a compound chosen from linear or cyclic, volatile or nonvolatile silicones and mixtures thereof, polydimethylsiloxanes, quaternized polyorganosiloxanes, polyorganosiloxanes containing aminoalkyl groups modified with alkoxycarbonylalkyl groups, polydimethylsiloxanes containing stearoxydimethicone end groups, polydimethylsiloxane-dialkylammonium acetate copolymers, polydimethylsiloxane-polyalkylbetaine copolymers, polysiloxanes organomodified with mercapto or mercaptoalkyl groups, and silanes.

21. Composition according to one of Claims 13 to 20, **characterized in that** it also comprises a compound chosen from cationic polymers of the ionene type, basic amino acids, glutamic acid, aspartic acid, peptides and derivatives thereof, protein hydrolysates, waxes, swelling agents and penetrating agents, dimethylisosorbitol, urea and its derivatives, pyrrolidone, N-alkylpyrrolidones, thiamorpholinone, alkylene glycol alkyl ethers or dialkylene glycol alkyl ethers, C₃-C₆ alkane diols, 2-imidazolidinone, fatty alcohols, lanolin derivatives, pantothenic acid, agents for preventing hair loss, antidandruff agents, thickeners, suspending agents, sequestering agents, opacifiers, colorants, sunscreens, fragrances and preserving agents.

22. Kit for permanently reshaping the hair, comprising, in a first compartment, as a reducing composition, a composition according to one of Claims 13 to 21 and, in a second compartment, an oxidizing composition.

23. Dimercaptoamide corresponding to general formula (II) below:
HS-A-CO-NH-B-SH (II)
in which:
(i) A represents a radical CH₂ and B represents a radical (CH₂)ₓ in which x = 2, 3, 4 or 5; a radical C (CH₃)₂-CH₂; a radical CH(Et)-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(ii) A represents a radical CH(CH₃) and B represents a radical (CH₂)ₓ in which x = 1, 2, 3, 4 or 5; a radical C(CH₃)₂-CH₂; a radical CH(Et)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(iii) A represents a radical CH-R3 in which R3 denotes a linear or branched C₂-C₅ alkyl radical and B represents a radical (CH₂)ₓ x in which x = 1, 2, 3, 4 or 5; a radical C(CH₃)₂-CH₂; a radical CH(Et)-CH₂; a radical CH(COOH)-CH₂; a radical CH(COOH)-CH₂-CH₂; or a radical CH(CO-NR1R2)-CH₂ or CH(CO-NRlR2)-CH₂-CH₂ in which R1 and R2 represent, independently of one another, a hydogen atom or a methyl, ethyl or 2-hydroxypropyl radical,
(iv) A represents a radical CH₂-CH₂ or C(CH₃)₂-CH₂ and B represents a radical (CH₂)ₓ in which x = 1, 3 or 4; a radical C (CH₃)₂-CH₂; a radical CH(Et)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(v) A represents a radical CH₂-CH(CH₃) and B represents a radical (CH₂)ₓ in which x = 1, 2, 3 or 4; a radical C(CH₃)₂-CH₂; a radical CH(Et)-CH₂; a radical CH(COOH)-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(vi) A represents a radical CH₂-CH-R4 in which R4 denotes a phenyl radical or a radical COO-CH₃ or COO-CH₂-CH₃ and B represents a radical (CH₂)ₓ in which x = 1, 2, 3 or 4; a radical C (CH₃) ₂-CH₂; a radical CH(Et)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(vii) A represents a radical CH₂-CH(CH₂-COOH) or CH₂-CH(NH-CH₂-COOH) and B represents a radical (CH₂)ₓ in which x = 1, 2, 3 or 4; a radical C(CH₃)₂-CH₂; a radical CH(Et)-CH₂; a radical CH(COOH)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(viii) A represents a radical C(CH₃)₂ and B represents a radical (CH₂)ₓ in which x = 1, 3, 4 or 5; a radical C (CH₃) ₂-CH₂; a radical CH(Et)-CH₂; a radical CH(CO-NHCH₃)-CH₂; a radical CH(CO-NHEt)-CH₂; a radical CH(CO-NH-CH₂-CHOH-CH₃)-CH₂; or CH(CO-NR1R2)-CH₂-CH₂ in which R1 and R2 represent, independently of one another, a hydrogen atom or a methyl, ethyl or 2-hydroxypropyl radical,
(ix) A represents a radical C(Et)₂ et B represents a radical (CH₂)ₓ in which x = 1, 2, 3, 4 or 5; a radical C (CH₃) ₂-CH₂; a radical CH(Et)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(x) A represents a radical CH₂-C(CH₃)₂ or CH(CH₂-Ph) and B represents a radical (CH₂)ₓ in which x = 1, 2, 3 or 4; a radical C(CH₃)₂-CH₂; a radical CH(Et)-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(xi) A represents a radical CH₂-C(Et)₂ and B represents a radical (CH₂)ₓ in which x = 1, 2, 3 or 4; a radical C(CH₃)₂-CH₂; a radical CH(Et)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(xii) A represents a radical CH₂-CH(NH₂) and B represents a radical (CH₂)ₓ in which x = 1, 3 or 4; a radical C (CH₃) ₂-CH₂; a radical CH(Et)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(xiii) A represents a radical CH₂-CH(NHAc) and B represents a radical (CH₂)ₓ in which x = 1, 3 or 4; a radical C(CH₃)₂-CH₂; a radical CH(Et)-CH₂; a radical CH(COOH)-CH₂-CH₂; a radical CH(CO-NH-CH₃)-CH₂; a radical CH(CO-NH-Et)-CH₂; a radical CH(CO-N(CH₃)₂)-CH₂; or a radical CH(CO-NR1R2)-CH₂-CH₂ in which R1 and R2 represent, independently of one another, a hydrogen atom or a methyl, ethyl or 2-hydroxypropyl radical,
(xiv) A represents a radical CH₂-CH(NH-CO-CH₂-CH₂-CH(NH₂)COOH) or CH₂-CH(NH-CO-CH₂-NH₂) or CH₂-CH(NH-CO-CH(NH₂)-CH₂-CH(COOH)₂ or CH₂-CH(NH-CO-CH₂-CH₂-CH₂-CH(NH₂)COOH) and B represents a radical (CH₂) x in which x = 1, 2, 3 or 4; a radical C(CH₃)₂-CH₂; a radical CH(Et)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(xv) A represents a radical (CH₂)₃ and B represents a radical (CH₂)ₓ in which x = 1, 2 or 3; a radical C(CH₃)₂-CH₂; a radical CH(Et)-CH₂; a radical CH(COOH)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(xvi) A represents a radical (CH₂)₄ and B represents a radical (CH₂)ₓ in which x = 1 or 2; a radical C(CH₃)₂-CH₂; a radical CH(Et)-CH₂; a radical CH(COOH)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical,
(xvii) A represents a radical (CH₂)₅ and B represents a radical CH₂,
(xviii) A represents a radical CH-Ph and B represents a radical (CH₂)ₓ x in which x = 1, 2, 3, 4 or 5; a radical C (CH₃) ₂-CH₂; a radical CH(Et)-CH₂; a radical CH(COOH)-CH₂; a radical CH(COOH)-CH₂-CH₂; or a radical CH(CO-NR1R2)-CH₂ or CH(CO-NR1R2)-CH₂-CH₂ in which R1 and R2 represent, independently of one another, a hydrogen atom or a methyl, ethyl or 2-hydroxypropyl radical, and the organic and inorganic salts of said compounds of formula (II).

24. Dimercaptoamide according to Claim 23, **characterized in that** it is chosen from the following compounds:
- 2-mercapto-N-(2-mercaptoethyl)acetamide
- 2-mercapto-N-(3-mercaptopropyl)acetamide
- 2-mercapto-N-(4-mercaptobutyl)acetamide
- 2-mercapto-N-(5-mercaptopentyl)acetamide
- 3-mercapto-2-(2-mercaptoacetylamino)propionamide
- 3-mercapto-2-(2-mercaptoacetylamino)-N,N-dimethylpropionamide
- 2-mercapto-N-(2-mercapto-1,1-dimethylethyl)acetamide
- 2-mercapto-N-(1-mercaptomethylpropyl)acetamide
- 2-mercapto-N-mercaptomethylpropionamide
- 2-mercapto-N-(2-mercaptoethyl)propionamide
- 2-mercapto-N-(3-mercaptopropyl)propionamide
- 2-mercapto-N-(4-mercaptobutyl)propionamide
- 2-mercapto-N-(5-mercaptopentyl)propionamide
- N-(1-carbamoyl-2-mercaptoethyl)-2-mercaptopropionamide
- N-(1-dimethylcarbamoyl-2-mercaptoethyl)-2-mercaptopropionamide
- 4-mercapto-2-(2-mercaptopropionylamino)butyric acid
- 2-mercapto-N-(2-mercapto-1,1-dimethylethyl)propionamide
- 2-mercaptobutanoic acid mercaptomethylamide
- 2-mercaptobutanoic acid (2-mercaptoethyl)amide
- 2-mercaptobutanoic acid (3-mercaptopropyl)amide
- 2-mercaptobutanoic acid (4-mercaptobutyl)amide
- 2-mercaptobutanoic acid (5-mercaptopentyl)amide
- 2-mercaptobutanoic acid (1-carbamoyl-2-mercaptoethyl)amide
- N-(1-dimethylcarbamoyl-2-mercaptoethyl)-2-mercaptobutyramide
- 2-mercaptobutanoic acid (1-carbamoyl-3-mercaptopropyl)amide
- 2-mercaptobutanoic acid (2-mercapto-1,1-dimethylethyl)amide
- 2-mercaptopentanoic acid mercaptomethylamide
- 2-mercaptopentanoic acid (2-mercaptoethyl)amide
- 2-mercaptopentanoic acid (3-mercaptopropyl)amide
- 2-mercaptopentanoic acid (4-mercaptobutyl)amide
- 2-mercaptopentanoic acid (5-mercaptopentyl)amide
- 3-mercapto-2-(2-mercaptopentanoylamino)propionic acid
- 2-mercaptopentanoic acid (1-carbamoyl-2-mercaptoethyl)amide
- 2-mercaptopentanoic acid (1-dimethylcarbamoyl-2-mercaptoethyl)amide
- 2-mercaptopentanoic acid (1-carbamoyl-3-mercaptopropyl)amide
- 2-mercaptopentanoic acid (2-mercapto-1,1-dimethylethyl)amide
- 3-mercapto-N-mercaptomethylpropionamide
- 3-mercapto-N-(3-mercaptopropyl)propionamide
- 3-mercapto-N-(4-mercaptobutyl)propionamide
- 3-mercapto-2-(3-mercaptopropionylamino)propionamide
- N-(1-dimethylcarbamoyl-2-mercaptoethyl)-3-mercaptopropionamide
- 4-mercapto-2-(3-mercaptopropionylamino)butyric acid
- 3-mercapto-N-(2-mercapto-1,1-dimethylethyl)propionamide
- 3-mercapto-N-mercaptomethyl-2-methylpropionamide
- 3-mercapto-N-(2-mercaptoethyl)-2-methylpropionamide
- 3-mercapto-N-(3-mercaptopropyl)-2-methylpropionamide
- 3-mercapto-N-(4-mercaptobutyl)-2-methylpropionamide
- 3-mercapto-2-(3-mercapto-2-methylpropionylamino)-propionic acid
- N-(1-carbamoyl-2-mercaptoethyl)-3-mercapto-2-methylpropionamide
- N-(1-dimethylcarbamoyl-2-mercaptoethyl)-3-mercapto-2-methylpropionamide
- 3-mercapto-N-(2-mercapto-1,1-dimethylethyl)-2-methylpropionamide
- N-mercaptomethyl-3-mercaptomethylsuccinamic acid
- N-(2-mercaptoethyl)-3-mercaptomethylsuccinamic acid
- N-(3-mercaptopropyl)-3-mercaptomethylsuccinamic acid
- N-(4-mercaptobutyl)-3-mercaptomethylsuccinamic acid
- N-(1-carboxy-2-mercaptoethyl)-3-mercaptomethylsuccinamic acid
- N-(1-carboxy-3-mercaptopropyl)-3-mercaptomethylsuccinamic acid
- N-(2-mercapto-1,1-dimethylethyl)-3-mercaptomethylsuccinamic acid
- N-(2-mercapto-1-ethyl)-3-mercaptomethylsuccinamic acid
- 2-mercapto-N-mercaptomethyl-2-methylpropionamide
- 2-mercapto-N-(3-mercaptopropyl)-2-methylpropionamide
- 2-mercapto-N-(4-mercaptobutyl)-2-methylpropionamide
- 2-mercapto-N-(5-mercaptopentyl)-2-methylpropionamide
- 2-mercapto-N-(2-mercapto-1,1-dimethylethyl)-2-methylpropionamide
- 2-mercapto-N-(2-mercapto-1-ethyl)-2-methylpropionamide
- 3-mercapto-N-mercaptomethyl-3-methylbutyramide
- 3-mercapto-N-(3-mercaptopropyl)-3-methylbutyramide
- 3-mercapto-N-(4-mercaptobutyl)-3-methylbutyramide
- N-(1-carbamoyl-2-mercaptoethyl)-3-mercapto-3-methylbutyramide
- N-(1-dimethylcarbamoyl-2-mercaptoethyl)-3-mercapto-3-methylbutyramide
- 4-mercapto-2-(3-mercapto-3-methylbutyrylamino)butyric acid
- 3-mercapto-N-(2-mercapto-1,1-dimethylethyl)-3-methylbutyramide
- 3-mercapto-N-(2-mercapto-1-ethyl)-3-methylbutyramide
- 3-mercapto-N-mercaptomethyl-2,2-dimethylpropionamide
- 3-mercapto-N-(2-mercaptoethyl)-2,2-dimethylpropionamide
- 3-mercapto-N-(3-mercaptopropyl)-2,2-dimethylpropionamide
- 3-mercapto-N-(4-mercaptobutyl)-2,2-dimethylpropionamide
- 3-mercapto-N-(2-mercapto-1,1-dimethylethyl)-2,2-dimethylpropionamide
- N-(1-carbamoyl-2-mercaptoethyl)-3-mercapto-2,2-dimethylpropionamide
- N-(1-dimethylcarbamoyl-2-mercaptoethyl)-3-mercapto-2,2-dimethylpropionamide
- 2-amino-3-mercapto-N-mercaptomethylpropionamide
- 2-amino-3-mercapto-N-(3-mercaptopropyl)propionamide
- 2-amino-3-mercapto-N-(4-mercaptobutyl)propionamide
- 2-amino-N-(1-carbamoyl-2-mercaptoethyl)-3-mercaptopropionamide
- 2-amino-N-(1-carbamoyl-2-mercaptoethyl)-3-mercapto-N,N-dimethylpropionamide
- 2-(2-amino-3-mercaptopropionylamino)-4-mercaptobutyric acid
- 2-amino-3-mercapto-N-(2-mercapto-1,1-dimethylethyl)-propionamide
- 2-amino-3-mercapto-N-(2-mercapto-1-ethyl)propionamide
- 2-acetylamino-3-mercapto-N-mercaptomethylpropionamide
- 2-acetylamino-3-mercapto-N-(3-mercaptopropyl)propionamide
- 2-acetylamino-3-mercapto-N-(4-mercaptobutyl)propionamide
- 2-(2-acetylamino-3-mercaptopropionylamino)-3-mercapto-3-methylbutyric acid
- 2-acetylamino-3-mercapto-N-(1-mercaptomethylpropyl)-propionamide
- 2-acetylamino-N-(1-dimethylcarbamoyl-2-mercaptoethyl)-3-mercaptopropionamide
- 2-(2-acetylamino-3-mercaptopropionylamino)-4-mercaptobutyric acid
- 2-(carboxymethylamino)-3-mercapto-N-mercaptomethylpropionamide
- 2-(carboxymethylamino)-3-mercapto-N-(2-mercaptoethyl)propionamide
- 2-(carboxymethylamino)-3-mercapto-N-(3-mercaptopropyl)propionamide
- 2-(carboxymethylamino)-3-mercapto-N-(4-mercaptobutyl)propionamide
- 2-(carboxymethylamino)-N-(1-carbamoyl-2-mercaptoethyl)-3-mercaptopropionamide
- 2-[2-(carboxymethylamino)-3-mercaptopropionylamino]-3-mercaptopropionic acid
- [1-(1-carbamoyl-2-mercaptoethylcarbamoyl)-2-mercaptoethylamino]acetic acid
- 2-(carboxymethylamino)-3-mercapto-N-(2-mercapto-1,1-dimethylethyl)propionamide
- 2-amino-4-[2-mercapto-1-(mercaptomethylcarbamoyl)-ethylcarbamoyl]butyric acid
- 2-amino-4-[2-mercapto-1-(2-mercaptoethylcarbamoyl)-ethylcarbamoyl]butyric acid
- 2-amino-4-[2-mercapto-1-(3-mercaptopropylcarbamoyl)-ethylcarbamoyl]butyric acid
- 2-amino-4-[2-mercapto-1-(4-mercaptobutylcarbamoyl)-ethylcarbamoyl]butyric acid
- 2-[2-(4-amino-4-carboxybutyrylamino)-3-mercapto-propionylamino]-4-mercaptobutyric acid
- 2-amino-4-[1-(1-carbamoyl-2-mercaptoethylcarbamoyl)-2-mercaptoethylcarbamoyl]butyric acid
- 2-amino-4-[1-(1-dimethylcarbamoyl-2-mercaptoethylcarbamoyl)-2-mercaptoethylcarbamoyl]butyric acid
- 2-amino-4-[2-mercapto-1-(2-mercapto-1,1-dimethylethylcarbamoyl)ethylcarbamoyl]butyric acid
- 2-amino-4-[2-mercapto-1-(2-mercapto-1-ethylethylcarbamoyl)ethylcarbamoyl]butyric acid
- 2-(2-aminoacetylamino)-3-mercapto-N-mercaptomethylpropionamide
- 2-(2-aminoacetylamino)-3-mercapto-N-(2-mercaptoethyl)propionamide
- 2-(2-aminoacetylamino)-3-mercapto-N-(3-mercaptopropyl)propionamide
- 2-(2-aminoacetylamino)-3-mercapto-N-(4-mercaptobutyl)propionamide
- 2-(2-aminoacetylamino)-3-mercapto-N-(5-mercaptopentyl)propionamide
- 2-[2-(2-aminoacetylamino)-3-mercaptopropionylamino]-4-mercaptobutyric acid
- 2-(2-aminoacetylamino)-3-mercapto-N-(2-mercapto-1,1-dimethylethyl)propionamide
- 2-(2-aminoacetylamino)-3-mercapto-N-(1-mercaptomethylpropyl)propionamide
- 4-mercapto-N-mercaptomethylbutyramide
- 4-mercapto-N-(2-mercaptoethyl)butyramide
- 4-mercapto-N-(3-mercaptopropyl)butyramide
- N-(4-mercapto-1-oxobutyl)-L-cysteine
- 4-mercapto-2-(3-mercaptopropionylamino)butyramide
- N-(1-dimethylcarbamoyl-2-mercaptoethyl)-4-mercaptobutyramide
- N-(4-mercapto-1-oxobutyl)-L-homocysteine
- 4-mercapto-N-(2-mercapto-1,1-dimethylethyl)butyramide
- 4-mercapto-N-(l-mercaptomethylpropyl)butyramide
- 5-mercaptopentanoic acid mercaptomethylamide
- 5-mercaptopentanoic acid (2-mercaptoethyl)amide
- N-(5-mercapto-l-oxopentyl)-L-cysteine
- 5-mercapto-2-(3-mercaptopropionylamino)pentanamide
- N-(1-dimethylcarbamoyl-2-mercaptoethyl)-5-mercaptopentanamide
- 5-mercapto-N-(2-mercapto-1,1-dimethylethyl)pentanamide
- 5-mercapto-N-(1-mercaptomethylpropyl)pentanamide
- 6-mercaptohexanoic acid mercaptomethylamide

25. Dimercaptoamide according to Claim 23, **characterized in that** it is chosen from the following compounds:
- 2-mercapto-N-(2-mercaptoethyl)acetamide
- 2-mercapto-N-(3-mercaptopropyl)acetamide
- 2-mercapto-N-(4-mercaptobutyl)acetamide
- 2-mercapto-N-(5-mercaptopentyl)acetamide
- 3-mercapto-2-(2-mercaptoacetylamino)propionamide
- 3-mercapto-2-(2-mercaptoacetylamino)-N,N-dimethylpropionamide
- 2-mercapto-N-(2-mercapto-1,1-dimethylethyl)acetamide
- 2-mercapto-N-(1-mercaptomethylpropyl)acetamide
- 2-mercapto-N-mercaptomethylpropionamide
- 2-mercapto-N-(2-mercaptoethyl)propionamide
- 2-mercapto-N-(3-mercaptopropyl)propionamide
- 2-mercapto-N-(4-mercaptobutyl)propionamide
- 2-mercapto-N-(5-mercaptopentyl)propionamide
- N-(1-carbamoyl-2-mercaptoethyl)-2-mercaptopropionamide
- N-(1-dimethylcarbamoyl-2-mercaptoethyl)-2-mercaptopropionamide
- 4-mercapto-2-(2-mercaptopropionylamino)butyric acid
- 2-mercapto-N-(2-mercapto-1,1-dimethylethyl)propionamide
- 2-mercaptobutanoic acid mercaptomethylamide
- 2-mercaptobutanoic acid (2-mercaptoethyl)amide
- 2-mercaptobutanoic acid (3-mercaptopropyl)amide
- 2-mercaptobutanoic acid (4-mercaptobutyl)amide
- 2-mercaptopentanoic acid mercaptomethylamide
- 2-mercaptopentanoic acid (2-mercaptoethyl)amide
- 2-mercaptopentanoic acid (3-mercaptopropyl)amide
- 3-mercapto-N-mercaptomethylpropionamide
- 3-mercapto-N-(3-mercaptopropyl)propionamide
- 3-mercapto-2-(3-mercaptopropionylamino)propionamide
- N-(1-dimethylcarbamoyl-2-mercaptoethyl)-3-mercaptopropionamide
- 4-mercapto-2-(3-mercaptopropionylamino)butyric acid
- 3-mercapto-N-(2-mercapto-1,1-dimethylethyl)propionamide
- 3-mercapto-N-mercaptomethyl-2-methylpropionamide
- 3-mercapto-N-(2-mercaptoethyl)-2-methylpropionamide
- 3-mercapto-N-(3-mercaptopropyl)-2-methylpropionamide
- 3-mercapto-N-(4-mercaptobutyl)-2-methylpropionamide
- 3-mercapto-2-(3-mercapto-2-methylpropionylamino)-propionic acid
- N-(1-carbamoyl-2-mercaptoethyl)-3-mercapto-2-methylpropionamide
- N-(1-dimethylcarbamoyl-2-mercaptoethyl)-3-mercapto-2-methylpropionamide
- 3-mercapto-N-(2-mercapto-1,1-dimethylethyl)-2-methylpropionamide
- N-mercaptomethyl-3-mercaptomethylsuccinamic acid
- N-(2-mercaptoethyl)-3-mercaptomethylsuccinamic acid
- N-(3-mercaptopropyl)-3-mercaptomethylsuccinamic acid
- N-(4-mercaptobutyl)-3-mercaptomethylsuccinamic acid
- N-(1-carboxy-2-mercaptoethyl)-3-mercaptomethylsuccinamic acid
- 2-mercapto-N-mercaptomethyl-2-methylpropionamide
- 2-mercapto-N-(3-mercaptopropyl)-2-methylpropionamide
- 2-mercapto-N-(4-mercaptobutyl)-2-methylpropionamide
- 2-mercapto-N-(2-mercapto-1,1-dimethylethyl)-2-methylpropionamide
- 2-mercapto-N-(2-mercapto-1-ethyl)-2-methylpropionamide
- 3-mercapto-N-mercaptomethyl-3-methylbutyramide
- 3-mercapto-N-(3-mercaptopropyl)-3-methylbutyramide
- N-(1-carbamoyl-2-mercaptoethyl)-3-mercapto-3-methylbutyramide
- N-(1-dimethylcarbamoyl-2-mercaptoethyl)-3-mercapto-3-methylbutyramide
- 4-mercapto-2-(3-mercapto-3-methylbutyrylamino)butyric acid
- 3-mercapto-N-mercaptomethyl-2,2-dimethylpropionamide
- 3-mercapto-N-(2-mercaptoethyl)-2,2-dimethylpropionamide
- 3-mercapto-N-(3-mercaptopropyl)-2,2-dimethylpropionamide
- 3-mercapto-N-(4-mercaptobutyl)-2,2-dimethylpropionamide
- N-(1-carbamoyl-2-mercaptoethyl)-3-mercapto-2,2-dimethylpropionamide
- N-(1-dimethylcarbamoyl-2-mercaptoethyl)-3-mercapto-2,2-dimethylpropionamide
- 2-amino-3-mercapto-N-mercaptomethylpropionamide
- 2-amino-3-mercapto-N-(3-mercaptopropyl)propionamide
- 2-amino-3-mercapto-N-(4-mercaptobutyl)propionamide
- 2-amino-N-(1-carbamoyl-2-mercaptoethyl)-3-mercaptopropionamide
- 2-amino-N-(1-carbamoyl-2-mercaptoethyl)-3-mercapto-N,N-dimethylpropionamide
- 2-(2-amino-3-mercaptopropionylamino)-4-mercaptobutyric acid
- 2-amino-3-mercapto-N-(2-mercapto-1,1-dimethylethyl)-propionamide
- 2-amino-3-mercapto-N-(2-mercapto-1-ethyl)propionamide
- 2-acetylamino-3-mercapto-N-mercaptomethylpropionamide
- 2-(carboxymethylamino)-3-mercapto-N-mercaptomethylpropionamide
- 2-(carboxymethylamino)-3-mercapto-N-(2-mercaptoethyl)propionamide
- 2-(carboxymethylamino)-3-mercapto-N-(3-mercaptopropyl)propionamide
- 2-(2-aminoacetylamino)-3-mercapto-N-mercaptomethylpropionamide
- 2-(2-aminoacetylamino)-3-mercapto-N-(2-mercaptoethyl)propionamide
- 4-mercapto-N-mercaptomethylbutyramide
- 4-mercapto-N-(2-mercaptoethyl)butyramide
- 4-mercapto-N-(3-mercaptopropyl)butyramide
- N-(4-mercapto-1-oxobutyl)-L-cysteine
- 4-mercapto-2-(3-mercaptopropionylamino)butyramide
- N-(1-dimethylcarbamoyl-2-mercaptoethyl)-4-mercaptobutyramide
- 4-mercapto-N-(2-mercapto-1,1-dimethylethyl)butyramide
- 4-mercapto-N-(1-mercaptomethylpropyl)butyramide
- 5-mercaptopentanoic acid mercaptomethylamide
- 5-mercaptopentanoic acid (2-mercaptoethyl)amide
- N-(5-mercapto-1-oxopentyl)-L-cysteine
- 5-mercapto-2-(3-mercaptopropionylamino)pentanamide
- N-(1-dimethylcarbamoyl-2-mercaptoethyl)-5-mercaptopentanamide
- 5-mercapto-N-(2-mercapto-1,1-dimethylethyl)pentanamide
- 5-mercapto-N-(1-mercaptomethylpropyl)pentanamide
- 6-mercaptohexanoic acid mercaptomethylamide.

26. Reducing composition, **characterized in that** it comprises at least one dimercaptoamide according to one of Claims 23 to 25.

27. Process for preparing a dimercaptoamide of formula (II) according to Claim 23, **characterized in that** it comprises the reaction of a protected mercapto acid halide of formula P-S-A-CO-X with a protected aminothiol of formula H₂N-B-S-P, A and B having the same meaning as in Claim 23, P being a thiol function-protecting group, and X being chlorine or bromine, in an aprotic solvent, in the presence of an agent intended to capture the released hydrochloric or hydrobromic acid, such as for example a tertiary amine, so as to form a protected dimercaptoamide of formula P-S-A-CO-NH-B-S-P, and then the deprotection of the protected dimercaptoamide by hydrolysis or by reduction.

28. Process for preparing a dimercaptoamide of formula (II) according to Claim 23, **characterized in that** it comprises the reaction of a mercapto acid of formula HS-A-COOH with an aminothiol of formula H₂N-B-SH, A and B having the same meaning as in Claim 23, in the presence of a coupling and/or dehydrating agent, in an aprotic solvent.

29. Process for preparing a dimercaptoamide of formula (II) according to Claim 23, **characterized in that** it comprises the reaction of a mercapto ester of formula HS-A-COOR, R being a methyl or ethyl radical, with an aminothiol of formula H₂N-B-SH, A and B having the same meaning as in Claim 23, at a temperature of between 30°C and 180°C

30. Process for preparing a dimercaptoamide of formula (II) according to Claim 23, **characterized in that** it comprises:
a) the reaction of a halogenated acid halide of formula X-A-CO-C1 with a haloamine of formula H₂N-B-X, X representing a chlorine or bromine atom, A and B having the same meaning as in Claim 23, in an aprotic solvent and in the presence of an agent intended to capture the released hydrochloric or hydrobromic acid, so as to form a dihaloamide of formula X-A-CO-NH-B-X, and then
b) the conversion of the dihaloamide obtained to a dimercaptoamide of formula (II), either by direct reaction with hydrogen sulphide in an appropriate solvent, or by reaction with thioacetic acid, preferably in salified form, followed by acid or alkaline hydrolysis of the di(S-acetyl)mercaptoamide, or alternatively by reaction with thiourea followed by hydrolysis of the isothiouronium disalt.

31. Process for preparing a dimercaptoamide of formula (II) according to Claim 23, **characterized in that** it comprises the reaction of a thiolactone of formula 11 with an aminothiol of formula 5, in an aprotic solvent, according to the following reaction: 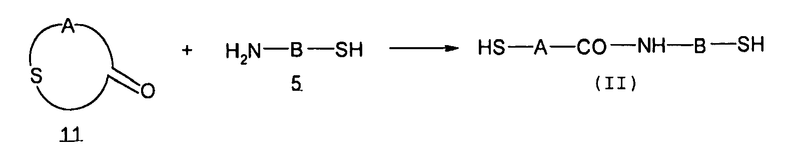 A and B having the same meaning as in Claim 23.

32. Process for preparing a dimercaptoamide of formula (II) according to Claim 23, **characterized in that** it comprises the reaction of a mercaptoamide of formula HS-A-CO-NH₂ with an aminothiol salt of formula HX, H₂N-B-SH, X representing a chlorine or bromine atom, A and B having the same meaning as in Claim 23, at a temperature of between 60 and 200°C.

## Patentansprüche

1. Verwendung eines Dimercaptoamids der folgenden allgemeinen Formel (I) als Reduktionsmittel für die dauerhafte Verformung von Keratinfasern und insbesondere menschlichen Keratinfasern wie Haaren:
HS-A-CO-NH-B-SH (I)
wobei in der Formel:
A bedeutet eine Gruppe (CH₂)ₙ, wobei n eine ganze Zahl im Bereich von 1 bis 5 ist, die gegebenenfalls substituiert ist mit:
(i) einer geradkettigen oder verzweigten C₁₋₅-Alkylgruppe; Phenyl; Benzyl; Amino; Acetylamino; NH-CO-CH₂-NH₂; NH-CO-CH₂-CH₂CH(NH₂)COOH; NH-CO-CH₂-CH₂-CH(COOH)COOH; CH₂-COOH; CH₂COOCH₃; oder CH₂-COOCH₂-CH₃, oder
(ii) zwei Methylgruppen oder zwei Ethylgruppen
und B bedeutet eine Gruppe (CH₂)_{P}, wobei p eine ganze Zahl im Bereich von 1 bis 5 ist, die gegebenenfalls substituiert ist mit:
(i) einer geradkettigen oder verzweigten C₁₋₅-Alkylgruppe; Carboxy; COOCH₃; COOEt; CONH₂; CONH-CH₃; CON(CH₃)₂; CONH-CH₂-CH₃; CON(CH₂-CH₃)₂; CONH-CH₂-CHOH-CH₃; CO-NH-CH(COOH)-(CH₂)₄-NH₂; CO-NH-CH(COOH)-(CH₂)₄-N(CH₃)₂; CO-NH-CH₂-CH₂-COOEt; CO-NH-CH(COOH)-iPr; CO-NH-CH₂-R mit R = CO-NH₂ oder (CH₂)₃-NH₂ oder (CH₂)₄-NH₂ oder (CH₂)₅-NH₂ oder (CH₂)₄-OH, oder
(ii) 2 Methylgruppen,
wobei die Summe n+p im Bereich von 2 bis 6 liegt,
und ihrer organischen und anorganischen Salze.

2. Verwendung nach Anspruch 1, **dadurch gekennzeichnet, dass** das Dimercaptoamid der Formel (I) unter den folgenden Verbindungen ausgewählt ist:
- 2-Mercapto-N-(mercaptomethyl)-acetamid
- 2-Mercapto-N-(2-mercaptoethyl)-acetamid
- 2-Mercapto-N-(3-mercaptopropyl)-acetamid
- 2-Mercapto-N-(4-mercaptobutyl)-acetamid
- 2-Mercapto-N-(5-mercaptopentyl)-acetamid
- 3-Mercapto-2-(2-mercapto-acetylamino)-propionamid
- 3-Mercapto-2-(2-mercapto-acetylamino)-N,N-dimethyl-propionamid
- 2-Mercapto-N-(2-mercapto-1,1-dimethyl-ethyl)-acetamid
- 2-Mercapto-N-(1-mercaptomethyl-propyl)-acetamid
- Ethyl-mercapto-[(mercaptoacetyl)amino]-acetat
- N-(Mercaptoacetyl)-L-cystein
- N-(Mercaptoacetyl)-L-homocystein
- N-(Mercaptoacetyl)-L-homocystein, Natriumsalz
- 2-Mercapto-N-mercaptomethyl-propionamid
- 2-Mercapto-N-(2-mercaptoethyl)-propionamid
- 2-Mercapto-N-(3-mercaptopropyl)-propionamid
- 2-Mercapto-N-(4-mercaptobutyl)-propionamid
- 2-Mercapto-N-(5-mercaptopentyl)-propionamid
- Mercapto-[(2-mercapto-1-oxopropyl)amino]-essigsäure
- N-(2-Mercapto-1-oxopropyl)-DL-cystein
- (R)-N-(2-Mercapto-1-oxopropyl)-L-cystein
- (S)-N-(2-Mercapto-1-oxopropyl)-L-cystein
- 2-Mercaptopropionyl-L-cystein
- N-(1-Carbamoyl-2-mercaptoethyl)-2-mercaptopropionamid
- N-(1-Dimethylcarbamoyl-2-mercaptoethyl)-2-mercaptopropionamid
- 4-Mercapto-2-(2-mercapto-propionylamino)-buttersäure
- 2-Mercapto-N-(2-mercapto-1,1-dimethylethyl)-propionamid
- 2-Mercaptobuttersäure-mercaptomethylamid
- 2-Mercaptobuttersäure-(2-mercaptoethyl)-amid
- 2-Mercaptobuttersäure-(3-mercaptopropyl)-amid
- 2-Mercaptobuttersäure-(4-mercaptobutyl)-amid
- 2-Mercaptobuttersäure-(5-mercaptopentyl)-amid
- 2-Mercaptobuttersäure-(1-carbamoyl-2-mercaptoethyl)-amid
- N-(1-Dimethylcarbamoyl-2-mercaptoethyl)-2-mercaptobutyramid
- 2-Mercaoptobuttersäure-(1-carbamoyl-3-mercaptopropyl)-amid
- 2-Mercaptobuttersäure-(2-mercapto-1,1-dimethylethyl)-amid
- 2-Mercaptopentansäure-mercaptomethyl-amid
- 2-Mercaptopentansäure-(2-mercaptoethyl)-amid
- 2-Mercaptopentansäure-(3-mercaptopropyl)-amid
- 2-Mercaptopentansäure-(4-mercaptobutyl)-amid
- 2-Mercaptopentansäure-(5-mercaptopentyl)-amid
- 3-Mercapto-2-(2-mercapto-pentanoylamino)-propionsäure
- 2-Mercaptopentansäure-(1-carbamoyl-2-mercaptoethyl)-amid
- 2-Mercaptopentansäure-(1-dimethylcarbamoyl-2-mercaptoethyl)-amid
- 2-Mercaptopentansäure-(1-carbamoyl-3-mercaptopropyl)-amid
- 2-Mercaptopentansäure-(2-mercapto-1,1-dimethylethyl)-amid
- Ethyl-mercapto-[(mercaptophenylacetyl)amino]-acetat
- (R)-N-[2-(mercaptomethyl)-1-oxo-3-phenylpropyl]-L-cystein
- (R)-N-[2-(mercaptomethyl)-1-oxo-3-phenylpropyl]-L-homocystein
- (S)-N-[2-(Mercaptomethyl)-1-oxo-3-phenylpropyl]-L-homocystein
- 3-Mercapto-N-(2-mercaptoethyl)-propanamid
- 3-Mercapto-N-mercaptomethyl-propionamid
- 3-Mercapto-N-(3-mercaptopropyl)-propionamid
- 3-Mercapto-N-(4-mercaptobutyl)-propionamid
- N-(3-Mercapto-1-oxopropyl)-L-cystein
- 3-Mercapto-2-(3-mercapto-propionylamino)-propionamid
- N-(1-Dimethylcarbamoyl-2-mercaptoethyl)-3-mercaptopropionamid
- 4-Mercapto-2-(3-mercapto-propionylamino)-buttersäure
- 3-Mercapto-N-(2-mercapto-1, 1 -dimethylethyl)-propionamid
- 3-Mercapto-N-mercaptomethyl-2-methyl-propionamid
- 3-Mercapto-N-(2-mercaptoethyl)-2-methyl-propionamid
- 3-Mercapto-N-(3-mercaptopropyl)-2-methyl-propionamid
- 3-Mercapto-N-(4-mercaptobutyl)-2-methyl-propionamid
- 3-Mercapto-2-(3-mercapto-2-methyl-propionylamino)-propionsäure
- N-(1-Carbamoyl-2-mercaptoethyl)-3-mercapto-2-methyl-propionamid
- N-(1-Dimethylcarbamoyl-2-mercaptoethyl)-3-mercapto-2-methylpropionamid
- 3-Mercapto-N-(2-mercapto-1,1-dimethylethyl)-2-methyl-propionamid
- N-(3-Mercapto-2-methyl-1-oxopropyl)-L-homocystein
- N-[2-(Mercaptomethyl)-1-oxo-3-phenylpropyl]-cystein
- Methyl-4-[(1-carboxy-2-mercaptoethyl)amino]-3-(mercaptomethyl)-4-oxo-butanoat
- N-Mercaptomethyl-3-mercaptomethyl-succinamsäure
- N-(2-Mercaptoethyl)-3-mercaptomethyl-succinamsäure
- N-(3-Mercaptopropyl)-3-mercaptomethyl-succinamsäure
- N-(4-Mercaptobutyl)-3-mercaptomethyl-succinamsäure
- N-(1-Carboxy-2-mercaptoethyl)-3-mercaptomethyl-succinamsäure
- N-(1-Carboxy-3-mercaptopropyl)-3-mercaptomethyl-succinamsäure
- N-(2-Mercapto- 1, 1 -dimethylethyl)-3-mercaptomethyl-succinamsäure
- N-(2-Mercapto-1-ethyl)-3-mercaptomethyl-succinamsäure
- 2-Mercapto-N-mercaptomethyl-2-methyl-propionamid
- 2-Mercapto-N-(2-mercaptoethyl)-2-methylpropanamid
- 2-Mercapto-N-(3-mercaptopropyl)-2-methylpropionamid
- 2-Mercapto-N-(4-mercaptobutyl)-2-methylpropionamid
- 2-Mercapto-N-(5-mercaptopentyl)-2-methylpropionamid
- 2-Mercapto-N-(2-mercapto-1, 1 -dimethylethyl)-2-methyl-propionamid
- 2-Mercapto-N-(2-mercapto-1-ethyl)-2-methyl-propionamid
- N-(2-Mercapto-2-methyl-1-oxopropyl)-D-cystein
- N-(2-Mercapto-2-methyl-1-oxopropyl)-cystein
- N-(2-Mercapto-2-methyl-1-oxopropyl)-L-cystein, Mononatriumsalz
- N-(2-Mercapto-2-methyl-1-oxopropyl)-L-cystein
- (R)-N-[2-Amino-1-(mercaptomethyl)-2-oxoethyl]-2-mercapto-2-methylpropanamid
- Methyl-N-(2-mercapto-2-methyl-1-oxopropyl)-L-cysteinat
- (2R)-3-Mercapto-2-[(2-mercapto-2-methyl-1-oxopropyl)amino]-N,N-dimethyl-propanamid
- N2-[N-(2-Mercapto-2-methyl-1-oxopropyl)-L-cysteinyl]-L-lysin
- N-(2-Mercapto-2-methyl-1-oxopropyl)-homocystein
- N-(2-Ethyl-2-mercapto-1-oxobutyl)-L-cystein
- 3-Mercapto-N-mercaptomethyl-3-methylbutyramid
- 3-Mercapto-N-(2-mercaptoethyl)-3-methyl-butanamid
- 3-Mercapto-N-(3-mercaptopropyl)-3-methyl-butyramid
- 3-Mercapto-N-(4-mercaptobutyl)-3-methyl-butyramid
- N-(3-Mercapto-3-methyl-1-oxobutyl)-L-cystein
- N-(1-Carbamoyl-2-mercaptoethyl)-3-mercapto-3-methyl-butyramid
- N-(1-Dimethylcarbamoyl-2-mercaptoethyl)-3-mercapto-3-methyl-butyramid
- 4-Mercapto-2-(3-mercapto-3-methyl-butyrylamino)-buttersäure
- 3-Mercapto-N-(2-mercapto-1,1-dimethylethyl)-3-methyl-butyramid
- 3-Mercapto-N-(2-mercapto-1-ethyl)-3-methyl-butyramid
- 3-Mercapto-N-mercaptomethyl-2,2-dimethyl-propionamid
- 3-Mercapto-N-(2-mercaptoethyl)-2,2-dimethyl-propionamid
- 3-Mercapto-N-(3-mercaptopropyl)-2,2-dimethyl-propionamid
- 3-Mercapto-N-(4-mercaptobutyl)-2,2-dimethyl-propionamid
- 3-Mercapto-N-(2-mercapto-1,1-dimethylethyl)-2,2-dimethyl-propionamid
- N-(3-Mercapto-2,2-dimethyl-1-oxopropyl)-D-cystein
- N-(3-Mercapto-2,2-dimethyl-1-oxopropyl)-L-cystein
- N-(1-Carbamoyl-2-mercaptoethyl)-3-mercapto-2,2-dimethyl-propionamid
- N-(1-Dimethylcarbamoyl-2-mercaptoethyl)-3-mercapto-2,2-dimethylpropionamid
- N-(3-Mercapto-2,2-dimethyl-1-oxopropyl)-D-cysteinyl-glycinamid
- N6-[(1,1-Dimethylethoxy)carbonyl]-N2-[N-(3-mercapto-2,2-dimethyl-1-oxopropyl)-L-cysteinyl]-L-lysin
- N-[2-[(4-Aminobutyl)amino]-1-(mercaptomethyl)-2-oxoethyl]-3-mercapto-2,2-dimethyl-propanamid, Monohydrochlorid
- N-[2-[(5-Aminopentyl)amino]-1-(mercaptomethyl)-2-oxoethyl]-3-mercapto-2,2-dimethyl-propanamid, Monohydrochlorid
- N-[2-[(6-Aminohexyl)amino]-1-(mercaptomethyl)-2-oxoethyl]-3-mercapto-2,2-dimethyl-propanamid, Monohydrochlorid
- (R)-N-[2-[(5-Hydroxypentyl)amino]-1-(mercaptomethyl)-2-oxoethyl]-3-mercapto-2,2-dimethyl-propanamid
- N2-[N-(3-Mercapto-2,2-dimethyl-1-oxopropyl)-L-cysteinyll-L-lysin
- N2-[N-(3-Mercapto-2,2-dimethyl-1-oxopropyl)-L-cysteinyl]-N6,N6-dimethyl-L-lysin
- 3-Mercapto-N-(3-mercapto-2,2-dimethyl-1-oxopropyl)-D-valin
- N-(3-Mercapto-2,2-dimethyl-1-oxopropyl)-homocystein
- N-[2-Ethyl-2-(mercaptomethyl)-1-oxobutyl)-L-cystein
- 2-Amino-3-mercapto-N-mercaptomethyl-propionamid
- (2R)-2-Amino-3-mercapto-N-(2-mercaptoethyl)-propanamid
- (2S)-2-Amino-3-mercapto-N-(2-mercaptoethyl)-propanamid
- 2-Amino-3-mercapto-N-(3-mercaptopropyl)-propionamid
- 2-Amino-3-mercapto-N-(4-mercaptobutyl)-propionamid
- L-Cysteinyl-L-cystein
- Methyl-mercapto-[(mercaptoacetyl)amino]-acetat
- Ethyl-L-cysteinyl-L-cysteinat
- 2-Amino-N-(1-carbamoyl-2-mercaptoethyl)-3-mercaptopropionamid
- 2-Amino-N-(1-carbamoyl-2-mercaptoethyl)-3-mercapto-N,N-dimethylpropionamid
- 2-(2-Amino-3-mercapto-propionylamino)-4-mercaptobuttersäure
- 2-Amino-3-mercapto-N-(2-mercapto-1, 1 -dimethylethyl)-propionamid
- 2-Amino-3-mercapto-N-(2-mercapto-1-ethyl)-propionamid
- 2-Acetylamino-3-mercapto-N-mercaptomethyl-propionamid
- (2R)-2-(Acetylamino)-3-mercapto-N-(2-mercaptoethyl)-propanamid
- 2-Acetylamino-3-mercapto-N-(3-mercaptopropyl)-propionamid
- 2-Acetylamino-3-mercapto-N-(4-mercaptobutyl)-propionamid
- 2-(2-Acetylamino-3-mercapto-propionylamino)-3-mercapto-3-methylbuttersäure
- 2-Acetylamino-3-mercapto-N-(1-mercaptomethylpropyl)-propionamid
- 2-Acetylamino-N-(1-dimethylcarbamoyl-2-mercaptoethyl)-3-mercapto-propionamid
- N-Acetyl-L-cysteinyl-L-cystein
- N-(N-Acetylcysteinyl)-L-cystein
- N-(N-Acetylcysteinyl)-DL-cystein
- Ethyl-N-acetyl-L-cysteinyl-D-cysteinat
- Ethyl-N-acetyl-L-cysteinyl-L-cysteinat
- 2-Acetamido-N-(1-carbamoyl-2-mercaptoethyl)-3-mercapto-propionamid
- Ethyl-N-acetyl-L-cysteinyl-L-cysteinyl-glycinat
- 2-(2-Acetylamino-3-mercapto-propionylamino)-4-mercaptobuttersäure
- 2-(Carboxymethylamino)-3-mercapto-N-mercaptomethyl-propionamid
- 2-(Carboxymethylamino)-3-mercapto-N-(2-mercaptoethyl)-propionamid
- 2-(Carboxymethylamino)-3-mercapto-N-(3-mercaptopropyl)-propionamid
- 2-(Carboxymethylamino)-3-mercapto-N-(4-mercaptobutyl)-propionamid
- 2-(Carboxymethylamino)-N-(1-carbamoyl-2-mercaptoethyl)-3-mercapto-propionamid
- 2-[2-(Carboxymethylamino)-3-mercapto-propionylamino]-3-mercaptopropionsäure
- [1-(1-Carbamoyl-2-mercapto-ethylcarbamoyl)-2-mercaptoethylamino]-essigsäure
- 2-(Carboxymethylamino)-3-mercapto-N-(2-mercapto-1,1-dimethylethyl)-propionamid
- N-[N-[N-(Carboxymethyl)-L-cysteinyl]-L-cysteinyl]-D-valin
- 2-Amino-4-[2-mercapto-1-(mercapto-methylcarbamoyl)-ethylcarbamoyl]-buttersäure
- 2-Amino-4-[2-mercapto-1-(2-mercapto-ethylcarbamoyl)-ethylcarbamoyl]-buttersäure
- 2-Amino-4-[2-mercapto-1-(3-mercapto-propylcarbamoyl)-ethylcarbamoyl]-buttersäure
- 2-Amino-4-[2-mercapto-1-(4-mercapto-butylcarbamoyl)-ethylcarbamoyl]-buttersäure
- L-gamma-Glutamyl-L-cysteinyl-L-cystein
- 2-[2-(4-Amino-4-carboxy-butyrylamino)-3-mercapto-propionylamino]-4-mercaptobuttersäure
- 2-Amino-4-[1-(1-carbamoyl-2-mercapto-ethylcarbamoyl)-2-mercapto-ethylcarbamoyl]-buttersäure
- 2-Amino-4-[1-(1-dimethylcarbamoyl-2-mercapto-ethylcarbamoyl)-2-mercapto-ethylcarbamoyl]-buttersäure
- 2-Amino-4-[2-mercapto-1-(2-mercapto-1,1-dimethyl-ethylcarbamoyl)-ethylcarbamoyl]-buttersäure
- 2-Amino-4-[2-mercapto-1-(2-mercapto-1-ethyl-ethylcarbamoyl)-ethylcarbamoyl]-buttersäure
- 2-(2-Aminoacetylamino)-3-mercapto-N-mercaptomethyl-propionamid
- 2-(2-Aminoacetylamino)-3-mercapto-N-(2-mercaptoethyl-propionamid
- 2-(2-Aminoacetylamino)-3-mercapto-N-(3-mercaptopropyl-propionamid
- 2-(2-Aminoacetylamino)-3-mercapto-N-(4-mercaptobutyl-propionamid
- 2-(2-Aminoacetylamino)-3-mercapto-N-(5-mercaptopentyl-propionamid
- Glycyl-L-cysteinyl-L-cystein
- 2-[2-(2-Amino-acetylamino)-3-mercapto-propionylamino]-4-mercaptobuttersäure
- 2-(2-Amino-acetylamino)-3-mercapto-N-(2-mercapto-1,1-dimethylethyl]-propionamid
- 2-(2-Amino-acetylamino)-3-mercapto-N-(1-mercaptomethylpropyl]-propionamid
- 4-Carboxy-L-alpha-glutamyl-L-cysteinyl-L-cystein
- (S)-N-[N-(5-Amino-5-carboxy-1-oxopentyl)-L-homocysteinyl]-L-cystein
- 4-Mercapto-N-mercaptomethyl-butyramid
- 4-Mercapto-N-(2-mercaptoethyl)-butyramid
- 4-Mercapto-N-(3-mercaptopropyl)-butyramid
- N-(4-Mercapto-1-oxobutyl)-L-cystein
- 4-Mercapto-2-(3-mercapto-propionylamino)-butyramid
- N-(1-Dimethylcarbamoyl-2-mercapto-ethyl)-4-mercapto-butyramid
- N-(4-Mercapto-1-oxobutyl)-L-homocystein
- 4-Mercapto-N-(2-mercapto-1,1-dimethyl-ethyl)-butyramid
- 4-Mercapto-N-(1-mercaptomethyl-propyl)-butyramid
- 5-Mercaptopentansäure-mercaptomethylamid
- 5-Mercaptopentansäure-(2-mercapto-ethyl)-amid
- N-(5-Mercapto-1-oxopentyl)-L-cystein
- 5-Mercapto-2-(3-mercapto-propionylamino)-pentanamid
- N-(1-Dimethylcarbamoyl-2-mercapto-ethyl)-5-mercapto-pentanamid
- 5-Mercapto-N-(2-mercapto-1,1-dimethyl-ethyl)-pentanamid
- 5-Mercapto-N-(1-mercaptomethyl-propyl)-pentanamid
- 6-Mercapto-hexansäure-mercaptomethylamid.

3. Verwendung nach Anspruch 2, **dadurch gekennzeichnet, dass** das Dimercaptoamid der Formel (I) unter den folgenden Verbindungen ausgewählt ist:
- 2-Mercapto-N-(mercaptomethyl)-acetamid
- 2-Mercapto-N-(2-mercaptoethyl)-acetamid
- 2-Mercapto-N-(3-mercaptopropyl)-acetamid
- 2-Mercapto-N-(4-mercaptobutyl)-acetamid
- 2-Mercapto-N-(5-mercaptopentyl)-acetamid
- 3-Mercapto-2-(2-mercapto-acetylamino)-propionamid
- 3-Mercapto-2-(2-mercapto-acetylamino)-N,N-dimethyl-propionamid
- 2-Mercapto-N-(2-mercapto-1,1 -dimethyl-ethyl)-acetamid
- 2-Mercapto-N-(1-mercaptomethyl-propyl)-acetamid
- N-(Mercaptoacetyl)-L-cystein
- N-(Mercaptoacetyl)-L-homocystein
- 2-Mercapto-N-mercaptomethyl-propionamid
- 2-Mercapto-N-(2-mercaptoethyl)-propionamid
- 2-Mercapto-N-(3-mercaptopropyl)-propionamid
- 2-Mercapto-N-(4-mercaptobutyl)-propionamid
- 2-Mercapto-N-(5-mercaptopentyl)-propionamid
- Mercapto-[(2-mercapto-1-oxopropyl)amino]-essigsäure
- 2-Mercaptopropionyl-L-cystein
- N-(1-Carbamoyl-2-mercaptoethyl)-2-mercaptopropionamid
- N-(1-Dimethylcarbamoyl-2-mercaptoethyl)-2-mercaptopropionamid
- 4-Mercapto-2-(2-mercapto-propionylamino)-buttersäure
- 2-Mercapto-N-(2-mercapto-1,1-dimethylethyl)-propionamid
- 2-Mercaptobuttersäure-mercaptomethylamid
- 2-Mercaptobuttersäure-(2-mercaptoethyl)-amid
- 2-Mercaptobuttersäure-(3-mercaptopropyl)-amid
- 2-Mercaptobuttersäure-(4-mercaptobutyl)-amid
- 2-Mercaptopentansäure-mercaptomethylamid
- 2-Mercaptopentansäure-(2-mercaptoethyl)-amid
- 2-Mercaptopentansäure-(3-mercaptopropyl)-amid
- 3-Mercapto-N-(2-mercaptoethyl)-propanamid
- 3-Mercapto-N-mercaptomethyl-propionamid
- 3-Mercapto-N-(3-mercapto-propyl)-propionamid
- 3-Mercapto-N-(4-mercapto-butyl)-propionamid
- N-(3-Mercapto-1-oxopropyl)-L-cystein
- 3-Mercapto-2-(3-mercapto-propionylamino)-propionamid
- N-(1-Dimethylcarbamoyl-2-mercaptoethyl)-3-mercaptopropionamid
- 4-Mercapto-2-(3-mercapto-propionylamino)-buttersäure
- 3-Mercapto-N-(2-mercapto- 1, 1 -dimethylethyl)-propionamid
- 3-Mercapto-N-mercaptomethyl-2-methyl-propionamid
- 3-Mercapto-N-(2-mercaptoethyl)-2-methyl-propionamid
- 3-Mercapto-N-(3-mercaptopropyl)-2-methyl-propionamid
- 3-Mercapto-N-(4-mercaptobutyl)-2-methyl-propionamid
- 3-Mercapto-2-(3-mercapto-2-methyl-propionylamino)-propionsäure
- N-(1-Carbamoyl-2-mercaptoethyl)-3-mercapto-2-methyl-propionamid
- N-(1-Dimethylcarbamoyl-2-mercaptoethyl)-3-mercapto-2-methyl-propionamid
- 3-Mercapto-N-(2-mercapto-1,1-dimethylethyl)-2-methyl-propionamid
- N-(3-Mercapto-2-methyl-1-oxopropyl)-L-homocystein
- N-Mercaptomethyl-3-mercaptomethyl-succinamsäure
- N-(2-Mercaptoethyl)-3-mercaptomethyl-succinamsäure
- N-(3-Mercaptopropyl)-3-mercaptomethyl-succinamsäure
- N-(4-Mercaptobutyl)-3-mercaptomethyl-succinamsäure
- N-(1-Carboxy-2-mercaptoethyl)-3-mercaptomethyl-succinamsäure
- 2-Mercapto-N-mercaptomethyl-2-methyl-propionamid
- 2-Mercapto-N-(2-mercaptoethyl)-2-methylpropanamid
- 2-Mercapto-N-(3-mercaptopropyl)-2-methylpropionamid
- 2-Mercapto-N-(4-mercaptobutyl)-2-methylpropionamid
- 2-Mercapto-N-(2-mercapto-1,1-dimethylethyl)-2-methyl-propionamid
- 2-Mercapto-N-(2-mercapto-1-ethyl)-2-methyl-propionamid
- N-(2-Mercapto-2-methyl-1-oxopropyl)-L-cystein
- N-(2-Ethyl-2-mercapto-1-oxobutyl)-L-cystein
- 3-Mercapto-N-mercaptomethyl-3-methylbutyramid
- 3-Mercapto-N-(2-mercaptoethyl)-3-methyl-butanamid
- 3-Mercapto-N-(3-mercaptopropyl)-3-methyl-butyramid
- N-(3-Mercapto-3-methyl-1-oxobutyl)-L-cystein
- N-(1-Carbamoyl-2-mercaptoethyl)-3-mercapto-3-methyl-butyramid
- N-(1-Dimethylcarbamoyl-2-mercaptoethyl)-3-mercapto-3-methyl-butyramid
- 4-Mercapto-2-(3-mercapto-3-methyl-butyrylamino)-buttersäure
- 3-Mercapto-N-mercaptomethyl-2,2-dimethyl-propionamid
- 3-Mercapto-N-(2-mercaptoethyl)-2,2-dimethyl-propionamid
- 3-Mercapto-N-(3-mercaptopropyl)-2,2-dimethyl-propionamid
- 3-Mercapto-N-(4-mercaptobutyl)-2,2-dimethyl-propionamid
- N-(3-Mercapto-2,2-dimethyl-1-oxopropyl)-L-cystein
- N-(1-Carbamoyl-2-mercaptoethyl)-3-mercapto-2,2-dimethyl-propionamid
- N-(1-Dimethylcarbamoyl-2-mercaptoethyl)-3-mercapto-2,2-dimethylpropionamid
- N-[2-Ethyl-2-(mercaptomethyl)-1-oxobutyl)-L-cystein
- 2-Amino-3-mercapto-N-mercaptomethyl-propionamid
- (2R)-2-Amino-3-mercapto-N-(2-mercaptoethyl)-propanamid
- (2S)-2-Amino-3-mercapto-N-(2-mercaptoethyl)-propanamid
- 2-Amino-3-mercapto-N-(3-mercaptopropyl)-propionamid
- 2-Amino-3-mercapto-N-(4-mercaptobutyl)-propionamid
- L-Cysteinyl-L-cystein
- 2-Amino-N-(1-carbamoyl-2-mercaptoethyl)-3-mercaptopropionamid
- 2-Amino-N-(1-carbamoyl-2-mercaptoethyl)-3-mercapto-N,N-dimethylpropionamid
- 2-(2-Amino-3-mercapto-propionylamino)-4-mercaptobuttersäure
- 2-Amino-3-mercapto-N-(2-mercapto-1,1-dimethylethyl)-propionamid
- 2-Amino-3-mercapto-N-(2-mercapto-1-ethyl)-propionamid
- 2-Acetylamino-3-mercapto-N-mercaptomethyl-propionamid
- N-Acetyl-L-cysteinyl-L-cystein
- 2-(Carboxymethylamino)-3-mercapto-N-mercaptomethyl-propionamid
- 2-(Carboxymethylamino)-3-mercapto-N-(2-mercaptoethyl)-propionamid
- 2-(Carboxymethylamino)-3-mercapto-N-(3-mercaptopropyl)-propionamid
- L-gamma-Glutamyl-L-cysteinyl-L-cystein
- 2-(2-Aminoacetylamino)-3-mercapto-N-mercaptomethyl-propionamid
- 2-(2-Aminoacetylamino)-3-mercapto-N-(2-mercaptoethyl-propionamid
- Glycyl-L-cysteinyl-L-cystein
- 4-Carboxy-L-alpha-glutamyl-L-cysteinyl-L-cystein
- 4-Mercapto-N-mercaptomethyl-butyramid
- 4-Mercapto-N-(2-mercaptoethyl)-butyramid
- 4-Mercapto-N-(3-mercaptopropyl)-butyramid
- N-(4-Mercapto-1-oxobutyl)-L-cystein
- 4-Mercapto-2-(3-mercapto-propionylamino)-butyramid
- N-(1-Dimethylcarbamoyl-2-mercapto-ethyl)-4-mercapto-butyramid
- 4-Mercapto-N-(2-mercapto-1,1-dimethyl-ethyl)-butyramid
- 4-Mercapto-N-(1-mercaptomethyl-propyl)-butyramid
- 5-Mercaptopentansäure-mercaptomethylamid
- 5-Mercaptopentansäure-(2-mercapto-ethyl)-amid
- N-(5-Mercapto-1-oxopentyl)-L-cystein
- 5-Mercapto-2-(3-mercapto-propionylamino)-pentanamid
- N-(1-Dimethylcarbamoyl-2-mercapto-ethyl)-5-mercapto-pentanamid
- 5-Mercapto-N-(2-mercapto-1,1-dimethyl-ethyl)-pentanamid
- 5-Mercapto-N-(1-mercaptomethyl-propyl)-pentanamid
- 6-Mercapto-hexansäure-mercaptomethylamid.

4. Verfahren zur dauerhaften Verformung von Keratinfasern und insbesondere menschlichen Keratinfasern wie Haaren, das das Aufbringen einer reduzierenden Zusammensetzung und anschließend einer oxidierenden Zusammensetzung auf die Fasern umfasst, **dadurch gekennzeichnet, dass** die reduzierende Zusammensetzung als Reduktionsmittel mindestens ein Dimercaptoamid der folgenden Formel (I) enthält:
HS-A-CO-NH-B-SH (I)
wobei in der Formel:
A bedeutet eine Gruppe (CH₂)ₙ, wobei n eine ganze Zahl im Bereich von 1 bis 5 ist, die gegebenenfalls substituiert ist mit:
(i) einer geradkettigen oder verzweigten C₁₋₅-Alkylgruppe; Phenyl; Benzyl; Amino; Acetylamino; NH-CO-CH₂-NH₂; NH-CO-CH₂-CH₂CH(NH₂)COOH; NH-CO-CH₂-CH₂-CH(COOH)COOH; CH₂-COOH; CH₂COOCH₃; oder CH₂-COOCH₂-CH₃, oder
(ii) zwei Methylgruppen oder zwei Ethylgruppen
und B bedeutet eine Gruppe (CH₂)ₚ, wobei p eine ganze Zahl im Bereich von 1 bis 5 ist, die gegebenenfalls substituiert ist mit:
(i) einer geradkettigen oder verzweigten C₁₋₅-Alkylgruppe; Carboxy; COOCH₃; COOEt; CONH₂; CONH-CH₃; CON(CH₃)₂; CONH-CH₂-CH₃; CON(CH₂-CH₃)₂; CONH-CH₂-CHOH-CH₃; CO-NH-CH(COOH)-(CH₂)₄-NH₂; CO-NH-CH(COOH)-(CH₂)₄-N(CH₃)₂; CO-NH-CH₂-CH₂-COOEt; CO-NH-CH(COOH)-iPr; CO-NH-CH₂-R mit R = CO-NH₂ oder (CH₂)₃-NH₂ oder (CH₂)₄-NH₂ oder (CH₂)₅-NH₂ oder (CH₂)₄-OH, oder
(ii) 2 Methylgruppen,
wobei die Summe n+p im Bereich von 2 bis 6 liegt,
und ihre organischen und anorganischen Salze.

5. Verfahren nach Anspruch 4, **dadurch gekennzeichnet, dass** das Dimercaptoamid der Formel (I) in einer Konzentration von 0,05 bis 35 % und vorzugsweise 1 bis 20 Gew.-% des Gesamtgewichts der reduzierenden Zusammensetzung enthalten ist.

6. Verfahren nach Anspruch 4 oder 5, **dadurch gekennzeichnet, dass** der pH-Wert der reduzierenden Zusammensetzung im Bereich von 4 bis 11 und vorzugsweise 6 bis 10 liegt.

7. Verfahren nach einem der Ansprüche 4 bis 6, **dadurch gekennzeichnet, dass** die reduzierende Zusammensetzung ferner ein oder mehrere Reduktionsmittel enthält, die unter Thioglycolsäure, Thiomilchsäure, deren Esterderivaten und Amidderivaten, Cysteamin und seinen C₁₋₄-acylierten Derivaten, Cystein, N-Acetylcystein, Thioäpfelsäure, Pantethein, 2,3-Dimercaptobernsteinsäure, Sulfiten oder Bisulfiten eines Alkalimetalls oder Erdalkalimetalls, N-(Mercaptoalkyl)-ω-hydroxyalkylamiden, N-Mono- oder N,N-Dialkyl-mercapto-4-butyramiden, Aminomercaptoalkylamiden, Derivaten von N-(Mercaptoalkyl)succinamsäuren und N-(Mercaptoalkyl)succinimiden, Alkylaminomercaptoalkylamiden, dem azeotropen Gemisch von 2-Hydroxypropylthioglycolat und (2-Hydroxy-1-methyl)ethylthioglycolat, Mercaptoalkylaminoamiden, N-Mercaptoalkylalkandiamiden und Derivaten von Formamidinsulfinsäurederivaten ausgewählt sind.

8. Verfahren nach einem der Ansprüche 4 bis 7, **dadurch gekennzeichnet, dass** die reduzierende Zusammensetzung ferner mindestens einen nichtionischen, anionischen, kationischen oder amphoteren grenzflächenaktiven Stoff oder ein Gemisch enthält.

9. Verfahren nach Anspruch 8, **dadurch gekennzeichnet, dass** der grenzflächenaktive Stoff unter den Alkylsulfaten, Alkylbenzolsulfaten, Alkylethersulfaten, Alkylsulfonaten, quartären Ammoniumsalzen, Alkylbetainen, ethoxylierten Alkylphenolen, Alkanolamiden von Fettsäuren, ethoxylierten Fettsäureestern, Hydroxypropylethern ausgewählt ist.

10. Verfahren nach einem der Ansprüche 4 bis 9, **dadurch gekennzeichnet, dass** die reduzierende Zusammensetzung ferner eine Verbindung enthält, die unter den geradkettigen oder cyclischen, flüchtigen oder nichtflüchtigen Siliconen und deren Gemischen, Polydimethylsiloxanen, quaternisierten Polyorganosiloxanen, Polyorganosiloxanen mit Aminoalkylgruppen, die mit Alkoxycarbonylalkylgruppen modifiziert sind, Polydimethylsiloxanen mit endständigen Stearoxydimethicongruppen, Polydimethylsiloxan/Dialkylammoniumacetat-Copolymeren, Polydimethylsiloxan/Polylalkylbetain-Copolymeren, mit Mercapto- oder Mercaptoalkylgruppen organomodifizierten Polysiloxanen und Silanen ausgewählt sind.

11. Verfahren nach einem der Ansprüche 4 bis 9, **dadurch gekennzeichnet, dass** die reduzierende Zusammensetzung ferner eine Verbindung enthält, die unter den kationischen Polymeren vom Typ der Ionene, basischen Aminosäuren, Glutaminsäure, Asparaginsäure, Peptiden und ihren Derivaten, Proteinhydrolysaten, Wachsen, Quellmitteln und Penetrationsförderem, Dimethylisosorbitol, Harnstoff und seinen Derivaten, Pyrrolidon, N-Alkyl-pyrrolidonen, Thiamorpholinon, Alkylethern von Alkylenglycol oder Dialkylenglycol, C₃₋₆-Alkandiolen, Imidazolidinon-2, Fettalkoholen, Lanolinderivaten, Panthothensäure, Wirkstoffen gegen Haarausfall, Antischuppenmitteln, Verdickungsmitteln, Suspendiermitteln, Maskierungsmitteln, Trübungsmitteln, Farbmitteln, Sonnenschutzfiltern, Parfums und Konservierungsmitteln, Verdickungsmitteln und Gelbildnern ausgewählt ist.

12. Verfahren nach einem der Ansprüche 4 bis 11, **dadurch gekennzeichnet, dass** die oxidierende Zusammensetzung als Oxidationsmittel Wasserstoffperoxid, ein Alkalibromat, ein Persalz, ein Polythionat oder ein Gemisch aus einem Alkalibromat und einem Persalz enthält.

13. Reduzierende Zusammensetzung, **dadurch gekennzeichnet, dass** sie als Reduktionsmittel mindestens ein Dimercaptoamid der folgenden allgemeinen Formel (I) enthält:
HS-A-CO-NH-B-SH (I)
wobei in der Formel:
A bedeutet eine Gruppe (CH₂)ₙ, wobei n eine ganze Zahl im Bereich von 1 bis 5 ist, die gegebenenfalls substituiert ist mit:
(i) einer geradkettigen oder verzweigten C₁₋₅-Alkylgruppe; Phenyl; Benzyl; Amino; Acetylamino; NH-CO-CH₂-NH₂; NH-CO-CH₂-CH₂CH(NH₂)COOH; NH-CO-CH₂-CH₂-CH(COOH)COOH; CH₂-COOH; CH₂COOCH₃; oder CH₂-COOCH₂-CH₃, oder
(ii) zwei Methylgruppen oder zwei Ethylgruppen
und B bedeutet eine Gruppe (CH₂)_{p,} wobei p eine ganze Zahl im Bereich von 1 bis 5 ist, die gegebenenfalls substituiert ist mit:
(i) einer geradkettigen oder verzweigten C₁₋₅-Alkylgruppe; Carboxy; COOCH₃; COOEt; CONH₂; CONH-CH₃; CON(CH₃)₂; CONH-CH₂-CH₃; CON(CH₂-CH₃)₂; CONH-CH₂-CHOH-CH₃; CO-NH-CH(COOH)-(CH₂)₄-NH₂; CO-NH-CH(COOH)-(CH₂)₄-N(CH₃)₂; CO-NH-CH₂-CH₂-COOEt; CO-NH-CH(COOH)-iPr; CO-NH-CH₂-R mit R = CO-NH₂ oder (CH₂)₃-NH₂ oder (CH₂)₄-NH₂ oder (CH₂)₅-NH₂ oder (CH₂)₄-OH, oder
(ii) 2 Methylgruppen,
wobei die Summe n+p im Bereich von 2 bis 6 liegt,
und ihre organischen und anorganischen Salze.

14. Zusammensetzung nach Anspruch 13, **dadurch gekennzeichnet, dass** das Dimercaptoamid der Formel (I) unter den in Anspruch 2 oder 3 genannten Verbindungen ausgewählt ist.

15. Zusammensetzung nach Anspruch 13 oder 14, **dadurch gekennzeichnet, dass** das Dimercaptoamid der Formel (I) in einer Konzentration von 0,05 bis 35 % und vorzugsweise 1 bis 20 Gew.-% des Gesamtgewichts der reduzierenden Zusammensetzung enthalten ist.

16. Zusammensetzung nach einem der Ansprüche 13 bis 15, **dadurch gekennzeichnet, dass** der pH-Wert der Zusammensetzung im Bereich von 4 bis 11 und vorzugsweise 6 bis 10 liegt.

17. Zusammensetzung nach einem der Ansprüche 13 bis 16, **dadurch gekennzeichnet, dass** sie ferner ein oder mehrere Reduktionsmittel enthält, die unter Thioglycolsäure, Thiomilchsäure, deren Esterderivaten und Amidderivaten, Cysteamin und seinen C₁₋₄-acylierten Derivaten, Cystein, N-Acetylcystein, Thioäpfelsäure, Pantethein, 2,3-Dimercaptobernsteinsäure, Sulfiten oder Bisulfiten eines Alkalimetalls oder Erdalkalimetalls, N-(Mercaptoalkyl)-ω-hydroxyalkylamiden, N-Mono- oder N,N-Dialkyl-mercapto-4-butyramiden, Aminomercaptoalkylamiden, Derivaten von N-(Mercaptoalkyl)succinamsäuren und N-(Mercaptoalkyl)succinimiden, Alkylaminomercaptoalkylamiden, dem azeotropen Gemisch von 2-Hydroxypropylthioglycolat und (2-Hydroxy-1-methyl)ethylthioglycolat, Mercaptoalkylaminoamiden, N-Mercaptoalkylalkandiamiden und Derivaten von Formamidinsulfinsäurederivaten ausgewählt sind.

18. Zusammensetzung nach einem der Ansprüche 13 bis 17, **dadurch gekennzeichnet, dass** sie ferner mindestens einen nichtionischen, anionischen, kationischen oder amphoteren grenzflächenaktiven Stoff oder ein Gemisch enthält.

19. Zusammensetzung nach Anspruch 18, **dadurch gekennzeichnet, dass** der grenzflächenaktive Stoff unter den Alkylsulfaten, Alkylbenzolsulfaten, Alkylethersulfaten, Alkylsulfonaten, quartären Ammoniumsalzen, Alkylbetainen, ethoxylierten Alkylphenolen, Alkanolamiden von Fettsäuren, ethoxylierten Fettsäureestern und Hydroxypropylethern ausgewählt ist.

20. Zusammensetzung nach einem der Ansprüche 13 bis 19, **dadurch gekennzeichnet, dass** sie ferner eine Verbindung enthält, die unter den geradkettigen oder cyclischen, flüchtigen oder nichtflüchtigen Siliconen und deren Gemischen, Polydimethylsiloxanen, quaternisierten Polyorganosiloxanen, Polyorganosiloxanen mit Aminoalkylgruppen, die mit Alkoxycarbonylalkylgruppen modifiziert sind, Polydimethylsiloxanen mit endständigen Stearoxydimethicongruppen, Copolymern Polydimethylsiloxan/Dialkylammoniumacetat, Copolymeren Polydimethylsiloxan/Polylalkylbetain, mit Mercapto- oder Mercaptoalkylgruppen organomodifizierten Polysiloxanen und Silanen ausgewählt ist.

21. Zusammensetzung nach einem der Ansprüche 13 bis 20, **dadurch gekennzeichnet, dass** sie ferner eine Verbindung enthält, die unter den kationischen Polymeren vom Typ der Ionene, basischen Aminosäuren, Glutaminsäure, Asparaginsäure, Peptiden und ihren Derivaten, Proteinhydrolysaten, Wachsen, Quellmitteln und Penetrationshilfsmitteln, Dimethylisosorbitol, Harnstoff und seinen Derivaten, Pyrrolidon, N-Alkyl-pyrrolidonen, Thiamorpholinon, Alkylethern von Alkylenglycol oder Dialkylenglycol, C₃₋₆-Alkandiolen, Imidazolidinon-2, Fettalkoholen, Lanolinderivaten, Panthothensäure, Wirkstoffen gegen Haarausfall, Antischuppenmitteln, Verdickungsmitteln, Suspendiermitteln, Maskierungsmitteln, Trübungsmitteln, Farbmitteln, Sonnenschutzfiltern, Parfums und Konservierungsmitteln ausgewählt ist.

22. Kit für die dauerhafte Verformung von Haaren, das in einer ersten Abteilung als reduzierende Zusammensetzung eine Zusammensetzung nach einem der Ansprüche 13 bis 21 enthält, und in einer zweiten Abteilung eine oxidierende Zusammensetzung.

23. Dimercaptoamid der folgenden allgemeinen Formel (II):
HS-A-CO-NH-B-SH (II)
wobei in der Formel bedeuten:
(i) A bedeutet die Gruppe CH₂ und B ist eine Gruppe (CH₂)ₓ mit x = 2, 3, 4 oder 5; eine Gruppe C(CH₃)₂-CH₂; eine Gruppe CH(Et)-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(ii) A bedeutet eine Gruppe CH(CH₃) und B ist eine Gruppe (CH₂)ₓ mit x = 1, 2, 3, 4 oder 5; eine Gruppe C(CH₃)₂-CH₂; eine Gruppe CH(Et)-CH₂; eine Gruppe CH(COOH)-CH₂-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(iii) A bedeutet eine Gruppe CH-R₃, wobei R₃ eine geradkettige oder verzweigte C₂₋₅-Alkylgruppe ist, und B bedeutet eine Gruppe (CH₂)ₓ mit x = 1, 2, 3, 4 oder 5; eine Gruppe C(CH₃)₂-CH₂; eine Gruppe CH(Et)-CH₂; eine Gruppe CH(COOH)-CH₂; eine Gruppe CH(COOH)-CH₂CH₂; eine Gruppe CH(CO-NR₁R₂)-CH₂ oder CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(iv) A bedeutet eine Gruppe CH₂-CH₂ oder C(CH₃)₂-CH₂ und B ist eine Gruppe (CH₂)ₓ mit x = 1, 3 oder 4; eine Gruppe C(CH₃)₂-CH₂; eine Gruppe CH(Et)-CH₂; eine Gruppe CH(COOH)-CH₂-CH₂; eine Gruppe CH(CO-NR,R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(v) A bedeutet eine Gruppe CH₂-CH(CH₃) und B ist eine Gruppe (CH₂)ₓ mit x = 1, 2, 3 oder 4; eine Gruppe C(CH₃)₂-CH₂; eine Gruppe CH(Et)-CH₂; eine Gruppe CH(COOH)-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(vi) A bedeutet eine Gruppe CH₂-CH-R₄, wobei R₄ eine Phenylgruppe oder COO-CH₃ oder COO-CH₂-CH₃ bedeutet, und B ist eine Gruppe (CH₂)ₓ mit x = 1, 2, 3 oder 4; eine Gruppe C(CH₃)₂-CH₂; eine Gruppe CH(Et)-CH₂; eine Gruppe CH(COOH)-CH₂-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(vii) A bedeutet eine Gruppe CH₂-CH(CH₂-COOH) oder CH₂CH(NH-CH₂-COOH) und B ist eine Gruppe (CH₂)ₓ mit x = 1, 2, 3 oder 4; eine Gruppe C(CH)₂-CH₂; eine Gruppe CH(Et)-CH₂; eine Gruppe CH(COOH)-CH₂; oder eine Gruppe CH(COOH)-CH₂-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(viii) A bedeutet eine Gruppe C(CH₃)₂ und B bedeutet eine Gruppe (CH₂)ₓ mit x = 1, 3, 4 oder 5; eine Gruppe C(CH₃)₂-CH₂; eine Gruppe CH(Et)-CH₂; eine Gruppe CH(CO-NHCH₃)-CH₂); eine Gruppe CH(CO-NHEt)-CH₂; eine Gruppe CH(CO-NH-CH₂-CHOH-CH₃)-CH₂; oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(ix) A bedeutet eine Gruppe C(Et)₂ und B ist eine Gruppe (CH₂) mit x = 1, 2, 3, 4 oder 5; eine Gruppe C(CH₃)₂-CH₂; eine Gruppe CH(Et)-CH₂; eine Gruppe CH(COOH)-CH₂-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(x) A bedeutet eine Gruppe CH₂-C(CH₃)₂ oder CH(CH₂-Ph) und B ist eine Gruppe (CH₂)ₓ mit x = 1, 2, 3 oder 4; eine Gruppe C(CH₃)₂-CH₂; eine Gruppe CH(Et)-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(xi) A bedeutet eine Gruppe CH₂-C(Et)₂ und B bedeutet eine Gruppe (CH₂)ₓ mit x = 1, 2, 3 oder 4; eine Gruppe C(CH₃)₂-CH₂; eine Gruppe CH(Et)-CH₂; eine Gruppe CH(COOH)-CH₂-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(xii) A bedeutet eine Gruppe CH₂-CH(NH₂) und B ist eine Gruppe (CH₂)ₓ mit x = 1, 3 oder 4; eine Gruppe C(CH₃)₂-CH₂; eine Gruppe CH(Et)-CH₂; eine Gruppe CH(COOH)-CH₂-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(xiii) A bedeutet eine Gruppe CH₂-CH(NHAc) und B ist eine Gruppe (CH₂)ₓ mit x = 1, 3 oder 4; eine Gruppe C(CH₃)₂-CH₂; eine Gruppe CH(Et)-CH₂; eine Gruppe CH(COOH)-CH₂-CH₂; eine Gruppe CH(CO-NH-CH₃)-CH₂; eine Gruppe CH(CO-NH-Et)-CH₂; eine Gruppe CH(CO-N(CH₃)₂)-CH₂; eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(xiv) A bedeutet eine Gruppe CH₂-CH(NH-CO-CH₂-CH₂-CH(NH₂)COOH) oder eine Gruppe CH₂-CH(NH-CO-CH₂-NH₂) oder eine Gruppe CH₂-CH(NH-CO-CH(NH₂)-CH₂-CH(COOH)₂ oder eine Gruppe CH₂-CH(NH-CO-CH₂-CH₂-CH₂-CH(NH₂)COOH) und B ist eine Gruppe (CH₂)ₓ mit x = 1, 2, 3 oder 4; eine Gruppe C(CH₃)₂CH₂; CH(Et)-CH₂; eine Gruppe CH(COOH)-CH₂-CH₂; CH(CO-NR(R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(xv) A ist eine Gruppe (CH₂)₃ und B ist eine Gruppe (CH₂)ₓ mit x = 1, 2 oder 3; eine Gruppe C(CH₃)₂-CH₂; eine Gruppe CH(Et)-CH₂; eine Gruppe CH(COOH)-CH₂; eine Gruppe CH(COOH)-CH₂-CH₂; eine Gruppe CH(CO-NR₁R₂)-CH₂ oder eine Gruppe CH(CO-NR₁R₂)-CH₂CH₂, wobei R₁ und R₂ unabhängig voneinander ein Wasserstoffatom oder Methyl, Ethyl oder 2-Hydroxypropyl bedeuten;
(xvi) A ist eine Gruppe (CH₂)₄ und B ist eine Gruppe (CH₂)ₓ mit x = 1 oder 2; eine Gruppe C(CH₃)₂-CH₂; eine Gruppe CH(Et)-CH₂; eine Gruppe CH(COOH)-CH₂; eine Gruppe CH(COOH)-CH₂-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
(xvii) A ist eine Gruppe (CH₂)₅ und B bedeutet eine Gruppe CH₂;
(xviii) A bedeutet eine Gruppe CH-Ph und B ist eine Gruppe (CH₂)ₓ mit x = 1, 2, 3, 4 oder 5; eine Gruppe C(CH₃)₂-CH₂; eine Gruppe CH(Et)-CH₂; eine Gruppe CH(COOH)-CH₂; eine Gruppe CH(COOH)-CH₂-CH₂; eine Gruppe CH(CO-NR₁R₂)-CH₂ oder eine Gruppe CH(CO-NR₁R₂)-CH₂-CH₂, wobei R₁ und R₂ unabhängig voneinander ein Wasserstoffatom oder Methyl, Ethyl oder 2-Hydroxypropyl bedeuten;
und die organischen und anorganischen Salze der Verbindungen der Formel (II).

24. Dimercaptoamid nach Anspruch 23, **dadurch gekennzeichnet, dass** es unter den folgenden Verbindungen ausgewählt ist:
- 2-Mercapto-N-(2-mercaptoethyl)-acetamid
- 2-Mercapto-N-(3-mercaptopropyl)-acetamid
- 2-Mercapto-N-(4-mercaptobutyl)-acetamid
- 2-Mercapto-N-(5-mercaptopentyl)-acetamid
- 3-Mercapto-2-(2-mercapto-acetylamino)-propionamid
- 3-Mercapto-2-(2-mercapto-acetylamino)-N,N-dimethyl-propionamid
- 2-Mercapto-N-(2-mercapto-1,1-dimethyl-ethyl)-acetamid
- 2-Mercapto-N-(1-mercaptomethyl-propyl)-acetamid
- 2-Mercapto-N-mercaptomethyl-propionamid
- 2-Mercapto-N-(2-mercaptoethyl)-propionamid
- 2-Mercapto-N-(3-mercaptopropyl)-propionamid
- 2-Mercapto-N-(4-mercaptobutyl)-propionamid
- 2-Mercapto-N-(5-mercaptopentyl)-propionamid
- N-(1-Carbamoyl-2-mercaptoethyl)-2-mercaptopropionamid
- N-(1-Dimethylcarbamoyl-2-mercaptoethyl)-2-mercaptopropionamid
- 4-Mercapto-2-(2-mercapto-propionylamino)-buttersäure
- 2-Mercapto-N-(2-mercapto-1,1-dimethylethyl)-propionamid
- 2-Mercaptobuttersäure-mercaptomethylamid
- 2-Mercaptobuttersäure-(2-mercaptoethyl)-amid
- 2-Mercaptobuttersäure-(3-mercaptopropyl)-amid
- 2-Mercaptobuttersäure-(4-mercaptobutyl)-amid
- 2-Mercaptobuttersäure-(5-mercaptopentyl)-amid
- 2-Mercaptobuttersäure-(1-carbamoyl-2-mercaptoethyl)-amid
- N-(1-Dimethylcarbamoyl-2-mercaptoethyl)-2-mercaptobutyramid
- 2-Mercaoptobuttersäure-(1-carbamoyl-3-mercaptopropyl)-amid
- 2-Mercaptobuttersäure-(2-mercapto-1,1-dimethylethyl)-amid
- 2-Mercaptopentansäure-mercapto-methylamid
- 2-Mercaptopentansäure-(2-mercaptoethyl)-amid
- 2-Mercaptopentansäure-(3-mercaptopropyl)-amid
- 2-Mercaptopentansäure-(4-mercaptobutyl)-amid
- 2-Mercaptopentansäure-(5-mercaptopentyl)-amid
- 3-Mercapto-2-(2-mercapto-pentanoylamino)-propionsäure
- 2-Mercaptopentansäure-(1-carbamoyl-2-mercaptoethyl)-amid
- 2-Mercaptopentansäure-(1-dimethylcarbamoyl-2-mercaptoethyl)-amid
- 2-Mercaptopentansäure-(1-carbamoyl-3-mercaptopropyl)-amid
- 2-Mercaptopentansäure-(2-mercapto-1,1-dimethylethyl)-amid
- 3-Mercapto-N-mercaptomethyl-propionamid
- 3-Mercapto-N-(3-mercaptopropyl)-propionamid
- 3-Mercapto-N-(4-mercaptobutyl)-propionamid
- 3-Mercapto-2-(3-mercapto-propionylamino)-propionamid
- N-(1-Dimethylcarbamoyl-2-mercaptoethyl)-3-mercapto-propionamid
- 4-Mercapto-2-(3-mercapto-propionylamino)-buttersäure
- 3-Mercapto-N-(2-mercapto-1,1-dimethylethyl)-propionamid
- 3-Mercapto-N-mercaptomethyl-2-methyl-propionamid
- 3-Mercapto-N-(2-mercaptoethyl)-2-methyl-propionamid
- 3-Mercapto-N-(3-mercaptopropyl)-2-methyl-propionamid
- 3-Mercapto-N-(4-mercaptobutyl)-2-methyl-propionamid
- 3-Mercapto-2-(3-mercapto-2-methyl-propionylamino)-propionsäure
- N-(1-Carbamoyl-2-mercaptoethyl)-3-mercapto-2-methyl-propionamid
- N-(1-Dimethylcarbamoyl-2-mercaptoethyl)-3-mercapto-2-methyl-propionamid
- 3-Mercapto-N-(2-mercapto-1,1-dimethylethyl)-2-methyl-propionamid
- N-Mercaptomethyl-3-mercaptomethyl-succinamsäure
- N-(2-Mercaptoethyl)-3-mercaptomethyl-succinamsäure
- N-(3-Mercaptopropyl)-3-mercaptomethyl-succinamsäure
- N-(4-Mercaptobutyl)-3-mercaptomethyl-succinamsäure
- N-(1-Carboxy-2-mercaptoethyl)-3-mercaptomethyl-succinamsäure
- N-(1-Carboxy-3-mercaptopropyl)-3-mercaptomethyl-succinamsäure
- N-(2-Mercapto-1,1-dimethylethyl)-3-mercaptomethyl-succinamsäure
- N-(2-Mercapto-1-ethyl)-3-mercaptomethyl-succinamsäure
- 2-Mercapto-N-mercaptomethyl-2-methyl-propionamid
- 2-Mercapto-N-(3-mercaptopropyl)-2-methylpropionamid
- 2-Mercapto-N-(4-mercaptobutyl)-2-methylpropionamid
- 2-Mercapto-N-(5-mercaptopentyl)-2-methylpropionamid
- 2-Mercapto-N-(2-mercapto-1,1-dimethylethyl)-2-methyl-propionamid
- 2-Mercapto-N-(2-mercapto-1-ethyl)-2-methyl-propionamid
- 3-Mercapto-N-mercaptomethyl-3-methylbutyramid
- 3-Mercapto-N-(3-mercaptopropyl)-3-methyl-butyramid
- 3-Mercapto-N-(4-mercaptobutyl)-3-methyl-butyramid
- N-(1-Carbamoyl-2-mercaptoethyl)-3-mercapto-3-methyl-butyramid
- N-(1-Dimethylcarbamoyl-2-mercaptoethyl)-3-mercapto-3-methyl-butyramid
- 4-Mercapto-2-(3-mercapto-3-methyl-butyrylamino)-buttersäure
- 3-Mercapto-N-(2-mercapto- 1,1 -dimethylethyl)-3-methyl-butyramid
- 3-Mercapto-N-(2-mercapto-1-ethyl)-3-methyl-butyramid
- 3-Mercapto-N-mercaptomethyl-2,2-dimethyl-propionamid
- 3-Mercapto-N-(2-mercaptoethyl)-2,2-dimethyl-propionamid
- 3-Mercapto-N-(3-mercaptopropyl)-2,2-dimethyl-propionamid
- 3-Mercapto-N-(4-mercaptobutyl)-2,2-dimethyl-propionamid
- 3-Mercapto-N-(2-mercapto- 1, 1 -dimethylethyl)-2,2-dimethyl-propionamid
- N-(1-Carbamoyl-2-mercaptoethyl)-3-mercapto-2,2-dimethyl-propionamid
- N-(1-Dimethylcarbamoyl-2-mercaptoethyl)-3-mercapto-2,2-dimethylpropionamid
- 2-Amino-3-mercapto-N-mercaptomethyl-propionamid
- 2-Amino-3-mercapto-N-(3-mercaptopropyl)-propionamid
- 2-Amino-3-mercapto-N-(4-mercaptobutyl)-propionamid
- 2-Amino-N-(1-carbamoyl-2-mercaptoethyl)-3-mercaptopropionamid
- 2-Amino-N-(1-carbamoyl-2-mercaptoethyl)-3-mercapto-N,N-dimethylpropionamid
- 2-(2-Amino-3-mercapto-propionylamino)-4-mercaptobuttersäure
- 2-Amino-3-mercapto-N-(2-mercapto-1,1-dimethylethyl)-propionamid
- 2-Amino-3-mercapto-N-(2-mercapto-1-ethyl)-propionamid
- 2-Acetylamino-3-mercapto-N-mercaptomethyl-propionamid
- 2-Acetylamino-3-mercapto-N-(3-mercaptopropyl)-propionamid
- 2-Acetylamino-3-mercapto-N-(4-mercaptobutyl)-propionamid
- 2-(2-Acetylamino-3-mercapto-propionylamino)-3-mercapto-3-methylbuttersäure
- 2-Acetylamino-3-mercapto-N-(1-mercaptomethylpropyl)-propionamid
- 2-Acetylamino-N-(1-dimethylcarbamoyl-2-mercaptoethyl)-3-mercapto-propionamid
- 2-(2-Acetylamino-3-mercapto-propionylamino)-4-mercaptobuttersäure
- 2-(Carboxymethylamino)-3-mercapto-N-mercaptomethyl-propionamid
- 2-(Carboxymethylamino)-3-mercapto-N-(2-mercaptoethyl)-propionamid
- 2-(Carboxymethylamino)-3-mercapto-N-(3-mercaptopropyl)-propionamid
- 2-(Carboxymethylamino)-3-mercapto-N-(4-mercaptobutyl)-propionamid
- 2-(Carboxymethylamino)-N-(1-carbamoyl-2-mercaptoethyl)-3-mercapto-propionamid
- 2-[2-(Carboxymethylamino)-3-mercapto-propionylamino]-3-mercaptopropionsäure
- [1-(1-Carbamoyl-2-mercapto-ethylcarbamoyl)-2-mercaptoethylamino]-essigsäure
- 2-(Carboxymethylamino)-3-mercapto-N-(2-mercapto-1,1-dimethylethyl)-propionamid
- 2-Amino-4-[2-mercapto-1-(mercaptomethylcarbamoyl)-ethylcarbamoyl]-buttersäure
- 2-Amino-4-[2-mercapto-1-(2-mercaptoethylcarbamoyl)-ethylcarbamoyl]-buttersäure
- 2-Amino-4-[2-mercapto-1-(3-mercaptopropylcarbamoyl)-ethylcarbamoyl]-buttersäure
- 2-Amino-4-[2-mercapto-1-(4-mercaptobutylcarbamoyl)-ethylcarbamoyl]-buttersäure
- 2-[2-(4-Amino-4-carboxy-butyrylamino)-3-mercaptopropionylamino]-4-mercaptobuttersäure
- 2-Amino-4-[1-(1-carbamoyl-2-mercapto-ethylcarbamoyl)-2-mercapto-ethylcarbamoyl]-buttersäure
- 2-Amino-4-[1-(1-dimethylcarbamoyl-2-mercapto-ethylcarbamoyl)-2-mercapto-ethylcarbamoyl]-buttersäure
- 2-Amino-4-[2-mercapto-1-(2-mercapto-1,1-dimethyl-ethylcarbamoyl)-ethylcarbamoyl]-buttersäure
- 2-Amino-4-[2-mercapto-1-(2-mercapto-1-ethyl-ethylcarbamoyl)-ethylcarbamoyl]-buttersäure
- 2-(2-Aminoacetylamino)-3-mercapto-N-mercaptomethyl-propionamid
- 2-(2-Aminoacetylamino)-3-mercapto-N-(2-mercaptoethyl-propionamid
- 2-(2-Aminoacetylamino)-3-mercapto-N-(3-mercaptopropyl-propionamid
- 2-(2-Aminoacetylamino)-3-mercapto-N-(4-mercaptobutyl-propionamid
- 2-(2-Aminoacetylamino)-3-mercapto-N-(5-mercaptopentyl-propionamid
- 2-[2-(2-Amino-acetylamino)-3-mercapto-propionylamino]-4-mercaptobuttersäure
- 2-(2-Amino-acetylamino)-3-mercapto-N-(2-mercapto-1,1-dimethylethyl]-propionamid
- 2-(2-Amino-acetylamino)-3-mercapto-N-(1-mercaptomethylpropyl]-propionamid
- 4-Mercapto-N-mercaptomethyl-butyramid
- 4-Mercapto-N-(2-mercaptoethyl)-butyramid
- 4-Mercapto-N-(3-mercaptopropyl)-butyramid
- N-(4-Mercapto-1-oxobutyl)-L-cystein
- 4-Mercapto-2-(3-mercapto-propionylamino)-butyramid
- N-(1-Dimethylcarbamoyl-2-mercapto-ethyl)-4-mercapto-butyramid
- N-(4-Mercapto-1-oxobutyl)-L-homocystein
- 4-Mercapto-N-(2-mercapto-1,1-dimethyl-ethyl)-butyramid
- 4-Mercapto-N-(1-mercaptomethyl-propyl)-butyramid
- 5-Mercaptopentansäure-mercaptomethylamid
- 5-Mercaptopentansäure-(2-mercapto-ethyl)-amid
- N-(5-Mercapto-1-oxopentyl)-L-cystein
- 5-Mercapto-2-(3-mercapto-propionylamino)-pentanamid
- N-(1-Dimethylcarbamoyl-2-mercapto-ethyl)-5-mercapto-pentanamid
- 5-Mercapto-N-(2-mercapto-1,1-dimethyl-ethyl)-pentanamid
- 5-Mercapto-N-(1-mercaptomethyl-propyl)-pentanamid
- 6-Mercapto-hexansäure-mercaptomethylamid.

25. Dimercaptoamid nach Anspruch 23, **dadurch gekennzeichnet, dass** es unter den folgenden Verbindungen ausgewählt ist:
- 2-Mercapto-N-(2-mercaptoethyl)-acetamid
- 2-Mercapto-N-(3-mercaptopropyl)-acetamid
- 2-Mercapto-N-(4-mercaptobutyl)-acetamid
- 2-Mercapto-N-(5-mercaptopentyl)-acetamid
- 3-Mercapto-2-(2-mercapto-acetylamino)-propionamid
- 3-Mercapto-2-(2-mercapto-acetylamino)-N,N-dimethyl-propionamid
- 2-Mercapto-N-(2-mercapto-1,1-dimethyl-ethyl)-acetamid
- 2-Mercapto-N-(1-mercaptomethyl-propyl)-acetamid
- 2-Mercapto-N-mercaptomethyl-propionamid
- 2-Mercapto-N-(2-mercaptoethyl)-propionamid
- 2-Mercapto-N-(3-mercaptopropyl)-propionamid
- 2-Mercapto-N-(4-mercaptobutyl)-propionamid
- 2-Mercapto-N-(5-mercaptopentyl)-propionamid
- N-(1-Carbamoyl-2-mercaptoethyl)-2-mercaptopropionamid
- N-(1-Dimethylcarbamoyl-2-mercaptoethyl)-2-mercaptopropionamid
- 4-Mercapto-2-(2-mercapto-propionylamino)-buttersäure
- 2-Mercapto-N-(2-mercapto-1,1-dimethylethyl)-propionamid
- 2-Mercaptobuttersäure-mercaptomethylamid
- 2-Mercaptobuttersäure-(2-mercaptoethyl)-amid
- 2-Mercaptobuttersäure-(3-mercaptopropyl)-amid
- 2-Mercaptobuttersäure-(4-mercaptobutyl)-amid
- 2-Mercaptopentansäure-mercaptomethyl-amid
- 2-Mercaptopentansäure-(2-mercaptoethyl)-amid
- 2-Mercaptopentansäure-(3-mercaptopropyl)-amid
- 3-Mercapto-N-mercaptomethyl-propionamid
- 3-Mercapto-N-(3-mercaptopropyl)-propionamid
- 3-Mercapto-2-(3-mercapto-propionylamino)-propionamid
- N-(1-Dimethylcarbamoyl-2-mercaptoethyl)-3-mercaptopropionamid
- 4-Mercapto-2-(3-mercapto-propionylamino)-buttersäure
- 3-Mercapto-N-(2-mercapto-1,1-dimethylethyl)-propionamid
- 3-Mercapto-N-mercaptomethyl-2-methyl-propionamid
- 3-Mercapto-N-(2-mercaptoethyl)-2-methyl-propionamid
- 3-Mercapto-N-(3-mercaptopropyl)-2-methyl-propionamid
- 3-Mercapto-N-(4-mercaptobutyl)-2-methyl-propionamid
- 3-Mercapto-2-(3-mercapto-2-methyl-propionylamino)-propionsäure
- N-(1-Carbamoyl-2-mercaptoethyl)-3-mercapto-2-methyl-propionamid
- N-(1-Dimethylcarbamoyl-2-mercaptoethyl)-3-mercapto-2-methyl-propionamid
- 3-Mercapto-N-(2-mercapto-1,1-dimethylethyl)-2-methyl-propionamid
- N-Mercaptomethyl-3-mercaptomethyl-succinamsäure
- N-(2-Mercaptoethyl)-3-mercaptomethyl-succinamsäure
- N-(3-Mercaptopropyl)-3-mercaptomethyl-succinamsäure
- N-(4-Mercaptobutyl)-3-mercaptomethyl-succinamsäure
- N-(1-Carboxy-2-mercaptoethyl)-3-mercaptomethyl-succinamsäure
- 2-Mercapto-N-mercaptomethyl-2-methyl-propionamid
- 2-Mercapto-N-(3-mercaptopropyl)-2-methylpropionamid
- 2-Mercapto-N-(4-mercaptobutyl)-2-methylpropionamid
- 2-Mercapto-N-(2-mercapto-1,1-dimethylethyl)-2-methyl-propionamid
- 2-Mercapto-N-(2-mercapto-1-ethyl)-2-methyl-propionamid
- 3-Mercapto-N-mercaptomethyl-3-methylbutyramid
- 3-Mercapto-N-(3-mercaptopropyl)-3-methylbutyramid
- N-(1-Carbamoyl-2-mercaptoethyl)-3-mercapto-3-methyl-butyramid
- N-(1-Dimethylcarbamoyl-2-mercaptoethyl)-3-mercapto-3-methyl-butyramid
- 4-Mercapto-2-(3-mercapto-3-methyl-butyrylamino)-buttersäure
- 3-Mercapto-N-mercaptomethyl-2,2-dimethyl-propionamid
- 3-Mercapto-N-(2-mercaptoethyl)-2,2-dimethyl-propionamid
- 3-Mercapto-N-(3-mercaptopropyl)-2,2-dimethyl-propionamid
- 3-Mercapto-N-(4-mercaptobutyl)-2,2-dimethyl-propionamid
- N-(1-Carbamoyl-2-mercaptoethyl)-3-mercapto-2,2-dimethyl-propionamid
- N-(1-Dimethylcarbamoyl-2-mercaptoethyl)-3-mercapto-2,2-dimethylpropionamid
- 2-Amino-3-mercapto-N-mercaptomethyl-propionamid
- 2-Amino-3-mercapto-N-(3-mercaptopropyl)-propionamid
- 2-Amino-3-mercapto-N-(4-mercaptobutyl)-propionamid
- 2-Amino-N-(1-carbamoyl-2-mercaptoethyl)-3-mercaptopropionamid
- 2-Amino-N-(1-carbamoyl-2-mercaptoethyl)-3-mercapto-N,N-dimethylpropionamid
- 2-(2-Amino-3-mercapto-propionylamino)-4-mercaptobuttersäure
- 2-Amino-3-mercapto-N-(2-mercapto-1, 1 -dimethylethyl)-propionamid
- 2-Amino-3-mercapto-N-(2-mercapto-1-ethyl)-propionamid
- 2-Acetylamino-3-mercapto-N-mercaptomethyl-propionamid
- 2-(Carboxymethylamino)-3-mercapto-N-mercaptomethyl-propionamid
- 2-(Carboxymethylamino)-3-mercapto-N-(2-mercaptoethyl)-propionamid
- 2-(Carboxymethylamino)-3-mercapto-N-(3-mercaptopropyl)-propionamid
- 2-(2-Aminoacetylamino)-3-mercapto-N-mercaptomethyl-propionamid
- 2-(2-Aminoacetylamino)-3-mercapto-N-(2-mercaptoethyl-propionamid
- 4-Mercapto-N-mercaptomethyl-butyramid
- 4-Mercapto-N-(2-mercaptoethyl)-butyramid
- 4-Mercapto-N-(3-mercaptopropyl)-butyramid
- N-(4-Mercapto-1-oxobutyl)-L-cystein
- 4-Mercapto-2-(3-mercapto-propionylamino)-butyramid
- N-(1-Dimethylcarbamoyl-2-mercapto-ethyl)-4-mercapto-butyramid
- 4-Mercapto-N-(2-mercapto-1,1-dimethyl-ethyl)-butyramid
- 4-Mercapto-N-(1-mercaptomethyl-propyl)-butyramid
- 5-Mercaptopentansäure-mercaptomethylamid
- 5-Mercaptopentansäure-(2-mercapto-ethyl)-amid
- N-(5-Mercapto-1-oxopentyl)-L-cystein
- 5-Mercapto-2-(3-mercapto-propionylamino)-pentanamid
- N-(1-Dimethylcarbamoyl-2-mercapto-ethyl)-5-mercapto-pentanamid
- 5-Mercapto-N-(2-mercapto-1,1-dimethyl-ethyl)-pentanamid
- 5-Mercapto-N-(1-mercaptomethyl-propyl)-pentanamid
- 6-Mercapto-hexansäure-mercaptomethylamid.

26. Reduzierende Zusammensetzung, **dadurch gekennzeichnet, dass** sie mindestens ein Dimercaptoamid nach einem der Ansprüche 23 bis 25 enthält.

27. Verfahren zur Herstellung eines Dimercaptoamids der Formel (II) nach Anspruch 23, **dadurch gekennzeichnet, dass** es die Umsetzung eines geschützten Mercaptosäurehalogenids der Formel P-S-A-CO-X mit einem geschützten Aminothiol der Formel N₂N-B-S-P, wobei A und B die in Anspruch 23 angegebenen Bedeutungen aufweisen, P eine Schutzgruppe der Thiolfunktion ist und X Chlor oder Brom bedeutet, in einem aprotischen Lösungsmittel in Gegenwart eines Stoffes, der dazu vorgesehen ist, die frei gesetzte Salzsäure oder Bromwasserstoffsäure abzufangen, wie beispielsweise in Gegenwart eines tertiären Amins, um ein geschütztes Dimercaptoamid der Formel P-S-A-CO-NH-B-S-P zu bilden, und anschließend das Entfernen der Schutzgruppe von dem geschützten Dimercaptoamid durch Hydrolyse oder durch Reduktion umfasst.

28. Verfahren zur Herstellung eines Dimercaptoamids der Formel (II) nach Anspruch 23, **dadurch gekennzeichnet, dass** es die Umsetzung einer Mercaptosäure der Formel HS-A-COOH mit einem Aminothiol der Formel H₂N-B-SH, wobei A und B die in Anspruch 23 angegebenen Bedeutungen aufweisen, in Gegenwart eines Kupplungsmittels und/oder Dehydratisierungsmittels in einem aprotischen Lösungsmittel umfasst.

29. Verfahren zur Herstellung eines Dimercaptoamids der Formel (II) nach Anspruch 23, **dadurch gekennzeichnet, dass** es die Umsetzung eines Mercaptoesters der Formel HS-A-COOR, wobei R eine Methyl- oder Ethylgruppe bedeutet, mit einem Aminothiol der Formel H₂N-B-SH, wobei A und B die oben in Anspruch 23 angegebenen Bedeutungen aufweisen, bei einer Temperatur im Bereich von 30 bis 180 °C umfasst.

30. Verfahren zur Herstellung eines Dimercaptoamids der Formel (II) nach Anspruch 23, **dadurch gekennzeichnet, dass** es umfasst:
a) die Umsetzung eines halogenierten Säurehalogenids der Formel X-A-CO-Cl mit einem Halogenamin der Formel H₂N-B-X, wobei X ein Chloratom oder ein Bromatom ist und wobei A und B die in Anspruch 23 angegebenen Bedeutungen aufweisen, in einem aprotischen Lösungsmittel und in Gegenwart eines Stoffes, der die frei gesetzte Salzsäure oder Bromwasserstoffsäure abfangen soll, um ein Dihalogenamid der Formel X-A-CO-NH-B-X zu bilden, und anschließend
b) die Umwandlung des erhaltenen Dihalogenamids in ein Dimercaptoamid der Formel (II) entweder durch direkte Umsetzung mit Schwefelwasserstoff in einem geeigneten Lösungsmittel oder durch Umsetzung mit Thioessigsäure, vorzugsweise in Salzform, und anschließende saure oder alkalische Hydrolyse des Di(S-acetyl)mercaptoamids oder durch Umsetzung mit Thioharnstoff und anschließende Hydrolyse des Isothiouroniumdisalzes.

31. Verfahren zur Herstellung eines Dimercaptoamids der Formel (II) nach Anspruch 23, **dadurch gekennzeichnet, dass** es die Umsetzung eines Thiolactons der Formel 11 mit einem Aminothiol der Formel 5 in einem aprotischen Lösungsmittel gemäß der folgenden Reaktion umfasst: 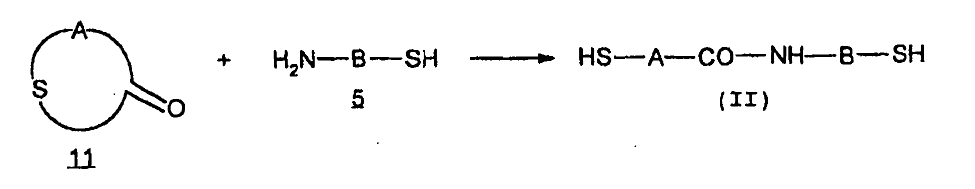 wobei A und B die in Anspruch 23 angegebenen Bedeutungen aufweisen.

32. Verfahren zur Herstellung eines Dimercaptoamids der Formel (II) nach Anspruch 23, **dadurch gekennzeichnet, dass** es die Umsetzung eines Mercaptoamids der Formel HS-A-CO-NH₂ mit einem Aminothiolsalz der Formel HX, H₂N-B-SH, wobei X ein Chloratom oder Bromatom bedeutet und A und B die in Anspruch 23 angegebenen Bedeutungen aufweisen, bei einer Temperatur im Bereich von 60 bis 200 °C umfasst.
